# EUROPEAN PATENT SPECIFICATION

(11) **EP 0 719 253 B1**
(45) Date of publication and mention of the grant of the patent: **28.04.2004**
(21) Application number: 94926394.1
(22) Date of filing: 13.09.1994
(51) Int. Cl.: C07D 207/16, C07D 211/60, A61K 31/40, A61K 31/445

(54) **3-AMINO-5-CARBOXY-SUBSTITUTED PIPERIDINES AND 3-AMINO-4-CARBOXY-SUBSTITUTED PYRROLIDINES AS TACHYKININ ANTAGONISTS**
3-AMINO-5-CARBOXY-SUBSTITUIERTE PIPERIDINE UND 3-AMINO-4-CARBOXY-SUBSTITUIERTE PYRROLIDINE ALS TACHYKININ-ANTAGONISTEN
PIPERIDINES 3-AMINO-5-CARBOXY-SUBSTITUEES ET PYRROLIDINES 3-AMINO-4-CARBOXY-SUBSTITUEES UTILISEES COMME ANTAGONISTES DE TACHYKININE

(30) Priority: 17.09.1993 JP 25506493
(43) Date of publication of application: 03.07.1996
(73) Proprietor: PFIZER INC., New York, N.Y. 10017-5755 (US)
(72) Inventor: IKUNAKA, Masaya, Chita-gun Aichi-ken 470-23 (JP); SHISHIDO, Yuuji 362, Azagiro Ooaza-okuda, Chita-gun Aichi-ken 470-23 (JP); NAKANE, Masami 6-28, Kawanahonmachi Showa-ku, Aichi-ken 466 (JP)
(74) Representative: Wood, David John
(86) International application number: PCT/JP1994/001514
(87) International publication number: WO 1995/007886

(56) References cited:
- EP-A- 0 610 021
- WO-A-91/09844
- WO-A-92/10476
- WO-A-92/20676
- WO-A-93/00331
- WO-A-93/19064
- WO-A-94/10170

## Description

### Thechnical Field

This invention relates to novel and useful substituted azaheterocyclecarboxylic acids of interest to those in the field of medical chemistry and chemotherapy. More particularly, it is concerned with a novel series of substituted azaheterocyclecarboxylic acids, including their pharmaceutically acceptable salts, which are of special value in view of their ability to antagonize substance P. In this way, these compounds are of use in treating gastrointestinal disorders, central nervous system disorders, inflammatory diseases, asthma, pain, migraine and emesis.

### Background Art

Substance P is a naturally occurring undecapeptide belonging to the tachykinin family of peptides, the latter being so-named because of their prompt stimulatory action on smooth muscle tissue. More specially, substance P is a pharmaceutically active neuropeptide that is produced in mammals (having originally been isolated from gut) and possesses a characteristic amino acid sequence that is illustrated by D. F. Veber et al. in US Pat. 4680283. The wide involvement of substance P and other tachykinins in the pathophysiology of numerous diseases has been amply demonstrated in the art. For instance, substance P has recently been shown to be involved in the transmission of pain or migraine, as well as in central nervous system disorders such as anxiety and schizophrenia, in respiratory and inflammatory diseases such as asthma and rheumatoid arthritis, respectively, and in gastrointestinal disorders and diseases of GI tract, like ulcerative colitis and Crohn's diseases, etc.

It is reported that the tachykinin antagonists are useful for allergic conditions, immunoregulation, vasodilation, bronchospasm, reflex or neuronal control of the viscera and senile dementia of the Alzheimer type and for the treatment of emesis and sunburn.

In the recent past, some attempts to develop the tachykinin antagonist such as the one against substance P have been carried out for the purpose of the treatment of the above disorders or diseases.

WO 91/09844 and WO 93/0031 disclose a wide variety of azaheterocyclic compounds as tachykinin antagonists such as substance P antagonists. However, none of the azaheterocyclic compounds disclosed in these references has a carboxy group on the azaheterocyclic ring.

Under the circumstances, the present inventors have worked to prepare compounds useful as substance P antagonist, and after extensive research, have succeeded in synthesizing a series of compounds as will be disclosed in detail herein.

The purpose of the present invention is to provide the novel substituted azaheterocyclecarboxylic acid with substance P antagonistic activity. In addition, the purpose of the invention is also to provide the pharmaceutical composition, which includes the substituted azaheterocyclecarboxylic acid as an active ingredient, for treating of gastrointestinal disorders, central nervous system disorders, allergy, inflammatory diseases, asthma, pain, migraine or emesis in mammalia, especially human.

### Brief Description of the Invention

The present invention provides novel substituted azaheterocyclecarboxylic acids of the following chemical formula (I) and its pharmaceutically acceptable salt. 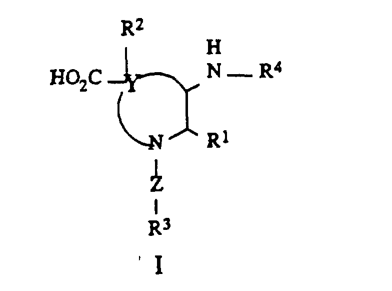 wherein Y is C₂-C₄ alkylene;
Z is a valence bond or C₁-C₆ alkylene;
R¹ is phenyl, biphenyl, indanyl, naphthyl, thienyl, furyl, pyridyl, thiazolyl, isothiazolyl, oxazolyl, isoxazolyl, tetrazolyl, quinolyl, phenyl C₁-C₆ alkyl- or benzhydryl, wherein each of the ring moieties may optionally be substituted by one or more substituents independently selected from halogen, C₁-C₆ alkyl, halosubstituted C₁-C₆ alkyl, C₁-C₆ alkoxy and halosubstituted C₁-C₆ alkoxy;
R² is hydrogen or C₁-C₆ alkyl;
R³ is hydrogen, hydroxy, cyano, amino or carboxy; and
R⁴ represents a group of the formula (II) 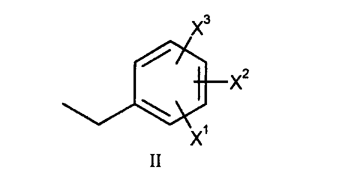 wherein X¹, X² and X³ are each halo, hydrogen, nitro, C₁-C₆ alkyl, halosubstituted C₁-C₆ alkyl, C₁-C₆ alkoxy, halosubstituted C₁-C₆ alkoxy, hydroxy, amino, C₁-C₆ alkylthio, C₁-C₆ alkylsulfinyl or C₁-C₆ alkylsulfonyl;
provided that (i) when Z is a valence bond, R₃ is hydrogen and (ii) when Z is a valence bond, R³ is hydrogen, Y is C₃ alkylene, R² is H and R¹ is phenyl, then R⁴ is other than a group of the formula:- 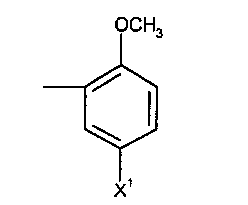 wherein X¹ is (C₁-C₅) alkyl, methylthio, methylsulfinyl or methylsulfonyl.

Also, the present invention provides a pharmaceutical composition which comprises a compound of formula (I) or pharmaceutically acceptable salt thereof together with a pharmaceutically acceptable carrier.

This invention also includes a compound of the formula (I) or pharmaceutically acceptable salt thereof for use as a medicament, particularly for use in treating or preventing gastrointestinal disorders, central nervous system disorders, allergy, inflammatory diseases, asthma, pain, migraine or emesis.

Further, the present invention provides the use of a compound of the formula (I) or pharmaceutically acceptable salt thereof for the manufacture of a medicament for treating or preventing gastrointestinal disorders, central nervous system disorders, allergy, inflammatory diseases, asthma, pain, migraine or emesis.

### Detailed Description of the Invention

In this specification, the term "alkyl" is used herein to mean straight or branched hydrocarbon chain radicals including, but not limited to, methyl, ethyl, n-propyl, isopropyl, n-butyl, isobutyl, t-butyl, and the like;
the term "alkoxy" is used herein to mean -O-alkyl including, but not limited to, methoxy, ethoxy, propoxy, isopropoxy, n-butoxy, isobutoxy, t-butoxy and the like;
the term "halo" is used herein to mean fluorine, chlorine, bromine and iodine;
the term "halosubstituted alkyl" is used herein to mean an alkyl radical substituted with one or more halogens including, but not limited to, chloromethyl, trifluoromethyl, 2,2,2-trichlorethyl and the like; and
the term "halosubstituted alkoxy" is used herein to mean an alkoxy radical substituted with one or more halogens including, but not limited to, chloromethoxy, trifluoromethoxy, 2,2,2-trichloroethoxy and the like.

The preferred values of Y are CH₂CH₂ (i.e., piperidine compounds) or CH₂ (i.e., pyrrolidine compounds), wherein the substituent -CO₂H is at 5- or 4 position, respectively.

The preferred group for R¹ is phenyl, thienyl, fluorophenyl, chlorophenyl and bromophenyl, especially phenyl.

A particularly preferred sub-group of compounds of the invention consists of the compounds of formula I, wherein R⁴ is formula (II), Y is ethylene or propylene, R¹ is phenyl, Z-R³ is hydrogen and R² is attached to the carbon atom adjacent to the nitrogen of NZR³. Especially preferred group for R² is hydrogen when Y is CH₂CH₂ and methyl when Y is CH₂.

Preferred individual compounds of this invention are the following:

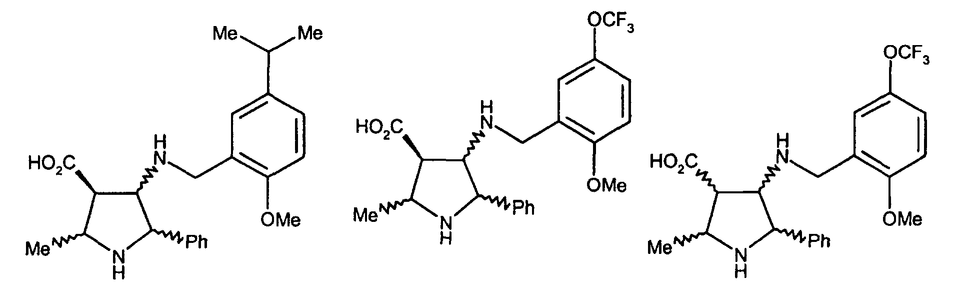

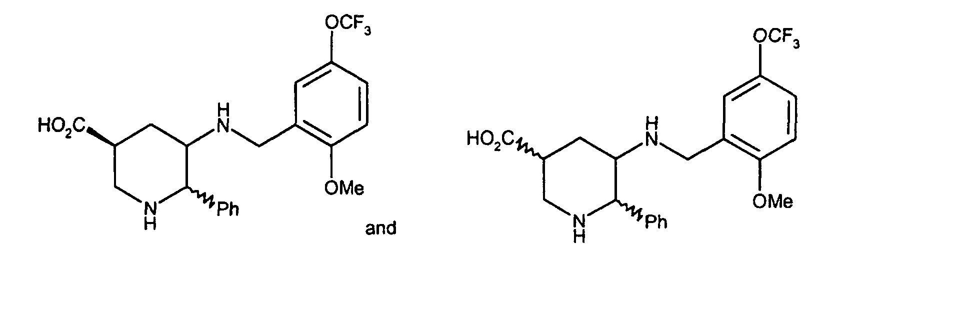

The compounds of formula I form acid addition salts. The pharmaceutically acceptable acid addition salts are those formed from acids which form non-toxic acid salts.

The compounds of formula I may be prepared by a number of synthetic methods well known to those skilled in the art. Thus, the following routes 1 and 2 are available to prepare the objective compounds of the present invention.

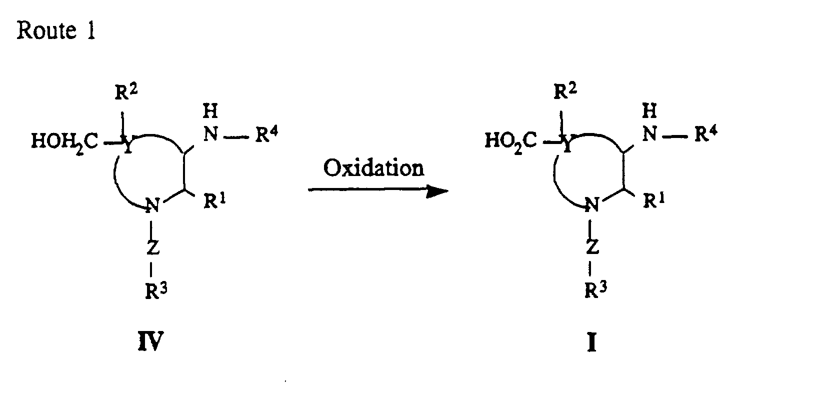

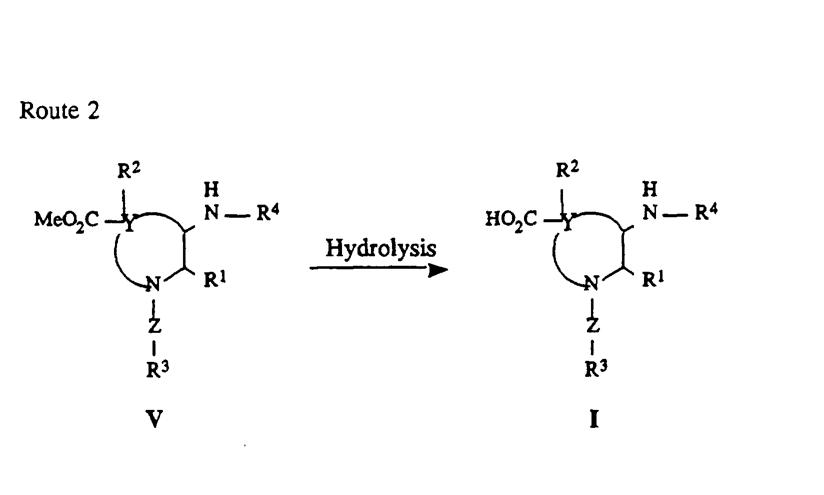

Route 1 is through the oxidation of an alchohol (IV). A lot of conditions which are well-known to those skilled in the art can be adopted for the oxidation. For example, reagents such as KMnO₄, K₂CrO₄, K₂Cr₂O₇ or enzymatic condition and the like can be used.

The second route 2 is through the hydrolysis of methyl ester (V). A lot of conditions which are well-known to those skilled in the art can be adopted for the hydrolysis. For example, acidic condition such as concentrated HCl at reflux temperature, alkaline condition, enzymatic condition and the like can be used.

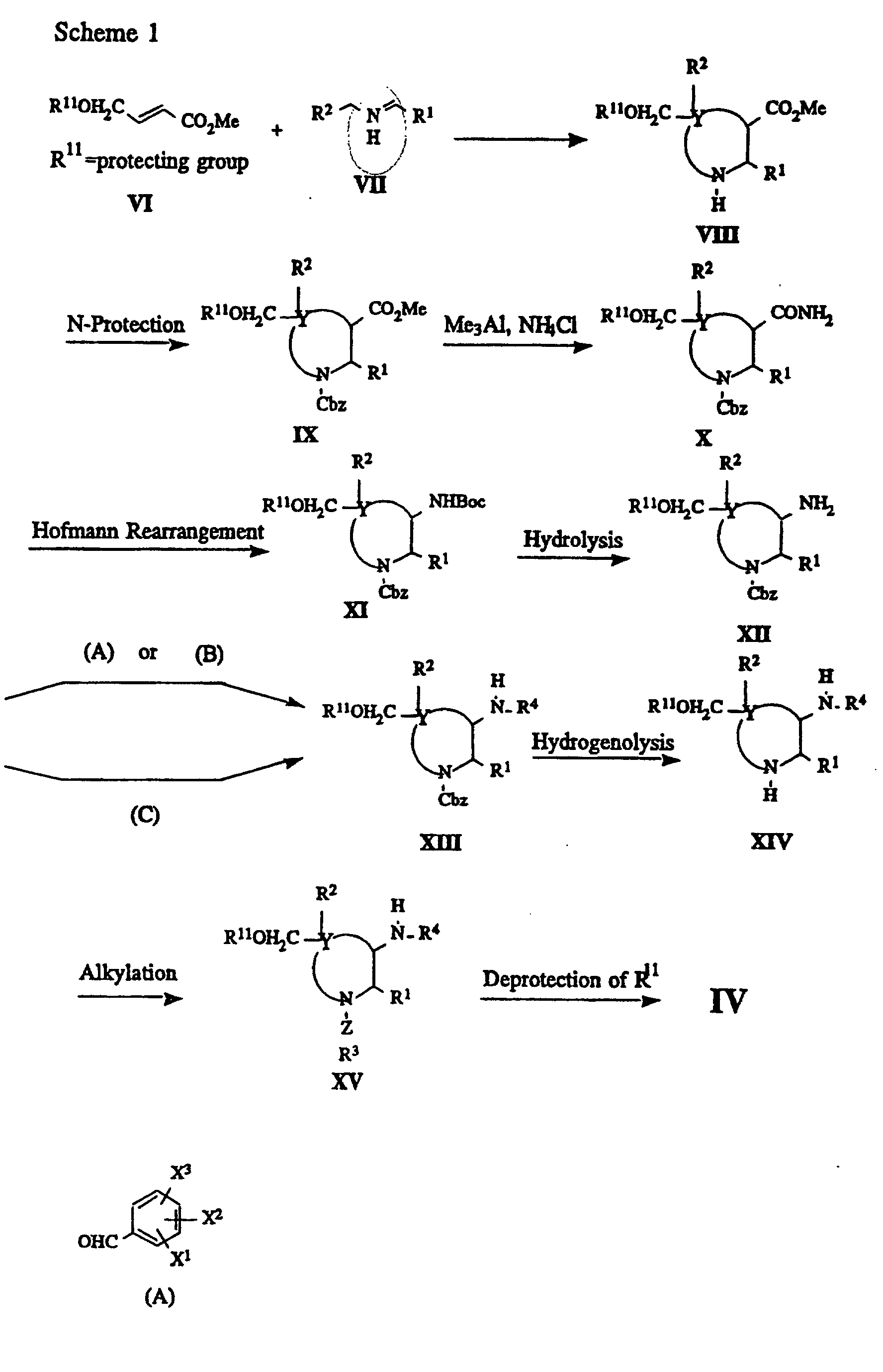

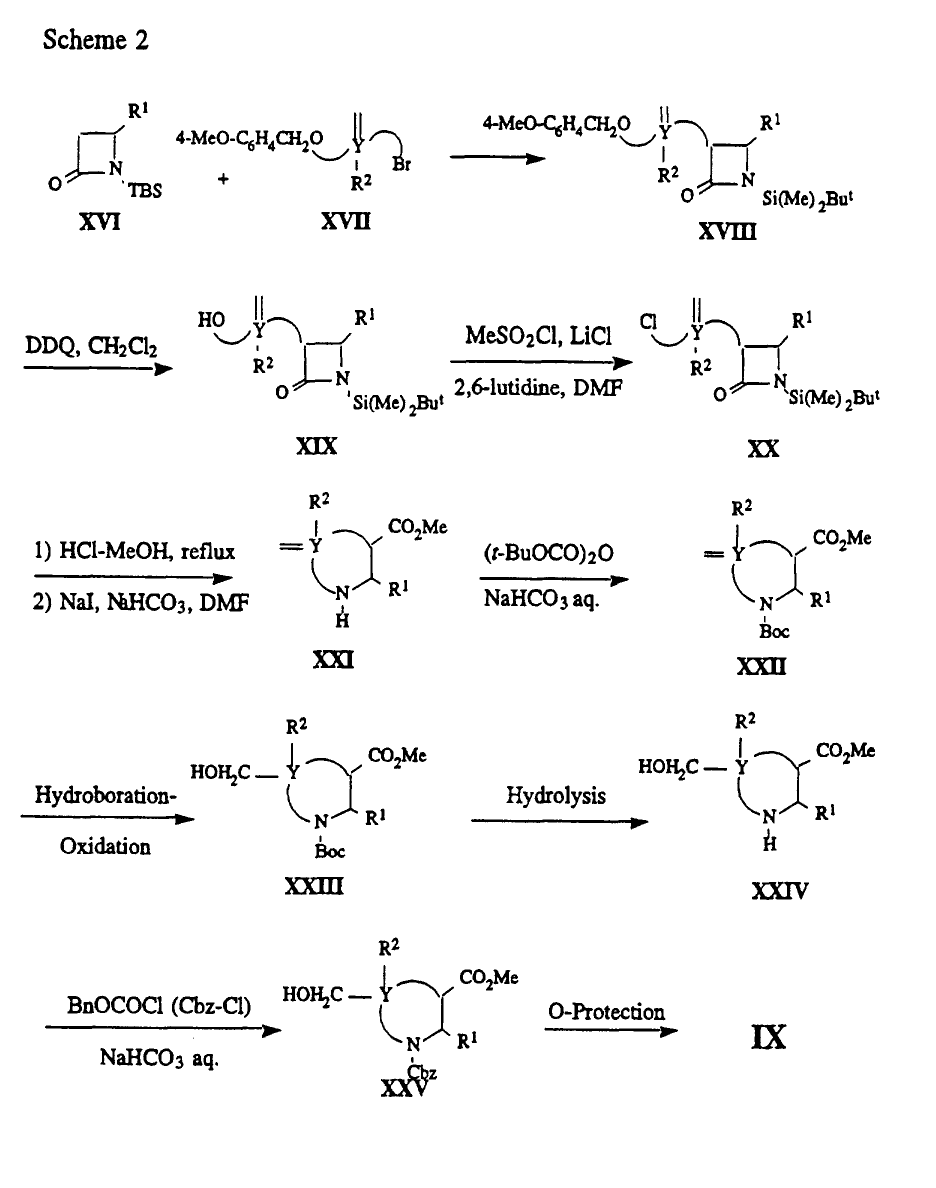

The starting material (IV) in route 1 can be synthesized according to the scheme 1.

The ring of the present invention can be synthesized by coupling of olefin (VI) with imine (VII) in the presence of a base in an inert solvent in the similar manner to the literature (J. Org. Chem., *53*, 1384 (1988)). Preferred solvent is selected from tetrahydrofuran (THF), dioxane, dimethoxyethane (DME), toluene and the like. Proffered base is selected from triethylamine (Et₃N), 1,8-diazabicyclo[5,4,0]undec-7-ene (DBU) and the like. The preffered temperature is 0°C to room temperature.

After N-protection of (VIII) by BnOCOCl (Cbz-Cl) in the presence of a base such as NaHCO₃ or Et₃N, the metoxycarbonyl side chain of (IX) is converted to amide by Me₃Al-NH₄Cl in an inert solvent such as CH₂Cl₂ or benzene at room temperature to 100°C. Hoffmann rearrangement on (X) using Pb(Ac)₄-^{t}BuOH gives (XI). Hydrolysis of (XI) is carried out by using cHCl-AcOEt, HCl-MeOH, CF₃CO₂H and the like to give an amine (XII). The side chain R⁴ is introduced by reductive alkylation with an aldehyde (A) in the presence of a reducing agent such as NaBH₃CN or NaBH(OAc)₃.

(XIII) is then subjected to hydrogenolysis to give XIV. On introduction of -Z-R3 to XIV, R11 is deprotected from XV to give the satarting material (IV) in the route 1. The typical conditions for hydrogenolysis of (XIII) employs H₂ or HCO₂NH₄ in the presence of catalytic amount of Pd-C, Pd(OH)₂-C and the like in an inert solvent such as MeOH or ethanol (EtOH).

The introductions for -Z-R³ can be carried out using an electrophile such as halogen-Z-R³ in the presence of a base such as Et₃N, KO^{t}Bu in an inert solvent such as CH₂CH₂ or Cl(CH₂CH₂)Cl at room temperature to 150°C.

The condition of deprotection of R¹¹ depends on the kind of the protecting group of R¹¹. For example, when R¹¹ is benzyl, catalytic hydrogenolysis (H₂/Pd-C, H₂/Pd(OH)₂-C, Raney Ni and the like in an inert solvent such as methanol) or catalytic transfer hydrogenolysis (HCO₂NH₄ or HCO₂H in the presence of catalytic amount of Pd-C, Pd(OH)₂-C and the like in an inert solvent such as methanol), and when R¹¹ is Si(Me)₂^{t}Bu, (nBu)₄NF, (nBu)₄NF-AcOH, HF, AcOH and the like in an inert solvent such as THF or MeCN can be adopted.

An alternative route for synthesis of (IX) in Scheme 1 is shown in Scheme 2, which employs the β-lactam intermediates. The starting material (XVIII) is synthesized by coupling (XVI) with (XVII). This coupling reaction is carried out in the presence of a base such as LiNEt₂, LiN¹Pr₂, LiN(SiMe₃)₂, or NaN(SiMe₃)₂ in an inert solvent such as THF, DME or Et₂O at -100°C to room temperature. In this reaction a cosolvent such as DMPU or HMPA can be used when it is needed. The beta-lactam (XVIII) is oxidatively deprotected, and the hydroxy group of (XIX) was substituted with Cl group. The β-lactam ring of (XX) was then converted to the ring of the present invention. The deprotection of 4-methoxybenzyl group in the step from (XVIII) to (XIX) is applied to the similar manner to the method described in Tetrahedron, *42*, 3021 (1982), that is, DDQ in phosphate buffer (pH 7). In the presence of a base such as 2,6-lutidine in an inert solvent such as DMF, MeSO₂Cl-LiCl is convenient for chlorination of hydroxy group of (XIX) to (XX) as described in J. Org. Chem., *36*, 3044 (1971). The conditions for the ring reorganization reaction from (XX) to (XXI) involves methanolysis with HCl-MeOH and treatment with NaI-NaHCO₃ in an inert solvent such as DMF at reflux temperature. After the NH group of the compound (XXI) is protected as a t-butyl carbamate using (^{t}BuOCO)₂O in the presence of a base such as NaHCO₃ in an inert solvent system containing H₂O, the exomethylene group in (XXII) is subjected to hydroboration- oxidation to give (XXIII). The hydroborating agent can be selected from BH₃-SMe₂, BH₃-THF, 9-BBN (9-borabicyclo[3.3.1]nonane), thexylborane, disiamylborane, catecholborane and catalytic (PPh₃)₃RhCl and the like in an inert solvent such as THF, DME, Et₂O or diglyme at 0°C to room tempereture. The oxidizing reagent can be selected from 30 % H₂O₂, triethylamine N-oxide, NaBrO₃ and the like.

The compound (XXIII) is then hydrolized in the similar manner to the step from (XI) to (XII), and (XXIV) is protected at NH group with Cbz-Cl in the similar manner to the step from (VIII) to (IX). The OH group of (XXV) is protected by R¹¹ to give (IX). When the R¹¹ group is ^{t}Bu(Me)₂Si-, ^{t}Bu(Me)₂SiCl can be used in the presence of a base such as imidazol or Et₃N in an inert solvent such as DMF or CH₂Cl_{2,} and when the R¹¹ group is benzyl (Bn), BnOC(=NH)CCl₃-CF₃SO₃H can be employed in an inert solvent, especially CH₂Cl₂-cyclohexane(1:2).

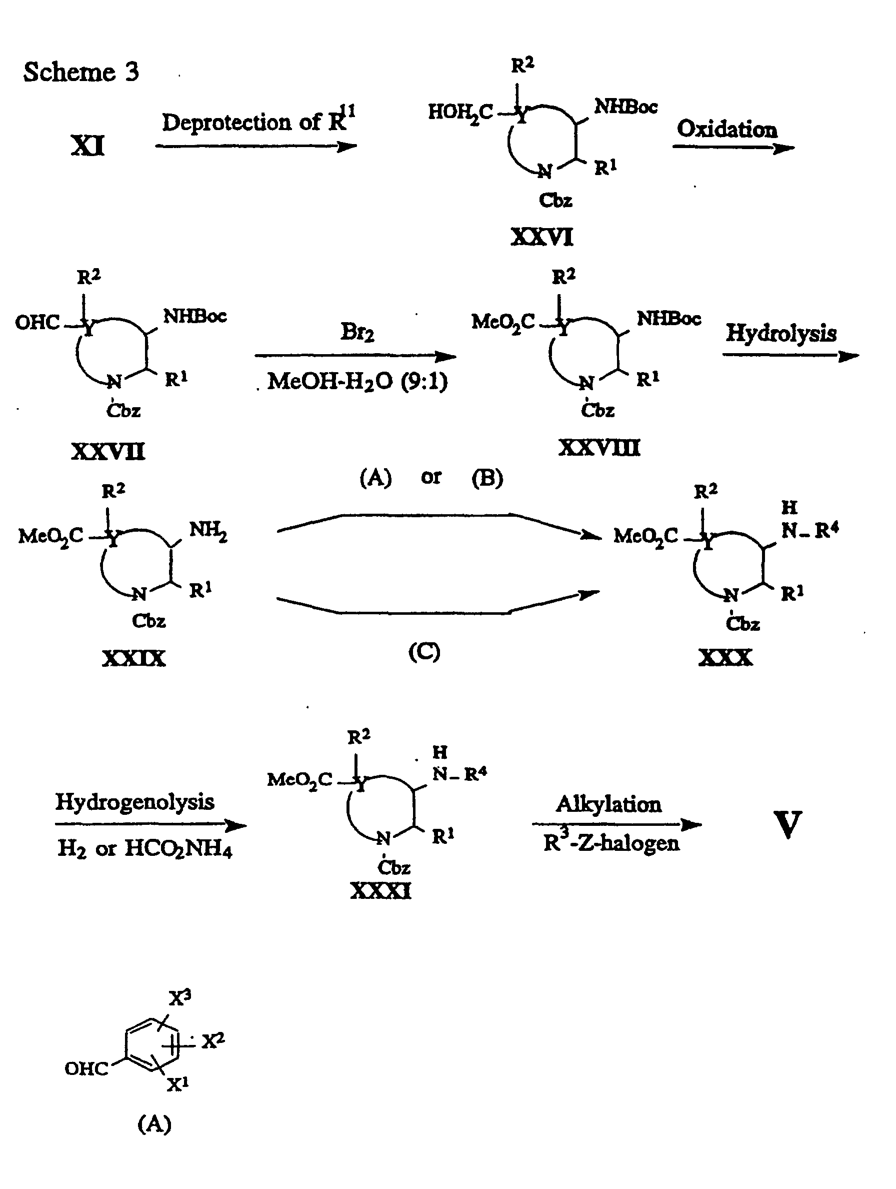

On the other hand, the starting material (V) in route 2 can be synthesized according to the Scheme 3. The compound (XI) described in scheme 1 is deprotected in the similar manner to the step from (XV) to (IV) in Scheme 1 to give the alchohol (XXVI). The alchohol (XXVI) is oxidized to aldehyde (XXVII), which is then subjected to oxidative esterification. PCC, PDC, SO₃.Py or (COCl)₂ can be used as a oxidant in an inert solvent such as CH₂Cl₂ or DMSO. A base such as Et₃N can be added to this oxidation reaction, if it is needed. In the coversion from aldehyde (XXVII) to ester (XXVIII), Br₂ in MeOH-H₂O (9:1) is employed satisfactorily. The deprotection of Boc group of (XXVIII) is carried out in the similar manner to the step from (XI) to (XII). The side chain R⁴ is introduced to the resulting amine (XXIX) by the reaction with (A) in the similar manner to the step from (XII) to (XIII). The Cbz group in the compound (XXX) is deprotected in the similar manner to the step from (XIII) to (XIV). Finally, -Z-R³ can be introduced to XXXI in the similar manner to the step from (XIV) to (XV) to give the starting material (V) in route 2.

In case of preparing the substituted benzaldehyde (A) in Scheme 2 and Scheme 3, the standard methods (formylation of a substituted alkoxybenzene) well-known to those skilled in the art can be used according to the literatures as follows: (1) Duff's reaction (hexamethylenetetramine / TFA), Synth. Commun., 15, 61 (1985), (2) TiCl₄ / dichloromethylmethylether, J. Org. Chem., 51, 4073 (1986), (3) A two step process involving chloromethylation (HCl, HCHO) and oxidation with 2-nitropropane and NaOMe), JP-58-501127 and (4) J. Amer. Chem. Soc., 2466, (1955). Additionally, in order to prepare the substituted benzaldehyde, a Pd-catalyzed coupling reaction on halosubstituted alkoxybenzene in the following literature can be employed: (5) Angew. Chem. Int. Ed. Engl., 25, 508 (1986), J. K. Stille et al., (6) J. Org. Chem., *53* 1170 (1988), J. K. Stille et al., (7) Tetrahedron Lett., 4467 (1975), K. Sonogashira et al., (8) Synthesis, 627 (1980), N. Hagihara et al.

The reaction in the general synthesis is easily monitored by thin-layer chromatography (TLC). The reaction time is in general from a few minutes to several hours. The compounds can be isolated and purified by conventional procedures, such as recrystallisation or chromatography.

As the substituted azaheterocyclecarboxylic acids of formula I of this invention all possess several asymmetric centers, they are capable of existing in a variety of stereoisomeric forms or configurations. The present invention is meant to include all such forms within its scope. For instance, diastereomeric mixtures can be separated by methods well known to those skilled in the art, e.g., by fractional crystallization and the like, while racemic mixtures can be separated by standard resolution methods of organic chemistry.

Since all the substituted azaheterocyclecarboxylic acids of this invention are basic compounds, they are all capable of forming a wide variety of different salts with various inorganic and organic acids.

Although such salts must be pharmaceutically acceptable for administration to animals, it is often desirable in practice to initially isolate the substituted azaheterocyclecarboxylic acids from the reaction mixture as a pharmaceutically unacceptable salt and then simply convert to the free base compound by treatment with an alkaline reagent and thereafter, subsequently convert the free base to a pharmaceutically acceptable acid addition salt. The acid addition salts of this invention are readily prepared by treating the base compound with a substantially equivalent amount of the chosen mineral or organic acid in an aqueous solvent or in a suitable organic solvent, such as methanol or ethanol. Upon careful evaporation of the solvent, the desired solid salt is readily obtained.

The acids which are used to prepare the pharmaceutically acceptable acid addition salts of the aforementioned base compounds of this invention are those which form non-toxic acid addition salts, i.e., salts containing pharmaceutically acceptable anions, such as the hydrochloride, hydrobromide, hydroiodide, nitrate, sulfate or bisulfate, phosphate or acid phosphate, acetate, lactate, citrate or acid citrate, tartrate or bi-tartrate, succinate, maleate, fumarate, gluconate, saccharate, benzoate, methanesulfonate, ethanesulfonate, benzenesulfonate, p-toluenesulfonate and pamoate (i.e., 1,1'-methylene-bis-(2-hydroxy-3-naphthoate))salts.

Substituted azaheterocyclecarboxylic acids of the invention which have also acidic groups are capable of forming base salts with various pharmaceutically acceptable cations. Examples of such salts include the alkali metal or alkaline-earth metal salts and particularly, the sodium and potassium salts. These salts are all prepared by conventional techniques.

The chemical bases which are used as reagents to prepare the pharmaceutically acceptable base salts of this invention are those which form non-toxic base salts with the substituted azaheterocyclecarboxylic acids herein described. These particular non-toxic base salts include those derived form such pharmaceutically acceptable cations as sodium, potassium, calcium and magnesium, etc. These salts can easily be prepared by treating the aforementioned substituted azaheterocyclecarboxylic acids with an aqueous solution containing the desired pharmaceutically acceptable cations, and then by evaporating the resulting solutions to dryness, preferably under reduced pressure. Alternatively, they may also be prepared by mixing lower alkanoic solutions of the acidic compounds and the suitable alkali metal alkoxide together, and then evaporating the resulting solution to dryness in the same manner as before. In either case, stoichiometric quantities of reagents are preferably employed in order to ensure completeness of reaction and production of the maximum yields of the desired final product.

The substituted azaheterocyclecarboxylic acids of the formula I exhibit significant substance P receptor-binding activity and therefore are of value in the treatment of a wide variety of clinical conditions in mammals which are characterized by the presence of an excess of said substance P activity. Such conditions include gastrointestinal disorders such as ulcer and colitis and other like diseases of the gastrointestinal tract; central nervous system disorders such as anxiety and psychosis; inflammatory diseases such as rheumatoid arthritis and inflammatory bowel diseases; respiratory diseases such as asthma; allergy; emesis; sunburn; urinary incontinence; angiogenesis; and pain, including migraine. Hence, these compounds are readily adapted to therapeutic use as substance P antagonists for the control and/or treatment of any of the aforesaid clinical conditions in mammals, including humans.

The radiolabelled substituted azaheterocyclecarboxylic acids of the formula (I) are useful as research and diagnostic tools in metabolism pharmacokinetic studies and in binding assays with the drug in both animal and human. Specific applications in research include radioligand binding assays, autoradiography studies and *in vivo* binding studies, while specific applications in the diagnostic area include studies of substance P receptor in the human brain, such as up/down regulation in a diseases state, and in vivo binding in the relevant tissues for inflammation, e.g., immune-type cell or cells that are directly involved in inflammatory bowel disorders and the like. Specifically, the radiolabelled forms of the substituted azaheterocyclecarboxylic acids are the tritium and ¹⁴C-isotope labelled substituted azaheterocyclecarboxylic acids in this invention.

The substituted azaheterocyclecarboxylic acids of formula (I) hereinbefore described can be administered to a mammalian subject, e.g., a human subject, via either the oral, parenteral or topical routes. In general, these compounds are typically administered to a human subject in doses ranging from about 1 mg to 300 mg per day. Variations will necessarily occur depending upon the weight and condition of the subject being treated, and the activity of the particular compound. However, a dosage level that is in the range from about 0.06 mg to about 6 mg per kg of body weight per day is most desirably employed for the treatment of inflammatory diseases or the like in a human subject. The compounds of the present invention may be administered alone or in combination with pharmaceutically acceptable carriers or diluents by any of the three routes previously indicated, and such administration can be carried out in single or multiple doses. More particularly, the novel therapeutic agents of of the invention can be administered in a wide variety of different dosage forms, i.e., they may be combined with various pharmaceutically acceptable inert carriers in the form of tablets, capsules, lozenges, troches, hard candies, powders, sprays, creams, salves, suppositories, jellies, gels, pastes, lotions, ointments, aqueous suspensions, injectable solutions, elixirs, syrups, and the like. Such carriers include solid diluents or fillers, sterile aqueous media and various non-toxic organic solvents, etc. Moreover, oral pharmaceutical compositions can be suitably sweetened and/or flavored. In general, the therapeutically-effective compounds of this invention are present in such dosage forms at concentration levels ranging about 5.0% to about 70% by weight.

For oral administration, tablets containing various excipients such as microcrystalline cellulose, sodium citrate, calcium carbonate, dicalcium phosphate and glycine may be employed along with various disintegrants such as starch and preferably corn, potato or tapioca starch, alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatine capsules; preferred materials in this connection also include lactose or milk sugar as well as high molecular weight polyethylene grycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a compound of the present invention in either sesame or peanut oil or in aqueous propylene glycol may be employed. The aqueous solutions should be suitably buffered (preferably pH)8) if necessary and the liquid diluent first rendered isotonic. These aqueous solutions are suitable for intravenous injection purposes. The oily solutions are suitable for intra-articular, intramuscular and subcutaneous injection purposes. The preparation of all these solutions under sterile conditions is readily accomplished by standard pharmaceutical techniques well-known to those skilled in the art. Additionally, it is also possible to administer the compounds of the present invention topically when treating inflammatory conditions of the skin and this may preferably be done by way of creams, jellies, gels, pastes, ointments and the like, in accordance with standard pharmaceutical practice.

The activity of the compounds of the present invention, as substance P antagonists, is determined by their ability to inhibit the binding of substance P at its receptor sites in bovine caudate tissue or IM-9 cells employing radioactive ligands. The substance P antagonist activity of the substituted azaheterocyclecarboxylic acids described herein is evaluated by using the standard assay procedure described by M. A. Cascieri et al., as reported in the Journal of Biological Chemistry, *258*, 5158 (1983). This method essentially involves determining the concentration of the individual compound required to reduce by 50% the amount of radiolabelled substance P ligands bound at their receptor sites in said isolated cow tissues or IM-9 cells, thereby affording characteristic IC₅₀ values for each compound tested. In this test, some preferred compounds indicated low IC₅₀ values of less than 1 nM with respect to inhibition of binding at its receptor.

Some compounds of the present invention, when tested as an antiinflammatory agent, exhibit a significant degree of activity in a capsaicin-induced plasma extravasation test [described by A. Nagahisa et al., European Journal of Pharmacology, 217, 191 (1992)].

In this test, antiinflammatory activity is determined as the percent inhibition of plasma protein extravasation in the ureter of male Hartley quinea pigs (weighing 300-350g) in response to the intraperitoneal injection of capsaicin into anesthetized animals. The compounds of the present invention are dissolved in 0.1 % methyl cellulose/water and dosed orally 1h before capsaicin challenge. Evans Blue dye (30mg/kg) is administered intravenously 5 min before capsaicin challenge. The animals are killed 10min after capsaicin injection and both right and left ureters are removed. The Evans Blue dye is extracted and determined colorimetrically.

The anti-psychotic activity of the compounds of the present invention as neuroleptic agents for the control of various psychotic disorders is determined primarily by a study of their ability to suppress substance P-induced hypermotility in rats. This study is carried out by first dosing the rats with a control compound or with an appropriate test compound of the present invention, then injecting the rats with substance P by intracerebral administration via canula and thereafter measuring their individual locomotor response to the said stimuli.

In the above capsaicin-induced plasma extravasation test and anti-psychotic tests, compounds are considered active if the difference in response between the drug-treated animals and a control group receiving the vehicle alone is statistically significant.

### Examples

The present invention is illustrated by the following examples. However, it should be understood that the invention is not limited to the specific details of these examples. Melting points were taken with a Yanako micro melting point apparatus and uncorrected. Infrared ray absorption spectra (IR) were measured by a Shimazu infrared spectrometer (IR-470). The peak shapes are denoted as follows: vs, very strong; s, strong; m, medium; w, weak. Proton nuclear magnetic resonance spectra (NMR) were measured in CDCl₃ by a JEOL NMR spectrometer (JNM-GX270, 270MHz) unless otherwise indicated and peak positions are expressed in parts per million (ppm) downfield from tetramethylsilane. The peak shapes are denoted as follows: s, singlet; d, doublet; t, triplet; m, multiplet; br, broad.

### Example 1

### (1) Compound (1)

To a stirred suspension of glycine methyl ester hydrochloride (11.8g, 94.2mmol) in EtOH (80ml) was added Et₃N (13.1ml, *d*0.726, 94.2mmol) at room temperature. To this heterogeneous mixture was added a solution of benzaldehyde (10.0g, 94.2mmol) in EtOH (20ml) dropwise at room temperature. At the end of the addition turbidity of the reaction mixture decreased to a considerable extent. After stirring at room temperature for 2 hours, the solvent EtOH was evaporated *in vacuo*. To the residue was added half sat. NaCl aq. solution (20ml), and the mixture was extracted with AcOEt (80ml x 1). The AcOEt solution was washed with sat. NaCl aq. solution (x 1), dried (Na₂SO₄), and concentrated *in vacuo* to give crude *N*-benzylidene glycine methyl ester (15.5g, 93 %). To a stirred mixture of freshly prepared crude *N*-benzylidene glycine methyl ester (15.5g, 87.5mmol), anhydrous LiBr (11.4g, 131mmol), and molecular sieves 4A (activated powder, Aldrich, 20.0g) in dry THF (160ml) was added a solution of 1,8-diazabicyclo[5.4.0]undec-7-ene (DBU, 16.0g, 105mmol) in dry THF (20.0ml) dropwise with cooling in a dry ice-acetone bath under an atmosphere of N₂. To the resulting orange reaction mixture was added a solution of methyl (E)-4-benzyloxybutenoate (15.7g, 76.1mmol, known compound in the literatures: (a) Villieras, J.; Rambaud, M.; Graff, M.; *Tetrahedron Lett., 26,* 53 (1985), (b) Villieras, J.; Rambaud, M.; Synthesis 300 (1983) and (c) Solladi , G.; Frechou, C.; Hutt, J.; Demailly, G.; *Bull. Soc. Chim. Fr.,* 827, (1987)) in dry THF (20ml) dropwise with dry ice-acetone cooling, during which the orange color vanished. The white reaction mixture was stirred under the same cooling conditions for an hour, and then at room temperature for additional 30 minutes; at this point, the reaction mixture became yellow, and methyl (E)-4-benzyloxybutenoate was consumed completely. The reaction mixture was re-cooled in the dry ice acetone bath, and sat. NH₄Cl aq. solution (50ml) was added. After adding Celite, the mixture was filtered through a pad of Celite, and the filter cake was washed with AcOEt (50ml x 3). The organic layer was separated from the combined filtrate and washings, and the aqueous layer was extracted with AcOEt (30ml x 3). The combined organic layer and AcOEt extracts were washed with sat. NaCl aq. solution (x 2), dried (Na₂SO₄), and concentrated *in vacuo* to give a pale yellow oil (31.8g). This was chromatographed over silica gel (SiO₂, Merck Kieselgel 60, 250g). Elution with *n*-hexane-AcOEt(7:1-> 5:1) gave practically pure title compound (19.8g, 68%) as a pale yellow viscous oil: IR ₘₐₓ (film): 3350(w), 1735(vs), 1605(w), 1490(m), 1205(s), 1170(s), 750(s), 700(vs) cm⁻¹; ¹H-NMR δ: 7.48-7.18(m, 10H), 4.60(d, *J*=8.1 Hz, 1H), 4.56(s, 2H), 3.89(d, *J*=7.7 Hz, 1H), 3.79(s, 3H), 3.67(d, *J*=4.8 Hz, 2H), 3.37(dd, *J*=8.1, 5.5 Hz, 1H), 3.20(s, 3H), 2.96(ddt, *J*=5.5, 7.7, 4.8 Hz, 1H) ppm.

### (2) Compound (2)

To a stirred and ice-cooled solution of Compound (1) (42.27g, 0.11mol) in anhydrous MeOH (Dojindo, 350ml) was added NaBH₄ (12.51g, 0.33mol) portionwise. The turbid reaction mixture was stirred with ice-cooling for 2 hours, and then at room temperature for 4.5 hours. The reaction mixture was re-cooled in the ice bath, and Hydrogen Chloride-Methanol Reagent 10 (Tokyo Kasei) was added until the mixture became acidic (pH 1-2). This was concentrated *in vacuo*, and the syrupy residue was basified to pH ca. 10 by adding 10% NaOH aq. solution. The mixture was extracted with AcOEt (80ml x 5). The combined AcOEt extracts were washed with sat. NaCl aq. solution (x 1), dried (Na₂SO₄), and concentrated *in vacuo* to give crude title compound (37.0g, 95%) as a viscous pale yellow oil: IR νₘₐₓ (film): 3330(s), 1730(vs), 1605(w), 1595(w), 1495(s), 1170(s), 750(s), 700(s) cm⁻¹; ¹H-NMR δ: 7.45-7.16(m, 10H), 4.58(d, *J*=8.3 Hz, 1H), 4.55(s, 2H), 3.85(dd, *J*=11.2, 4.6 Hz, 1H), 3.79(dd, *J*=11.2, 3.9 Hz, 1H), 3.58(dd, *J*=9.2, 5.3 Hz, 1H), 3,52(dd, *J*=9.2, 6.6 Hz, 1H), 3.35-3.25(m, 1H), 3.15(s, 3H), 3.14(dd, *J*=8.3, 5.7 Hz, 1H), 2.17(br.s, 2H) ppm. This was employed in the next step for salt formation without further purification.

### (3) Compound 3

To a solution of crude Compound (2) (38.0g, 0.11mol) in EtOH (120ml) was added a warmed solution of fumaric acid (6.21g, 0.054mol) in EtOH (60ml); on addition, precipitation of white solids took place immediately. To this was added EtOH (80ml) and Et₂O (60ml), and the mixture was left to stand in a refrigirator at 4 C overnight. The white solids were collected by filtration under suction, washed with Et₂O (x 1), and dried *in vacuo* at a temperature of 60 C for 2 days to give the title semifumarate (35.9g, 81%) as white powders. The combined filtrate and washings were concentrated *in vacuo* to give a pale yellow foam (8.08g), which was crystallized from EtOH-Et₂O to give a second crop of the title compound (1.42g, 3.2%): a total yield of the title compound, 37.3g (84%); mp 154-156 C: IR ₘₐₓ (nujol): 3340(s), 2800-2100(br.) 1730(vs) 1130(s), 750(s), 700(s), 675(s) cm⁻¹; ¹H-NMR δ (DMSO-*d*₆): 7.45-7.15(m, 10H), 6.60(s, 1H), 4.50(d, *J*=9.0 Hz, 1H), 4.48(s, 2H), 3.80-3.35(m, 7H), 3.15(dd, *J*=8.8, 6.2 Hz, 1H), 3.03(s, 3H), 2.99(dd, *J*=9.0, 5.2 Hz, 1H), 2.60-2.38(m, 1H) ppm.

### (4) Compound (4)

To a stirred and ice-cooled suspension of Compound (3) (12.0g, 29.0mmol) in 1M NaOH aqueous solution (70.0ml, 70.0mmol) and DME (40ml) was added benzyl chloroformate (Cbz-Cl, 4.97ml, *d*1.195, 34.8mmol) rapidly. After the heterogeneous white reaction mixture was stirred with ice-cooling for an hour, H₂O (40ml) and AcOEt(40ml) was added to dissolve the white solids, and then a further amount of Cbz-Cl (1.0ml, *d*1.195, 7.0mmol) was added to the mixture at room temperature; on the addition, the reaction mixture became neutral. After stirring at room temperature for 30 minutes, the reaction went to almost completion. The stirring was continued at room temperature overnight, and then the layers were separated. The aqueous layer was extracted with AcOEt (50ml x 4). The combined organic layer and AcOEt extracts were washed with 0.5M NaOH aq. solution (x 2), half sat. NaCl aq. solution (x 1), 0.5M HCl aq. solution (x 1), sat. NaCl aq. solution (x 1), sat. NaHCO₃ aq. solution (x 1) and sat. NaCl aq. solution (x 1), dried (MgSO₄), and concentrated *in vacuo* to give crude title compound (15.9g, quant.) as an almost colorless viscous oil: IR ₘₐₓ (film): 3420(s), 1738(vs), 1698(vs), 1680(vs), 1605(w), 1595(w), 1492(s), 1280(s), 1210(s), 1075(s), 758(s), 699(vs) cm⁻¹; ¹H-NMR δ: 7.53-7.10(m, 14H), 6.94(br.s, 1H), 5.30(d, *J*=9.5 Hz, 1H), 5.02(br.s, 2H), 4.53(d, *J*=12.1 Hz, 1H), 4.47(d, *J*=12.1 Hz, 1H), 4.13-3.90(m, 3H), 3.62(dd, *J*=9.7, 3.5 Hz, 1H), 3.58-3.41(m, 1H), 3.53(dd, *J*=9.7, 4.6 Hz, 1H), 3.29(s, 3H), 2.73(br.s, 1H), 1.61(br.s, O*H*, 1H) ppm. This was employed in the next step without further purification.

### (5) Compound (5)

To a stirred and ice-cooled solution of crude Compound (4) (15.9g, ca.29.0mmol) and imidazole (Im, 7.90g, 116mmol) in DMF (32.0ml) was added *t*-butyldimethylsilyl chloride (8.74g, 58.0mmol)in one portion. The cooling bath was removed immediately after the addition, and the stirring was continued at room temperature for ca.5 hours. The reaction mixture was diluted with PhMe-AcOEt (2:1, 300ml), washed with H₂O (x 3), and sat. NaCl aq. solution (x 2), dried (MgSO₄), and concentrated *in vacuo* to give a colorless oil (20.5g). This was chromatographed over SiO₂ (Merck Kieselgel 60, 200g). Elution with *n*-hexane-AcOEt (50:1->20:1->15:1) gave title compound (16.8g, 96%) as a colorless viscous oil: IR νₘₐₓ (film): 1743(s), 1705(vs), 1495(m), 1100(s), 780(s), 745(s), 737(s), 700(s) cm⁻¹; ¹H-NMR δ: 7.48-7.37(m, 3H), 7.37-7.14(m, 11H), 6.90(br.s, 1H), 5.27(br.d, *J*=6.1 Hz, 1H), 5.02(br.s, 2H), 4.51-4.41(m, 1H), 4.47(s, 2H), 3.97(dd, *J*=10.3, 2.6 Hz, 1H), 3.94-3.83(m, 1H), 3.60(d, *J*=3.7 Hz, 2H), 3.54(dd, *J*=11.8, 9.5 Hz, 1H), 3.33-3.15(m,1H), 3.28(s, 3H), 0.90(br.s, 9H), 0.05(br.s, 6H) ppm.

### (6) Compound (6)

Under an atmosphere of N₂, a 2.0M solution of Me₃Al in toluene (PhMe)(Aldrich, 150ml, 300mmol) was added dropwise to a stirred and ice-cooled suspension of NH₄Cl (14.9g, 278mmol) in dry PhMe (280ml). The stirring at room temperature for 30 minutes gave rise to the clear and homogeneous reaction mixture. To this was added a solution of Compound (5) (16.8g, 27.8mmol) in dry PhMe (30ml) dropwise. The resultant turbid reaction mixture was stirred and heated at an inner temperature of 50 C for 24 hours. H₂O (20ml) was added cautiuosly to the ice-cooled and stirred reaction mixture. To this was then added a 1.0M potassium sodium tartrate aqueous solution (280ml, 280mmol), and the stirring was continued under the same cooling conditions for an hour. The mixture was filtered through a pad of Celite, and the filter cake was washed with AcOEt throughly. From the combined filtrate and AcOEt washings the organic layer was separated, and the aqueous layer was extracted with AcOEt (100ml x 3). The combined organic layer and AcOEt extracts were washed with sat. NaCl aq. solution (x 1), dried (MgSO₄), and concentrated *in vacuo* to give a foam (15.4g). This was chromaotgraphed over SiO₂ (Merck Kieselgel 60, 160g). Elution with *n*-hexane-AcOEt (6:1->4:1-> 1:1) gave pure title compound (12.0g, 73%) as a white glass: IR ₘₐₓ (film): 3350(m), 3200(m), 1699(vs), 1680(vs), 1605(m), 1490(m), 1100(s), 780(s), 730(s), 697(s) cm⁻¹; ¹H-NMR δ: 7.53-7.40(m, 3H), 7.40-7.03(m, 12H), 5.60(br.s, 1H), 5.27(d, *J*=9.2 Hz, 1H), 5.03(br.s, 2H), 4.48(br.s, 2H), 4.47(dd, *J*=10.3, 8.1 Hz, 1H), 4.27(br.s, 1H), 3.99(dd, *J*=10.3, 2.9 Hz, 1H), 3.88-3.76(m, 1H), 3.70(dd, *J*=10.3, 3.3 Hz, 1H), 3.60(dd, *J*=10.3, 4.7Hz, 1H), 3.32(dd, *J*=11.7, 9.2 Hz, 1H), 3.24-3.08(m, 1H), 0.90(br.s, 9H), 0.05(br.s, 6H) ppm.

### (7) Compound (7)

To a warmed and stirred solution of Compound (6) (12.6g, 21.3mmol) in *t*-BuOH (200ml) was added Pb(OAc)₄ (16. 1g, 36.3mmol) in one portion.⁷⁾ The resulting brown mixture was stirred and heated at a gentle reflux for 45 minutes. The mixture was ice-cooled, neutralized with sat. NaHCO₃ aq. solution, and then filtered through a pad of Celite. The filter cake was washed with CH₂Cl₂. The combined filtrate and washings were concentrated *in vacuo*. The aqueous residue was extracted with CH₂Cl₂ (50ml x 3). The combined CH₂Cl₂ extracts were washed with sat. NaCl aq. solution (x 1), dried (MgSO₄), and concentrated *in vacuo* to give title compound (14.5g, quant.) as a pale yellow oil: IR ₘₐₓ (film): 1715(vs), 1700(vs), 1683(vs), 778(s), 738(s), 698(s) cm⁻¹; ¹H-NMR δ: 5.13(br.d, *J*=7.7 Hz, 1H; NC*H*₂Ph), 4.51(br.s, 2H; OC*H*₂Ph), 1.25[s, 9H; NHCO₂C(C*H*₃)₃], 0.92[s, 9H; OSiC(C*H*₃)₃], 0.10[br.s, 6H; OSi(C*H*₃)₂] ppm. This was employed in the next step without further purification.

### (8) Compound (8)

To a stirred and ice-cooled solution of Compound (7) (14.5g, ca.21.3mmol) in dry THF (20ml) was added a mixture of glacial AcOH (1.59ml, *d*1.049, 27.7mmol) and a 1.0M solution of (*n*-Bu)₄NF in THF (Aldrich, 32.0ml, 32.0mmol)dropwise. After stirring at room temperature for 6 hours, a further amount of a 1.0M solution of (*n*-Bu)₄NF in THF (15.0ml, 15.0mmol) was added dropwise at room temperature. After the stirring was continued for 2 days, the resultant orange mixture was neutralized with sat. NaHCO₃ aq. solution, and concentrated *in vacuo* at a bath temperature of 40 C. The residue was diluted with AcOEt (200ml), washed with H₂O (x 2), sat. NaHCO₃ aq. solution (x 1), and sat. NaCl aq. solution (x 1), dried (MgSO₄), and concentrated *in vacuo* to give a dark red viscous oil (13.9g). This was chromaotgraphed over SiO₂ (Merck Kieselgel 60, 130g). Elution with *n*-hexane-AcOEt (20:1->10:1->5:1->4:1->2:1) gave a red solid (10.6g). This was recrystallized from AcOEt-*n*-hexane; the crystals were filtered off, washed with AcOEt-*n*-hexane (1:4, x 1), and dried *in vacuo* to give the title compound (9.0g, 78%) as a slightly purple crystals. A portion of it was recrystallized from AcOEt-*n*-hexane to afford an analytical sample of the title compound: mp 126.5-129.5 C (white fine needles); IR ₘₐₓ (nujol): 3430(s), 3380(s), 1690(vs), 1518(s), 1498(m), 1100(s), 785(m), 770(s), 747(s), 740(s), 715(s), 695(s) cm⁻¹; ¹H-NMR δ: 7.45-7.15(m, 17H), 6.94(br.s, 1H), 5.22(d, *J*=8.1 Hz, 1H), 5.02(br.s, 2H), 4.87(br.s, 1H), 4.52(s, 2H), 4.30(dd, *J*=19.0, 9.0 Hz, 1H), 4.20-4.00(m, 2H), 4.03-3.90(m, 1H), 3.68-3.50(m, 2H), 2.10-1.85(m, 1H), 1.37(br.s, 9H) ppm.

### (9) Compound (9)

To a stirred mixture of Compound (8) (9.00g, 16.5mmol), Ph₃P (10.8g, 41.3mmol) and Im (2.81g, 41.3mmol) in dry PhMe (240ml) was added I₂ (8.38g, 33.0mmol) in one portion at room temperature. After stirring and heating at a gentle reflux for an hour, the dark brown mixture was allowed to cool to room temperature, and then sat. Na₂SO₃ aq. solution (70ml) was added until the dark color due to the excess I₂ was discharged. The layers were separated, and the organic layer was washed with sat. NaCl aq. solution (x 1), dried (MgSO₄), and concentrated *in vacuo* to give a deep red oil (22.9g). This was chromaotgraphed over SiO₂ (Merck Kieselgel 60, 230g). Elution with *n*-hexane-AcOEt (30:1->10:1->6:1) gave the title compound (9.95g, 92%) as a white solid. A portion of it (105mg) was recrystallized from isopropyl ether (IPE) to afford an analytical sample of the title compound as white powders (mp 107.5-108.5 C) ; IR ₘₐₓ (nujol): 3440(s), 1705(vs), 1500(s), 1165(s), 780(m), 770(m), 755(m), 740(m), 707(s), 695(s) cm⁻¹; ¹H-NMR δ: 7.60-7.15(m, 14H), 7.09(br.s, 2H), 5.19(d, *J*=8.8 Hz, 1H), 5.03(br.s, 2H), 4.54(s, 2H), 4.44(ddd, *J*=9.0, 9.0, 9.0 Hz, 1H), 4.15-3.97(m, 1H), 3.97-3.75(m, 2H), 3.71(dd, *J*=9.7, 5.3 Hz, 1H), 3.64(dd, J=9.7, 5.3 Hz, 1H), 2.34-2.18(m, 1H), 1.36(br.s, 9H) ppm.

### (10) Compound (10)

A mixture of Compound (9) (9.85g, 15.0mml), Et₃N (5.23ml, *d*0.726, 37.5mmol), and 10% Pd-C (1.83g) in EtOH-AcOEt (2:1, 210ml) was stirred under a slightly positive pressure of H₂ (baloon) at room temperature for 5 hours, during which the white solids of Et₃NH⁺I⁻ precipitated. The catalysts and white precipitates were filtered off by the aid of a Celite pad, and washed with AcOEt followed by CH₂Cl₂ throughly. The combined filtrate and washings were concentrated *in vacuo*. The residue was diluted with PhMe-AcOEt (1:1, 200ml), washed with sat. Na₂SO₃ aq. solution (x 3), H₂O (x 2), and sat. NaCl aq. solution (x 1), dried (MgSO₄), and concentrated *in vacuo* to give the title compound (7.60g, 95%) as slightly yellow solids. This was employed in the next step without further purification. A portion of it (0.15g) was recrystallized from AcOEt-*n*-hexane to give an analytical sample of the title compound (0.11g, 65%) as white fine needles (mp 143-144 C): IR ₘₐₓ (nujol): 3380(s), 1710(s), 1688(s), 1520(s), 1500(w), 770(m), 738(s), 715(s), 698(s) cm⁻¹; ¹H-NMR δ: 7.50-7.17(m, 14H), 7.12-6.93(m,1H), 5.25(d, *J*=8.4 Hz, 1H), 5.04(br.s, 2H), 4.49(br.s, 2H), 4.26(ddd, *J*=11.3, 9.0, 9.0 Hz, 1H), 4.16-4.00(m, 1H), 3.95-3.77(m, 1H), 3.55(br.d, *J*=4.8 Hz, 2H), 1.97-1.80(m, 1H), 1.63(br.d, *J*=4.4 Hz, 3H), 1.40(br.s, 9H) ppm.

### (11) Compound (11)

To a stirred solution of Compound (10) (0.60g, 1.13mmol) in AcOEt (13.0 ml) was added conc. HCl aq. (8.0ml) at room temperature; on the addition, evolution of CO₂ gas was observed. After stirring at room temperature for 30 minutes, the reaction mixture was concentrated *in vacuo*. The residue was basified with 10% NaOH aq. solution to pH 10-11, and the mixture was extracted with CH₂Cl₂. The combined CH₂Cl₂ extracts were washed with sat. NaCl aq. solution, dried (K₂CO₃), and concentrated *in vacuo* to give crude title compound (0.51g, quant.) as a yellow viscous oil: IR ₘₐₓ (nujol): 3380(w), 3330(w), 1698(s), 1495(m), 1110(m), 738(s), 698(s) cm⁻¹; ¹H-NMR δ: 7.60-7.15(m, 9H), 7.02(br.s, 1H), 5.04(br.s, 2H), 5.01(d, *J*=8.3 Hz, 1H), 4.52(s, 2H), 3.83(dq, *J*=9.2, 5.9 Hz, 1H), 3.61(d, *J*=4.8 Hz, 2H), 3.51(dd, *J*=11.0, 8.3 Hz, 1H), 1.86-1.67(m, 1H), 1.60(d, *J*=5.9 Hz, 3H), 1.45-1.07(br.m, 3H; N*H*₂ + O*H*) ppm. This was employed in the next step without further purification.

### (12) Compound (12)

To a stirred solution of crude Compound (11) (0.51g, 1.13mmol) in dry CH₂Cl₂ (12ml) was added a solution of 5-isopropyl-2-methoxybenzaldehyde (0.25g, 1.41mmol) in dry CH₂Cl₂ (2ml) at room temperature. To this solution was added NaBH(OAc)₃ (0.36g, 1.70mmol) portionwise at room temperature. The added NaBH(OAc)₃ dissolved gradually with stirring at room temperature. After 3 hours the slightly turbid reaction mixture was basified to pH 10-11 with 10% NaOH aq. solution, and the mixture was separated. The aqueous layer was extracted with CH₂Cl₂ (x 3). The combined CH₂Cl₂ solution was washed with sat. NaCl aq. solution (x 1), dried (K₂CO₃), and concentrated *in vacuo* to give a viscous oil (0.79g). This was subjected to SiO₂ chromaotgraphy (Merck Kieselgel 60, 8.0g) eluted with CH₂Cl₂-MeOH (300:1) to give title compound (0.16g, 24%) as a viscous colorless oil and an oily mixture (0.47g) of 5-isopropyl-2-methoxybenzaldehyde and title compound. The latter mixture was chromatographed again over SiO₂ (Merck Kieselgel 60, 7.0g). Elution with *n*-hexane-AcOEt (30: 1) followed by CH₂Cl₂-MeOH (100: 1) gave an additional amount of the title compound (0.45g, 67%); the total yield of the title compound amounted to 0.61g (91 %): IR ₘₐₓ (film): 3350(w), 1700(vs), 1610(w), 1497(s), 1250(s), 1110(s), 815(m), 772(m), 735(s), 698(s) cm⁻¹; ¹H-NMR δ: 7.50-7.11(m, 14H), 7.02(dd, *J*=8.2, 2.2 Hz, 1H), 7.01(br.s, 1H), 6.92(d, *J*=2.2 Hz, 1H), 6.70(d, *J*=8.2 Hz, 1H), 5.20-4.93(m, 3H), 3.95-3.78(m,1H), 3.78-3.49(m, 4H), 3.63(s, 3H), 3.33(dd, *J=*10.3, 8.0 Hz), 2.77 (sep, *J*=7.0 Hz, 1H), 2.11-1.95(m, 1H), 1.58(br.s, 1H; N*H*), 1.49(br.s, 3H), 1.17(d, *J*=7.0 Hz, 6H) ppm.

### (13) Compound (13)

20% Pd(OH)₂-C (Pearlman's catalyst, Aldrich; 0.33g) was added to a solution of Compound (12) (0.61g, 1.03mmol)andHCO₂NH₄ (0.39g, 6.18mmol) in MeOH (20ml). The mixture was stirred and heated at a gentle reflux for 30 minutes. The catalysts were filtered off by the aid of a Celite pad, and washed with MeOH. The combined filtrate and washings were concentrated *in vacuo*. To the residue was added 1M NaOH aq. solution until the mixture became basic (pH 10-11). This was extracted with CH₂Cl₂ (x 4). The combined CH₂Cl₂ extracts were washed with sat. NaCl aq. solution (x 1), dried (K₂CO₃), and concentrated *in vacuo* to give a pale yellow oil (0.47g). This was chromatographed over SiO₂ (Merck Kieselgel 60, 7.0g). Elution with CH₂Cl₂-MeOH (300:1->200:1->100->60:1-> 50:1) gave title compound (0.40g, 85%) as a pale yellow viscous oil: IR ₘₐₓ (film): 3340(w), 1610(m), 1498(s), 1250(s), 1110(s), 810(m), 738(s), 700(s) cm⁻¹; ¹H-NMR δ: 7.43-7.20(m, 10H), 7.01(dd, *J*=8.4, 2.2 Hz, 1H), 6.82(d, *J*=2.2 Hz, 1H), 6.65(d, *J*=8.4 Hz, 1H), 4.56(s, 2H), 4.20(d, *J*=5.5 Hz, 1H), 3.67(d, *J*=13.6 Hz, 1H), 3.493(dd, *J*=9.3, 6.2 Hz, 1H), 3,489(s, 3H), 3.57(dd, *J*=9.3, 6.4 Hz, 1H), 3.42(d, *J*=13.6 Hz, 1H), 3.13(dd, *J*=5.5, 2.6 Hz, 1H), 3.06(dq, *J*=6.2, 6.2 Hz, 1H), 2.74(sep, *J*=7.0 Hz, 1H), 2.17-1.85(m, 3H), 1.42(d, *J*=6.2 Hz, 3H), 1.16(d, J=7.0Hz, 6H) ppm.

### (14) Compound (14)

An excess amount of Hydrogen Chloride, Methanol Reagent 10 (Tokyo Kasei) was added to Compound (13) (0.40g, 0.87mmol) until the mixture became acidic. The solvent MeOH was evaporated *in vacuo*, and the syrupy residuue was crystallized from EtOH-Et₂O to give title compound (0.40g, 87%) as white powders (mp 129-133 C): IR ₘₐₓ (nujol): 3450(w), 3100-2100(br.s), 1510(s), 1255(s), 1087(m), 810(w), 755(m), 700(s) cm⁻¹; ¹H-NMR δ (DMSO-*d*₆): 7.83(br.s, 2H), 7.62-7.40(m, 3H), 7.40-7.33(m, 4H), 7.37-7.25(m, 1H), 7.25-7.12(m, 2H), 6.89(d, *J*=8.4 Hz, 1H), 5.11(br.s, 1H), 4.59(s, 2H), 3.78-3.50(m, 5H), 3.67(br.s, 2H), 3.45-3.30[m, 3H; overlaped with the signals (δ 3.35) due to H₂O], 3.35[s, 3H; overlaped with the signals (δ 3.35) due to H₂O], 2.77(sep, *J*=7.0 Hz, 1H), 2.55-2.48[m, 1H; overlaped with the signals (δ 2.50) due to DMSO], 1.64(br.d, *J*=5.9 Hz, 3H), 1.14(d, *J*=7.0 Hz, 6H) ppm.

### (15) Compound (15)

Na (1.88g, 81.8mmol) was added portionwise to liq. NH₃ (ca.250ml) with stirring and cooling in a dry ice-acetone bath. To the resulting deep blue solution was added dropwise a solution of Compound (10) 5.90g, 11.1mmol) in dry THF (50ml). After stirring with dry ice-acetone cooling for 2 hours the cooling bath was removed, and the stirring was continued under the NH₃-refluxing conditions for 30 minutes, during which the deep blue color persisted. The reaction mixture was cooled with dry ice-acetone, and solid NH₄Cl was added until the deep blue color was discharged. After the solvent NH₃ was evaporated, the white solid residue was extracted with CH₂Cl₂. The combined CH₂Cl₂ extracts were dried (K₂CO₃), and concentrated *in vacuo* to give a mixture (4.55g) of the title compound, Compound (10), and 1,2-diphenylethane in an approximate ratio of 1:0.085:0.5; the ratio was estimated by ¹H-NMR spectroscopy: δ 3.01 ppm (dq, *J*=8.8, 6.0 Hz, 1H; NC*H*Me for the title compound); δ 4.49 ppm (s, 0.17H; CH₂OCH₂Ph for Compound (10); δ 2.92 ppm [s, 2H; Ph(C*H*₂)₂Ph]. A solution of this crude product (4.55g) in dry THF (30ml) was added to a deep blue solution of Na (1.20g, 52.2mmol) in liq. NH₃ (ca.200ml) dropwise with stirring and dry ice-acetone cooling. The reaction mixture was stirred under the same cooling conditions for 2 hours, and then under the NH₃-refluxing conditions for 30 minutes. The deep blue color was discharged dy adding solid NH₄Cl with dry ice-acetone cooling. After evaporating the solvent NH₃, the solid residue was extracted with CH₂Cl₂. The combined CH₂Cl₂ extracts were dried (K₂CO₃), and concentrated *in vacuo* to give a 7:3 mixture (3.84g) of the title compound and 1,2-diphenylethane as a pale yellow viscous oil, the ratio being estimated also by ¹H-NMR spectroscopy; the net yield of title compound was calculated to be 90% based on the above ratio taken into account: IR ₘₐₓ (film): 3390(s), 1700(vs), 1685(vs), 1603(w), 1522(s), 1510(s), 1497(s),1170(s), 768(w), 738(w), 698(s) cm⁻¹; ¹H-NMR δ: title compound, 7.43-7.30(m, 5H), 4.40(d, *J*=7.4 Hz, 1H), 4.38(br.d, *J*=7.4 Hz, 1H; N*H*Boc), 4.08(ddd, *J*=7.4, 7.4, 5.5 Hz, 1H), 3.70-3.58(m, 2H), 3.01(dq, *J*=8.8, 6.0 Hz, 1H), 1.76-1.64(m, 1H), 1.35-1.22(m, 2H; O*H* + N*H*), 1.312(d, *J*=6.0 Hz, 3H), 1.309(s, 9H) ppm; 1,2-diphenylethane, 7.30-7,25(m, 1.8H), 7.22-7.15(m, 2.7H), 2.92(s, 1.8H) ppm. While this crude product solidified on standing at room temperature, it was employed in the next step without further purification.

### (16) Compound (16)

Benzyl chloroformate (Cbz-Cl, 1.57ml, *d*1.195, 11.0mmol) was added to a stirred and ice-ccooled mixture of crude Compound (15) (3.84g, ca.10.0mmol), and 2M NaOH aq. solution (8.0ml, 16.0mmol) in DME-THF (1:1, 14ml) . After stirring at room temperature for 2.5 hours, 2M NaOH aq. solution (2.0ml, 4.0mmol) and Cbz-Cl (0.29ml, *d*1.195, 2.0mmol) were added at room temperature, and the stirring was continued for 2 hours. The mixture was extracted with AcOEt (x 4). The combined AcOEt extracts were washed with sat. NaCl aq. solution (x 1), dried (MgSO₄), and concentrated *in vacuo* to give a white solid (5.57g). This was recrystallized from AcOEt-*n*-hexane to give pure title compound (3.33g, 76%). The yellow orange mother liquor (2.22g) was chromatographed over SiO₂ (Merck Kieselgel 60, 20g). Elution with *n*-hexane-AcOEt (10:1->5:1) give Compound (16a) (0.31g, 5.4%) as a fairly pure solid, which was recrystallied from AcOEt-*n*-hexane to give an analytical sample of Compound (16a) (0.28g, 4.9%): mp 148.0-148.5 C (white needles); IR ₘₐₓ (nujol): 3400(m), 1763(m), 1735(s), 1690(s), 1510(s), 782(m), 770(m), 740(s), 713(s), 695(s) cm⁻¹; ¹H-NMR δ: 7.44-7.13(m, 13H), 7.03(br.s, 2H), 5.22(d, *J*=8.8 Hz, 1H), 5.15(s, 2H), 5.04(br.s, 2H), 4.48-4.24(m, 2H), 4.25-4.10(m, 1H), 3.92(d, *J*=9.2 Hz, 1H), 3.84(dq, *J*=9.8, 6.1 Hz, 1H), 2.00-1.83(m, 1H), 1,63(d, *J*=4.8 Hz, 3H), 1.39(s, 9H) ppm; Further elution with *n*-hexane-AcOEt (5:1-> 1:1) gave an additional amount of title compound (0.48g) as a fairely pure solid, which was recrystallized from AcOEt-*n*-hexane to give pure title comp[ound (0.29g, 6.8%); the total yield of title compound amounted to 3.62g (82%): mp 153.5-154.0 C (white fine needles); IR ₘₐₓ (nujol): 3450(s), 3400(s), 1690vs), 1518(s), 1170(s), 772(m), 760(w), 740(m), 710(m), 700(s) cm⁻¹; ¹H-NMR δ: 7.50-7.13(m, 9H), 7.08(br.s, 1H), 5.15(d, *J*=8.4 Hz, 1H), 5.05(br.s, 2H), 4.36(ddd, *J*=11.0, 8.4, 8.4 Hz, 1H), 4.01(br.d, *J*=11.0 Hz, 1H), 3.90(dq, *J*=9.9, 6.0 Hz, 1H), 3.64(br.s, 2H), 1.65-1.45(m, 5H), 1.40(s, 9H) ppm.

### (17) Compound (17)

To a stirred solution of Compound (16a) (0.80g, 1,82mmol) in AcOEt (15ml) was added cone. HCl aq. (8.0ml) at room temperature; on the addition evolution of CO₂ gas took place. After stirring at room temperature for 1.5 hours, the reaction mixture was concentrated in *vacuo* to give a syrupy residue. This was basified to pH 10-11 with 20% NaOH aq. solution, and then extracted with AcOEt. The combined AcOEt extrtacts were washed with sat. NaCl aq. solution (x 1), dried (K₂CO₃), and concentrated *in vacuo* to give title compound (0.61g, 98%): IR ₘₐₓ (film): 3380(m), 1700(s), 1682(s), 1605(m), 1585(m), 1110(s), 750(m), 700(s) cm⁻¹; ¹H-NMR δ: 7.52-7.13(m, 9H), 6.99(br.s, 1H), 5.05(br.s, 3H), 4.92(d, *J*=8.1 Hz, 1H), 3.89(dd, *J*=10.4, 2.9 Hz, 1H), 3.76(dd, *J*=10.4, 10.4 Hz, 1H), 3.60-3.45 (m, 1H), 3.50(dd, *J*=11.2, 8.1Hz, 1H), 2.10-1.70(m, 3H), 1.61(br.d, *J*=4.3 Hz, 3H) ppm. This was employed in the next step without further purification.

### (18) Compound (18)

To a stirred solution of Compound (17) (0.61g, 1.79mmol) in dry CH₂Cl₂ (14ml) was added a solution of 5-isopropyl-2-methoxybenzaldehyde (0.40g, 2.24mmol) in dry CH₂Cl₂ (2ml) at room temperature. To this was then added NaBH(OAc)₃ (0.57g, 2.69mmol) portionwise at room temperature. The added NaBH(OAc)₃ dissolved gradually with stirring at room temperature. After stirring for 3 hours at room temperature, the reaction mixture was basified to pH 10-11 with 10% NaOH aq. solution. The mixture was separated, and the aqueous layer was extracted with CH₂Cl₂ (x 3). The combined CH₂Cl₂ solution was washed with sat. NaCl aq. solution (x 1), dried (K₂CO₃), and concentrated *in vacuo* to give a yellow oil (1.34g). This was chromaotgraphed over SiO₂ (Merck Kieselgel 60, 20g). Elution with *n*-hexane-AcOEt (30:1) followed by CH₂Cl₂-MeOH (400:1->200:1) gave title compound (0.70g, 78%) as a viscous pale yellow oil: IR ₘₐₓ (film): 3330(s), 1700(s), 1610(m), 1500(s), 1250(s), 1110(s), 1030(s), 815(s), 770(s), 738(s), 700(s) cm⁻¹; ¹H-NMR δ: 7.45-7.16(m, 9H), 7.05(dd, *J*=8.4, 1.8 Hz, 1H), 7.02(br.s, 1H), 6.88(d, *J*=1.8 Hz, 1H), 6.71(d, *J*=8.4 Hz, 1H), 5.33-4.93(m, 3H), 3.97-3.65(m, 3H), 3.81(d, *J*=12.1 Hz, 1H), 3.72(d, *J*=12.1 Hz, 1H), 3.67(s, 3H), 3.53-3.35(m, 1H), 3.38(dd, *J*=11.5, 7.9 Hz), 2.79 (sep, *J*=7.0 Hz, 1H), 2.17-1.98(m, 1H), 1.56(br.s, 4H), 1.18(d, *J*=7.0 Hz, 6H) ppm.

### (19) Compound (19)

20% Pd(OH)₂-C (Pearlman's catalyst, Aldrich; 0.44g) was added to a solution of Compound (18) (0.70g, 1.36mmol) and HCO₂NH₄ (0.51g, 8.16mmol) in MeOH (20ml). The reaction mixture was stirred and heated at a gentle reflux for 30 minutes. The catalysts were filtered off by the aid of a Celite pad, and washed with MeOH. The combined filtrate and washings were concentrated *in vacuo*. The residue was basified to pH 10 with 1M NaOH aq. solution, and extracted with AcOEt (x 4). The combined AcOEt extracts were dried (K₂CO₃), and concentrated *in vacuo* to give a pale yellow viscous oil (0.46g), which crystallized spontaneously on standing in a refrigirator for 2 days. This was recrystallized from AcOEt-*n*-hexane to give title compound (0.33g, 63%) as white crystals. The yellow mother liquor was chromatographed over SiO₂ (Merck Kieselgel 60, 2.0g). Elution with CH₂Cl₂-MeOH (50:1->20:1) gave an additional amount of title compound (0.06g, 12%) as a pale yellow viscous oil, which crystallized spontaneously on standing at room temperature; the total yield of title compound amounted to 0.39g (76%): mp 76.0-78.0 C (white powders); IR ₘₐₓ (nujol): 3180(w), 1610(w), 1509(s), 1240(s), 1050(m), 800(m), 750(m), 696(m) cm⁻¹; ¹H-NMR δ: 7.47-7.22(m, 5H), 7.01(dd, *J*=8.4, 2.2 Hz, 1H), 6.68(d, *J*=8.4 Hz, 1H), 6.65(d, *J*=2.2 Hz, 1H), 4.50(d, *J*=7.7 Hz, 1H), 3.78(dd, *J*=10.3, 4.3 Hz, 1H), 3.66(dd, *J*=10.3, 9.4 Hz, 1H), 3.64(s, 3H), 3.61(d, *J=*12.3 Hz, 1H), 3.47(d, *J=*12.3 Hz, 1H), 3.39(dd, *J*=9.7, 7.7 Hz, 1H), 2.92(dq, *J*=9.4, 6.0 Hz, 1H), 2.76(sep, *J*=6.8 Hz, 1H), 2.13(br.s, 3H), 1.84(ddd, *J*=9.4, 9.4, 4.3 Hz, 1H), 1.27(d, *J*=6.0 Hz, 3H), 1.16(d, *J*=6.8 Hz, 6H) ppm.

### (20) Compound (20)

Fumaric acid (94.6 mg, 0.81mmol) was added to a solution of Compound (19) (0.31g, 0.81mmol) in EtOH to give the clear solution, and the solvent EtOH was evaporated *in vacuo*. The residual solid was recrystallized from EtOH-AcOEt; after keeping in a freezer overnight, the white crystals were filtered off, washed with Et₂O-EtOH (20:1), and dried *in vacuo* at 40 C to afford the title fumarate (0.34g, 83%) as white powders (mp 161.0-162.5 C): IR ₘₐₓ (nujol): 3300(m), 3000-2200(br.,m), 1680(s), 1625(s), 1505(s), 1240(m), 1038(s), 820(s), 770(s), 750(s), 740(m), 700(s) cm⁻¹; ¹H-NMR δ (DMSO-d₆): 7.50-7.26(m, 5H), 7.01(dd, *J*=8.4, 1.8 Hz, 1H), 6.87(d, *J*=1.8 Hz, 1H), 6.76(d, *J*=8.4 Hz, 1H), 6.47(s, 2H), 4.45(d, *J*=5.5 Hz, 1H), 4.10-3.30(m, 7H), 3.51(s, 3H), 3.34-3.17(m, 2H), 3.17-3.06(m, 1H), 2.74(sep, *J*=7.0 Hz, 1H), 1.97-1.83(m, 1H), 1.36(d, *J*=6.2 Hz, 3H), 1.12(d, *J*=7.0 Hz, 6H) ppm.

### (21) Compound (21)

To a stirred and water-cooled solution of Compound (16) (2.60g, 5.90mmol) and Et₃N (5.70ml, *d*0.726, 40.9mmol) in DMSO (dried over molecular sieves 4 , 36ml) was added sulfer trioxide pyridine complex (SO₃ Py, Wako, 5.42g, 34.1mmol) portionwise. Just after the addition the water bath was removed, and the stirring was continued at room temperature for 1.5 hours. The reaction mixture was poured into a mixture of ice and water (80ml). The mixture was extracted with CH₂Cl₂ (30ml x 5). The combined CH₂Cl₂ extracts were washed with 10% citric acid aq. solution (30ml x 4) until the aqueous washing reached the acidity of citric acid (pH ca.3), H₂O (30ml x 1), sat. NaHCO₃ aq. solution (40ml x 1), and sat. NaCl aq. solution (x 1), dried (MgSO₄), and concentrated *in vacuo* to give crude title compound (3.66g, quant.) as a pale yellow viscous oil; while the yield was overestimated owing to contamination with the solvent employed, this crude product solidified on standing in a refrigirator: IR ₘₐₓ (film): 3400(m, shoulder), 3350(m), 2720(w), 1710(vs), 1690(vs, shoulder), 1605(w), 1585(w), 1520(s), 1500(s), 1165(s), 770(m), 730(s), 700(s) cm⁻¹; ¹H-NMR δ: 9.65(d, J=3.7 Hz, 1H), 7.40-7.10(m, 9H), 7.05(br.s, 1H), 5.27(d, *J*=8.5 Hz, 1H), 5.06(br.s, 2H), 4.78-4.57(m, 1H), 4.24(dq, J=9.5, 6.1 Hz, 1H), 4.05(br.d, J=9.2 Hz, 1H), 2.77-2.50(m, 1H), 1.61(d, *J*=6.1 Hz, 1H), 1.37(s, 9H) ppm. This was employed in the next step without further purification.

### (22) Compound (22)

To a stirred and ice-cooled mixture of crude Compound (21) (3.66g, 5.90mmol) and NaHCO₃ (19.8g, 0.24mol) in MeOH-H₂O (9:1 by volume; 30ml) was added a 2.0M solution of Br₂ in MeOH-H₂O (9:1; 30ml, 60mmol) dropwise. The reaction mixture was stirred at room temperature for 2 hours, during which the color changed from deep red to orange. Sat. Na₂S₂O₃ (hypo) aq. solution was added dropwise to the mixture with ice-ccoling, until the orange color due to the excess Br₂ was discharged. The mixture was filtered through a pad of Celite, and the filter cake was washed with CH₂Cl₂. The combined filtrate and washings were concentrated *in vacuo*. The syrupy residue was diluted with AcOEt-PhMe (2:1, 120ml), washed with sat. Na₂S₂O₃ aq. solution (x 2), sat. NaHCO₃ aq. solution (x 1), and sat. NaCl aq. solution, dried (MgSO₄), and concentrated *in vacuo* to give a white solid (2.96g). This was recrystallized from AcOEt-*n*-hexane to give title compound (2.44g, 88% in 2 steps from Compound (16) as white powders. mp 147-148 C; IR ₘₐₓ (nujol): 3310(s), 1728(s), 1701(s, shoulder), 1690(vs), 1537(s), 1535(s), 1280(m), 1165(m), 750(m), 700(s) cm⁻¹; ¹H-NMR δ: 7.52-7.15(m, 9H), 7.05(br.s, 1H), 5.28(br.d, *J*=8.1 Hz, 1H), 5.05(br.s, 2H), 4.60(ddd, *J*=11.7, 8.8, 8.1 Hz, 1H), 4.35-4.17(m, 1H), 4.02(br.d, *J*=8.8 Hz, 1H), 3.71(s, 3H), 2.58(dd, *J*=11.7, 9.9 Hz, 1H), 1.62(br.d, *J*=5.5 Hz, 3H), 1.39(s, 9H) ppm.

### (23) Compound (23)

Hydrogen chloride, Methanol reagent 10 (Tokyo Kasei, 25ml) was added to Compound (22) (1.25g, 2.67mmol) at room temperature. The resulting heterogeneous mixture was stirred and heared at reflux; the reaction mixure became homogeneous as the inner temperature increased, and then white solids precipitated. After 15 minutes, the solvent MeOH was evaporated *in vacuo*. To the solid residue was added 10% NaOH aq. solution until the resulting mixture became basic (pH 10-11). This was extracted AcOEt (x 4). The combined AcOEt extracts were washed with sat. NaCl aq. solution (x 1), dried (Na₂SO₄), and concentrated *in vacuo* to give crude title compound (0.93g, 95%) as yellow viscous oil: IR ₘₐₓ (film): 3395(m), 3320(w), 1730(vs), 1700(vs), 1605(w), 1585(w), 1495(s), 1210(s), 1165(s), 1105(s), 1025(s), 745(s), 700(s) cm⁻¹; ¹H-NMR δ: 7.46-7.18(m, 9H), 7.07(br.s, 1H), 5.08(d, *J*=8.4 Hz, 1H), 5.06(br.s, 2H), 4.13(dq, J=9.7, 6.1 Hz, 1H), 3.85(dd, *J*=11.0, 8.4 Hz, 1H), 3.75(s, 3H), 2.51(dd, *J*=11.0, 9.7 Hz, 1H), 1.64(br.d, *J*=6.1 Hz, 3H), 1.27(br.s, 2H; N*H*₂) ppm. This was employed in the next step without further purification.

### (24) Compound (24)

To a stirred solution of Compound (23) (0.93g, 2.52mmol) in dry CH₂Cl₂ (20ml) was added a solution of 5-isopropyl-2-methoxybenzaldehyde (0.56g, 3.15mmol) in dry CH₂Cl₂ (5ml) at room temperature. To this was added NaBH(OAc)₃ (0.80g, 3.78mmol) portionwise at room temperature. The added NaBH(OAc)₃ dissolved gradually with stirring at room temperature. After stirring for 1.5 hours at room temperature, the reaction mixture was basified to pH 10-11 by adding 10% NaOH aq. solution with ice-cooling. The layers were separated, and the aqueous layer was extracted with CHCl₃ (x 3). The combined CH₂Cl₂ layer and CHCl₃ extracts were washed with sat. NaCl aq. solution (x 1), dried (Na₂SO₄-K₂CO₃), and concentrated *in vacuo* to give a yellow viscous oil (1.69g). This was chromaotgraphed over SiO₂ (Merck Kieselgel 60, 20g). Elution with *n*-hexane-AcOEt (30:1) followed by CH₂Cl₂-MeOH (200: 1) gave title compound (1.36g, quant.) as a pale yellow viscous oil: IR ₘₐₓ (film): 3350(w), 1735(s), 1700(vs), 1487(s), 1290(s), 1250(s), 1030(s), 810(s), 735(s), 700(s) cm⁻¹; ¹H-NMR δ: 7.50-7.15(m, 10H), 7.03(dd, *J*=8.4, 2.2 Hz, 1H), 6.96(d, *J*=2.2 Hz, 1H), 6.70(d, *J*=8.4 Hz, 1H), 5.15(br.d, *J*=7.7 Hz, 1H), 5.03(br.s, 2H), 4.22-4.06(m, 1H), 3.84-3.58(m, 3H), 3.71(s, 3H), 3.64(s, 3H), 2.90-2.58 (m, 2H), 1.61(br.d, *J*=5.5 Hz, 3H), 1.19(d, *J*=7.0 Hz, 6H) ppm.

### (25) Compound (25)

20% Pd(OH)₂-C (Pearlman's catalyst, Aldrich; 0.81g) was added to a solution of Compound (24) (1.36g, 2.57mmol) and HCO₂NH₄ (0.97g, 15.4mmol) in MeOH (30ml). The reaction mixture was stirred and heated at a gentle reflux for 30 minutes. The catalysts were filtered off by the aid of a Celite pad, and washed with MeOH. The combined filtrate and washings were concentrated *in vacuo*. The syrupy residue was basified to pH 10 with 1M NaOH aq. solution, and extracted with CH₂Cl₂ (x 3). The combined CH₂Cl₂ extracts were washed with sat. NaCl aq. solution, dried (Na₂SO₄), and concentrated *in vacuo* to give a pale yellow viscous oil (0.94g). This was chromatographed over SiO₂ (Merck Kieselgel 60, 13.0g). Elution with *n*-hexane-AcOEt (20:1-> 10:1->5:1) followed by CH₂Cl₂-MeOH (60:1) gave title compound (0.73g, 72%) as a pale yellow viscous oil: IR ₘₐₓ (film): 3330(w), 1728(s), 1610(m), 1500(s), 1250(s), 1170(s), 813(s), 765(m), 750(m), 740(m), 700(s) cm⁻¹; ¹H-NMR δ: 7.42-7.21(m, 5H), 7.02(dd, *J*=8.4, 2.2 Hz, 1H), 6.83(d, *J*=2.2 Hz, 1H), 6.66(d, *J*=8.4 Hz, 1H), 4.35(d, *J*=5.8 Hz, 1H), 3.75(s, 3H), 3.68(d, *J*=13.2 Hz, 1H), 3.56(dd, *J*=5.8, 3.1 Hz, 1H), 3.51(s, 3H), 3.48(dq, *J*=7.7, 6.2 Hz, 1H), 3.41(d, *J*=13.2 Hz, 1H), 2.79(sep, *J*=7.0 Hz, 1H), 2.60(deformed dd, *J*=7.7, 3.1 Hz, 1H), 1.77(br.s, 2H; N*H* x 2), 1.44(d, *J*=6.2 Hz, 3H), 1.19(d, *J*=7.0 Hz, 6H) ppm.

### (26) Compound (26)

Fumaric acid (58.5mg, 0.50mmol) was added to a solution of Compound (25) (0.20g, 0.50mmol) in EtOH. The resulting heteogeneous mixture was sonicated to allow complete salt formation. The crystalline salts thus generated were dissolved in a minimum volume of EtOH with heatig at reflux, and recrystallized on cooling to room temperature. After keeping in a refrigirator overnight, the white crystals were filtered off, washed with Et₂O-EtOH (20:1, x 1), dried *in vacuo* at 40 C to give the title fumarate (0.20g, 85%) as white powders. An additional amount of the title compound (0.02g, 8%) was obtained as a second crop by crystallization of the mother liquor from EtOH-Et₂O; the total yield of the title compound amounted to 0.24g (93%): mp 169.0-171.0 (white powders); IR ₘₐₓ (nujol): 3330(s), 3000-2100(br., s), 1735(s), 1710(s), 1653(m), 1603(s), 1240(s), 1178(s), 818(s), 788(m), 768(s), 743(s), 700(s) cm⁻¹ ; ¹H-NMR δ (DMSO-d₆): 7.47-7.30(m, 4H), 7.35-7.21(m, 1H), 7.00(dd, *J*=8.4, 2.0 Hz, 1H), 6.79(d, *J*=2.0 Hz, 1H), 6.76(d, *J*=8.4 Hz, 1H), 6.59(s, 2H), 4.29(d, *J*=6.2 Hz, 1H), 4.15-3.10(m, 8H), 3.67(s, 3H), 3.54(s, 3H), 2.73(sep, *J*=7.0 Hz, 1H), 2.60-2.50(m, 1H), 1.30(d, *J*=6.2 Hz, 3H), 1.12(d, *J*=7.0 Hz, 6H) ppm.

### (27) Compound (27)

A solution of Compound (26) (0.39g, 0.98mmol) in 6M HCl aq. solution (6.0ml) was stirred and heated at a gentle reflux for 30 minutes, wherein precipitation of white solids initiated ca.10 minutes after refluxing. The reaction mixture was cooled to room temperature, and then EtOH (4.0ml) was added. After the mixture was ice-cooled for 30 minutes, the white precipitates were filtered off, washed with EtOH (x 2), and dried *in vacuo* at a temperature between 40 and 50 C for 2 days to afford title compound (0.43g, 96%) as white needles. 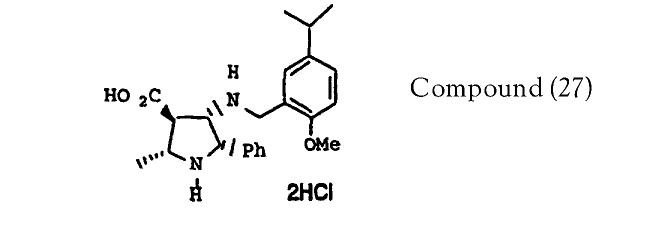 mp 159-164 C: IR ₘₐₓ (nujol): 3400(s), 3200(s), 3130(s), 2370(s), 2300(br., s), 1735(s), 1710(s), 1585(s), 1510(s), 1260(s), 1143(s), 1017(s), 830(s), 770(s), 745(s), 702(s) cm⁻¹ ; ¹H-NMR δ (DMSO-d₆): 7.90-7.73(m, 2H), 7.60-7.45(m, 3H), 7.25-7.13(m, 2H), 6.90(d, *J*=9.2 Hz, 1H), 5.35-5.15(m, 1H), 4.63-4,41(m, 1H), 4.20-4.01(m, 1H), 4.00-3.83(m, 1H), 3.80-3.15(m, 1H), 3.67(s, 3H), 2.79(sep, *J*=6.8 Hz, 1H), 2.55-2.42(m, 1H), 1.71(br.d, *J*=5.9 Hz, 3H), 1.15(d, *J*=6.8 Hz, 6H) ppm.

### Example 2

### (1) Compound (28)

To a stirred solution of the 4-methoxycarbonyl derivative of Compound (11) (793 mg, 2.15 mmol) and 2-methoxy-5-trifluoromethoxybenzaldehyde (569 mg, 2.58 mmol) in dry CH₂Cl₂ (10 ml) was added NaBH(OAc)₃ (639 mg, 3.01 mmol) portionwise at room temperature. After stirring for 2h at room temperature, the reaction mixture was basified to pH 10-11 by adding 10% NaOH aq. solution with ice-cooling. The organic layer was separated, and the aqueous layer was extracted with CH₂Cl₂ (60 ml x 4). The combined CH₂Cl₂ solution was washed with brine, dried(MgSO₄) and concentrated *in vacuo* to give crude title compound (1.32 g) as a yellow viscous oil. This was purified by column chromatography on silica gel (65 g) with hexane-ethyl acetate (3:1-2:1) to give the pure title ecompound (1.08 g, 88 %) as a pale yellow oil.
¹H-NMR δ 7.42-7.17 (m, 10 H), 7.08-6.99 (m, 2H), 6.78-6.70 (m, 1H), 5.18 (br. d, J = 7.7 Hz, 1H), 5.04 (br. s, 2H), 4.20-4.07 (m, 1H), 3.80-3.60 (m, 3H), 3.73 (s, 3H), 3.70 (s, 3H), 2.83-2.67 (m, 1H), 1.61 (d, J = 5.9 Hz, 3H), 1.50 (br. s, 1H) ppm.
IR (film, cm⁻¹) :ν 3350, 1732, 1700, 1607, 1493, 1456, 1438, 1404, 1339, 1245, 1222, 1158, 1028, 870, 810.

### (2) Compound (29) and Compound (30)

20% Pd(OH)₂-C (Pearlman's cat., 600 mg) was added to a solution of Compound (28) (1.08 g, 1.89 mmol) and HCO₂NH₄ (714 mg, 11.3 mmol) in methanol (25 ml). The reaction mixture was stirred and heated at a gentle reflux for 30 min. The catalyst was filtered off by the aid of a Celite pad, and washed with MeOH. The combined filtrate and washings were concentrated *in vacuo*.. The residue was basified to pH 10-11 with 10% NaOH aq. solution, and extracted with CH₂Cl₂ (60 ml x 4). The combined CH₂Cl₂ extracts were washed with brine, dried (MgSO₄) and concentrated *in vacuo* to give a crude mixture of Compound (29) and Compound (30) (794 mg) as a yellow oil. This was purified by column chromatography on silica gel (45 g) with hexane-ethyl acetate (1:1) to give Compound (30) (222 mg, 27.3 %) as a pale yellow solid, which was recrystallized from hexane-ethyl acetate to give Compound (30) (127 mg, 1st crop) as a white solid: mp 81-82 °C.
¹H-NMR δ 7.38-7.21 (m, 5H), 7.03 (dd, J = 2.6, 8.8 Hz, 1H), 6.90 (d, J = 2.6 Hz, 1H), 6.70 (d, J = 8.8 Hz, 1H), 4.36 (d, J = 5.9 Hz, 1H), 3.75 (s, 3H), 3.65 (d, J = 14.5 Hz, 1H), 3.56 (s, 3H), 3.58- 3.37 (m, 1H), 3.50 (dd, J = 3.3, 5.9 Hz, 1H), 3.40 (d, J = 14.5 Hz, 1H), 2.55 (dd, J = 3.3, 7.7 Hz, 1H), 1.85 (br. s, 2H), 1.43 (d, J = 6.2 Hz, 3H) ppm.
IR (KBr, cm⁻¹) :ν 3450, 1727, 1497, 1459, 1438, 1313, 1263, 1251, 1222, 1164, 1040, 885, 822.

Further elution with CH₂Cl₂-MeOH (20:1) gave Compound (29) (342 mg, 41.4 %) as a yellow oil.
¹H-NMR δ 7.40-7.25 (m, 5H), 7.02 (dd, J = 2.8, 8.7 Hz, 1H), 6.87 (d, J = 2.8 Hz, 1H), 6.69 (d, J = 8.7 Hz, 1H), 4.09 (d, J = 7.7 Hz, 1H), 3.75-3.52 (m, 4H), 3.71 (s, 3H), 3.67 (s, 3H), 2.60 (t, J = 8.1 Hz, 1H), 1.95 (br. s, 2H), 1.30 (d, J = 6.2 Hz, 3H) ppm.
IR (film, cm⁻¹) :ν 3340, 1728, 1496, 1456, 1438, 1251, 1225, 1167, 1031, 700.

### (3) Compound (31)

A solution of Compound (30) (410 mg, 0.25 mmol) in 6M HCl aq. solution (2 ml) was stirred and heated at a gentle reflux for 30 min. After the reaction mixture was cooled to room temperature, precipitation of white solids initiated. The mixture was diluted with ethanol (1.5 ml) and ice-cooled for 1h. The precipitated white solid was filtered off to give title compound (105 mg, 84.1 %) as a white needle: mp 155-159 °C. 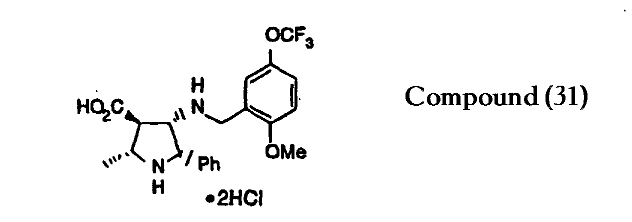 ¹H-NMR (DMSO-*d*₆) δ 11.27 (br. s, 1H), 10.04 (br. s, 1H), 7.86-7.78 (m, 2H), 7.56-7.33 (m, 5H), 7.09 (d, J = 8.8 Hz, 1H), 5.28 (br. s, 1H), 4.54 (br. s, 1H), 4.28-4.11 (m, 2H), 4.00-3.40 (m, 7H), 1.73 (d, J = 6.6 Hz, 3H) ppm.
IR (KBr, cm⁻¹) :ν 3420, 1708, 1585, 1503, 1455, 1257, 1232, 1212, 1173, 1014, 768.

### (4) Compound (32)

A solution of Compound (30) (162 mg, 0.37 mmol) in 6M HCl aq. solution (2.5 ml) was stirred and heated at a gentle reflux for 30 min. The reaction mixture was cooled to room temperature and the solvent was evaporated *in vacuo* to give crude title compound as a dark red amorphous solid. This was recrystallized from ethanol-ether to give the pure title compound (133 mg, 72.5 %) as a white solid. mp 137-140 °C. 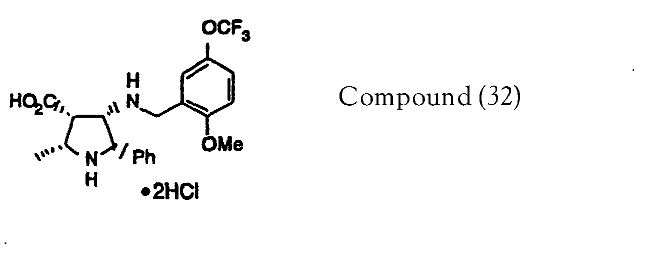 ¹H-NMR (DMSO-*d*₆) δ 10.38 (br. s, 1H), 7.66-7.57 (m, 2H), 7.55-7.44 (m, 3H), 7.42-7.29 (m, 2H), 7.10 (d, J = 8.8 Hz, 1H), 5.35 (br. s, 1H), 4.45 (br. s, 1H), 4.15-3.95 (m, 3H), 3.77 (s, 3H), 3.60-3.30 (m, 1H), 1.60 (d, J = 6.2 Hz, 3H) ppm. IR (KBr, cm⁻¹) :ν 3430, 2950, 1733, 1620, 1577, 1504, 1462, 1259, 1213, 1177, 1159, 1025, 820, 764, 700.

### Example 3

### (1)2-(4-Methyoybenzyloxy)methyl-2-propenol

A mixture of *p*-anisaldehyde (17.31g, 0.127mol), 2-methylene-1,3-propanediol (14.00g, 0.159mol), and *p*-TsOH H₂O (121mg, 0.64mmol) in dry PhH (100ml) was stirred and heated at reflux, and the water generated was azeotropically removed in a theoretical amount using a Dean-Stark apparatus. After 70ml of PhH was distilled off, the resultant red PhH solution containing 2-(4-methoxyphenyl)-5-methylene-1,3-dioxane was cooled with salt-ice, and a solution of diisobutylaluminum hydride (DIBAL-H) in PhMe (1.02M, 100ml and 1.50M, 67.0ml; total 0.203mol) was added dropwise under an atmosphere of N₂; at the point when 20ml of the PhMe solution of DIBAL-H was added, the reaction mixture changed from deep red to light yellow. After the addition was complete, the reaction mixture was allowed to stirr at room temperature overnight. The reaction mixture was ice-cooled, and MeOH (23ml) was added carefully. H₂O (12ml) was, then, added dropwise, wherein the mixture became thick and gelatinous, and finally granular. After the stirring was continued at room temperature for an hour, the mixture was filtered through a pad of Celite. The filter cake was washed with PhMe thoroughly. The combined filtrate and washings were dried (MgSO₄), and concentrated *in vacuo* at a bath temperature of 65 C to give a pale yellow oil (24.88g). This was distilled *in vacuo* to give title compound (18.52g, 70.1 %) as a colorless oil. bp 123-147 C/0.80-0.90mm; IR ₘₐₓ (film): 3420(s), 1615(s), 1515(s), 1250(s), 1070(s), 1035(s), 918(m), 820(s) cm⁻¹; ¹H-NMR δ: 7.26(d, J=8.8Hz, 2H), 6.88(d, J=8.8Hz, 2H), 5.19(br.s, 1H), 5.14(br.s, 1H), 4.45(s, 2H), 4.19(s, 2H), 4.07(s, 2H), 3.81(s, 3H), 1.98(br.s., 1H) ppm.

### (2)3-Bromo-2-[(4-methoxybenzyloxy)methyl]propene

To a stirred solution of 2-(4-methyoybenzyloxy)methyl-2-propenol (21.48g, 103.1mmol) and Ph₃P (32.45g, 123.72 mmol) in dry CH₂Cl₂ (200ml) was added *N*-bromosuccinimide (NBS, 20.19g, 113.41mmol) portionwise with cooling in a salt-ice bath. The reaction mixture was stirred under the same cooling conditions for an hour, and it became turbid with stirring. To the resultant white slurry were added Ph₃P (20.83g, 79.40mmol) and NBS (12.85g, 72.18mmol) successively, and the stirring was continued with salt-ice-cooling for additional 50 minutes, which led to the yellow reaction mixture. MeOH (3.34ml, *d*0.791, 82.5mmol) was added dropwise to destroy the exess reagents. After stirring with salt-ice-cooling for 40 minutes, the mixture was washed with sat. NaHCO₃ aq. solution (40ml x 2), and sat. NaCl aq. solution (40ml x 1). This dark green CH₂Cl₂ solution was dried (MgSO₄), and concentrated *in vacuo* to give a dark brown solid (100g). This was extracted with exhaustively with ice-chilled *n*-hexane-Et₂O (10:1). The combined extracts were filtered through a short pad of Celite, and the filtrate was, then, concentrated *in vacuo* to give title compound (20.59g, 73.5%) as a colorless oil. The solid residue was further extracted with *n*-hexane-Et₂O (4:1, 100ml x 5), and the combined extracts were filtered through a pad of Celite. The filtrate was concentrated *in vacuo* to give a solid white residue, which was extracted with ice-chilled *n*-hexane. The combined *n*-hexane extracts were filtered through a pad of Celite, and the filtrate was concentrated *in vacuo* to give an additional amount of title compound (1.84g, 6.6%) as a colorless oil; a total yield: 22.43g, 80.1%. IR ₘₐₓ (film): 1653(w), 1613(s), 1510(s), 1250(s), 1090(s), 1035(s), 912(s), 819(s) cm⁻¹; ¹H-NMR δ: 7.28(d, J=8.8Hz, 2H), 6.89(d, J=8.8Hz, 2H), 5.35(d, J=1.1Hz, 1H), 5.34(s, 1H), 4.46(s, 2H), 4.12(s, 2H), 4.04(s, 2H), 3.81(s, 3H) ppm. This was employed in the next step without further purification.

### (3)(3S*,4R*)-1-tert-Butyldimethylsilyl-3-[2-(4-methoxybenzyloxy)methyl-2-propenyl]-4-phenyl-2-azetidinone

Under an atmosphere of N₂, a 1.5M solution of LiN(Pr^{*i*})₂ THF in *c*-C₆H₆ (Aldrich; 43.9ml, 65.9mmol) was added dropwise to a stirred solution of 1-*tert*-butyldimethylsilyl-4-phenyl-2-azetidinone (13.24g, 50.64mmol) in dry THF (230ml) at an inner temperature between -78 and -73 C. After stirring at -78 C for 30 minutes, the reaction mixure became a white slurry. A solution of 3-bromo-2-[(4-methoxybenzyloxy)methyl]propene (14.69g, 54.18mmol) in dry THF (30.0ml) was added dropwise at an inner temperature between -78 and -71 C. After the addition was complete, the dropping funnel used was rinsed with dry THF (5ml x 2). The resultant homogeneous reaction mixtiure was stirreed at -78 C for an hour, and then at -10 C for 50 minutes. The reaction was quenched by adding sat. NH₄Cl aq. solution (30.0ml) below 0 C. The mixture was taken up in AcOEt (500ml). The AcOEt solution separated was washed with sat. NaCl aq. solution (100ml x 2), dried (MgSO₄), and concentrated *in vacuo* to give a heterogeneous yellow oil (26.51g). This was, then, diluted with Et₂O (250ml), washed with H₂O (x2), and sat. NaCl aq. solution (x1), dried (MgSO₄), and concentrated *in vacuo* to give a homogeneous yellow oil (24.91g). This was flash chromatographed over SiO₂ (Merck Kieselgel 60, 250g). Elution with *n*-hexane-AcOEt (40:1/30:1/20:1/10:1) gave title compound (19.73g, 86.3%) as a viscous pale yellow oil. IR ₘₐₓ (film): 1745(s), 1645(m), 1610(m), 1510(m), 990(shoulder, m), 910(m), 775(m), 745(w), 700(m), 680(m) cm⁻¹; ¹H-NMR δ: 7.40-7.25(m, 5H), 7.20(d, J=8.8Hz, 2H), 6.85(d, J=8.8Hz, 2H), 5.05(s, 1H), 4.84(s, 1H), 4.35(s, 2H), 4.20(d, J=2.6Hz, 1H), 3.86(s, 2H), 3.80(s, 3H), 3.25(ddd, J=10.5, 5.1, 2.6Hz, 1H), 2.70(dd, J=15.7, 5.1Hz, 1H), 2.48(dd, J=15.7, 10.7Hz, 1H), 0.92(s, 9H), 0.21(s, 3H), -0.21(s, 3H) ppm.

### (4)(3S*, 4R*)-1-tert-Butyldimethylsilyl-3-(2-hydroxymethyl-2-propenyl)-4-phenyl-2-azetidinone

To a stirred mixture of (3S*, 4R*)-1-*tert*-Butyldimethylsilyl-3-[2-(4-methoxybenzyloxy)methyl-2-propenyl]-4-phenyl-2-azetidinone (18.74g, 41.5mmol), pH7 phosphate buffer (15.0ml), and CH₂Cl₂ (250ml) was added 2,3-dichloro-5.6-dicyano-1,4-benzoquinone (DDQ, 15.1g, 66.4mmol) portionwise at room temperature; on the addition, the reaction mixture became dark green, and then red solids precipitated with stiirring. (In the course of addition, a slight heat evolution was observed.) The stirring was continued at room temperature for two hours, the reaction being monitored by TLC[Merck Kieselgel 60, *n*-hexane-AcOEt (3:1)]. sat. NaHCO₃ aq. solution (80.0ml) was added to quench the reaction, wherein gas evolution was observed. After Celite was added, the mixture was filterd through a pad of Celite, and the filter cake was washed with CH₂Cl₂. The combined filtrate and CH₂Cl₂ washings were washed with sat. NaHCO₃ aq. solution (x2), and dried (MgSO₄). The combined aqueous washings were filtered through a pad of Celite, and the filter cake was washed with CH₂Cl₂. From the combined filtrate and washings, the CH₂Cl₂ layer was separated, and the aqueous layer was extracted with CH₂Cl₂ (100ml x 3). The combined CH₂Cl₂ layer and washings were dried (MgSO₄). The CH₂Cl₂ solution dried were combined, treated with activated charcoal, and concentrated *in vacuo* to give an orange oil (22.1g). This was flash chromatographed over SiO₂ (Merck Kieselgel 60, 200g). Elution with *n*-hexane-AcOEt (15:1/5:1/3:1) gave title compound (11.18g, 81.2%) as a viscous pale yellow oil. IR ₘₐₓ (film): 3420(s), 1750(s), 1733(s), 1718(s), 1705(s), 1650(m), 1040(s), 900(s), 775(s) 700(s) cm⁻¹; ¹H-NMR δ: 7.43-7.25(m, 5H), 5.04(br.s, 1H), 4.82(br.s, 1H), 4.20(d, J=2.2Hz, 1H), 4.06(br.s, 2H), 3.26(ddd, J=8.2, 7.0, 2.2Hz, 1H), 2.67(dd, J=15.2, 7.0Hz, 1H), 2.54(dd, J=15.2, 8.2Hz, 1H), 2.42(br.s, 1H, O*H*), 0.92(s, 9H), 0.21(s, 3H), -0.19(s, 3H) ppm.

### (5)(3S*,4R*)-1-tert-Butyldimethylsilyl-3-(2-chloromethyl-2-propenyl)-4-phenyl-2-azetidinone

To a stirred and ice-cooled slurry of (3S*,4R*)-1-*tert*-Butyldimethylsilyl-3-(2-hydroxymethyl-2-propenyl)-4-phenyl-2-azetidinone (13.6g, 41.0mmol), 2,6-lutidine (15.3ml, *d*0.920, 123mmol), and LiCl (anhydros, 4.37g, 103mmol) in DMF (103ml) was added MeSO₂Cl (MsCl, 7.93ml, *d*1.480, 103mmol) dropwise under an atmosphere of N₂. After the reaction mixture was stirred with ice-cooling for 5 hours and 50 minutes, H₂O (40ml) was added. The stirring was continued at room temperature for additional 20 minutes to destroy the excess MsCl. The resultant dark red mixture was poured into H₂O (70ml). The mixture was extracted with Et₂O (70ml x 5). The combined Et₂O extracts were washed with H₂O (x3), sat. NaHCO₃ aq. solution (x1), and sat. NaCl aq. solution (x1), dried (MgSO₄), treated with activated charcoal, and concentrated *in vacuo* to give practically pure title compound (15.36g, quantitative) as a pale yellow oil. IR ₘₐₓ (film): 1735(v.s), 1650(w), 1175(s), 825(s), 750(s), 700(s) cm⁻¹; ¹H-NMR δ: 7.43-7.25(m, 5H), 5.14(br.s, 1H), 4.87(br.s, 1H), 4.22(d, J=2.6Hz, 1H), 3.99(s, 2H), 3.25(ddd, J=10.1, 5.1, 2.6Hz, 1H), 2.78(dd, J=16.1, 5.1Hz, 1H), 2.60(dd, J=16.1, 10.1Hz, 1H), 0.92(s, 9H), 0.22(s, 3H), -0.20(s, 3H) ppm. This was employed im the next step without further purification.

### (6)(2R*,3S*)-3-Methoxycarbonyl-5-methlene-2-phenylpiperidine

A homogeneous mixture of (3S*,4R*)-1-*tert*-butyldimethylsilyl-3-(2-chloromethyl-2-propenyl)-4-phenyl-2-azetidinone (12.98g, 33.8mmol) and Hydrogen Chloride, Methanol Reagent 10 (Tokyo Kasei, 150ml) was stirred and heated at reflux for an hour. The mixture was concentrated *in vacuo*. The syrupy dark orange residue was ice-cooled, and then basified with sat. Na₂CO₃ aq. (ca. 50ml) to pH 9-10. The mixture was extracted with AcOEt (50ml x 5). The combined AcOEt extracts were dried (Na₂SO₄), and concentrated *in vacuo* to give a yellow semisolid (9.74g). To this were added NaI (7.60g, 50.7mmol), NaHCO₃ (8.52g, 101 mmol), and DMF (70ml), and the resultant mixture was stirred and heated at a bath temperature of 160 C for 15 minutes. After the mixture was allowed to cool to room temperature, sat. Na₂CO₃ aq. solution (70ml) was added, and the insoluble inorganic materials were filtered off by the aid of Celite. The filter cake was washed with AcOEt-PhMe (2:1, x3). From the combined filtrate and washings was separated the AcOEt-PhMe layer, and the aqueous layer was extracted with AcOEt-PhMe (2:1, 30ml x 5). The combined AcOEt-PhMe layer and extracts were washed with H₂O (40ml x 3), and sat. NaCl aq. solution (40ml x 1), dried (Na₂SO₄), and concentrated *in vacuo* at a bath temperature below 70 C to give practically pure title compound (7.66g, overall 98.0% from (3S*,4R*)-1-*tert*-butyldimethylsilyl-3-(2-chloromethyl-2-propenyl)-4-phenyl-2-azetidinone) as a red orange oil. IR ₘₐₓ (film): 3320(w), 1728(s), 1650(w), 1165(s), 900(m), 738(m), 698(s) cm⁻¹; ¹H-NMR δ: 7.41-7.14(m, 5H), 4.88(br.s, 1H), 4.75(br.s, 1H), 4.09(d, J=2.9Hz, 1H), 3.68(br.d, J=14.0Hz, 1H), 3.45(s, 3H), 3.43(br.d, J=14.0Hz, 1H), 3.23-3.14(m, 1H), 2.83-2.62(m, 1H), 1.78(br.s, 1H, N*H*) ppm. This was employed in the next step without further purification.

### (7)(2R*,3S*)-3-Methoxycarbonyl-5-methlene-2-phenylpiperidine

To a stirred mixture of crude (2R*, 3S*)-3-methoxycarbonyl-5-methlene-2-phenylpiperidine (8.58g, 37.1mmol), NaHCO₃ (6.23g, 74.2mmol), and H₂O (75.0ml) was added a solution of di-*tert*-butyl dicarbonate [(Boc)₂O, 9.72g, 44.5mmol) in THF (5.0ml); on the addition, CO₂ gas evolved violently. The resultant pale yellow mixture was stirred at room temperature for 3 hours, and then extracted with AcOEt (70ml x 3). The combined AcOEt extracts were washed with sat. NaCl aq. solution (x1), dried (MgSO₄), and concentrated *in vacuo* to give an orange oil (14.35g). This was flash chromatographed over SiO₂ (Merck Kieselgel 60, 150g). Elution with *n*-hexane-AcOEt (50:1/20:1) gave title compound (11.91g, 96.9%) as a relatively unstable, almost colorless oil. IR ₘₐₓ (film): 1735(s), 1697(s), 1685(s), 1650(shoulder, m), 1170(s), 895(s), 765(m), 700(s) cm⁻¹; ¹H-NMR δ: 7.36-7.17(m, 5H), 4.95(br.s, 1H), 4.89(br.s, 1H), 4.40-4.20(m,1H), 3.80-3.35(m, 2H), 3.63(s, 3H), 3.14-3.03(m, 1H), 2.84-2.57(m, 2H), 1.42(br.s, 9H) ppm.

### (8)A mixture of (2R*,3S*,5S*)- and (2R*,3S*,5R*)-1-tert-Butoxycarbonyl-5-hydroxymethyl-3-methoxycarbonyl-2-phenylpiperidine

To a stirred solution of (2R*,3S*)-3-methoxycarbonyl-5-methylene-2-phenylpiperidine (9.90g, 29.9mmol) and (Ph₃P)₃RhCl (1.38g, 1.50mmol) in dry THF (150ml) was added a 1.0M solution of catecholborane in THF (Aldrich, 89.7ml, 89.7mmol) dropwise at room temperature under an atmosphere of N₂. The resultant dark red reaction mixture was allowed to stirr at room temperature for 2.5 hours. The mixture was ice-cooled, and then MeOH (150ml) was added carefully. 5% NaHCO₃ aq. solution (60.0ml, 35.7mmol) and 30% H₂O₂ aq. solution (99.0ml, 874mmol) were added successively, and the black mixture was stirred with ice-cooling for 1.5 hours. This was concentrated *in vacuo* at a bath temperature between 40 and 50 C. The aqueous residue was extracted with AcOEt (70ml x 5). The combined AcOEt extracts were washed with ice-chilled 1.0M NaOH aq. solution (x4), and sat. NaCl aq. solution (x1), dried (MgSO₄), treated with activated charcoal, and concentrated *in vacuo* to give a dark red syrup (11.42g). This was flash chromatographed over SiO₂ (Merck Kieselgel 60, 120g). Elution with *n*-hexane-AcOEt (10:1/5:1/3:1) gave a mixture of title compounds (9.17g, 87.8%) as a viscous pale orange oil. IR ₘₐₓ (film): 3450(s), 1730(s), 1680(br., s), 1290(br., s), 1150(br., s), 1040(s), 765(w), 738(m), 700(s) cm⁻¹; ¹H-NMR δ: in part, 3.61(s, 3H, CO₂C*H*₃), 1.53, 1.50, and 1.48(each s, total 9H, NCO₂C₄*H*₉t) ppm.

### (9) A mixture of (2R*, 3S*, 5S*)- and (2R*, 3S*, 5R*)-5-Hydroxymethyl-3-methoxycarbonyl-2-phenylpiperidine

A mixture of (2R*,3S*,5S*)- and (2R*,3S*,5R*)-1-*tert*-Butoxycarbonyl-5-hydroxymethyl-3-methoxycarbonyl-2-phenylpiperidine (19.31g, 55.26mmol) was dissolved in MeOH (50ml), and Hydrogen Chloride, Methanol Reagent 10 (Tokyo Kasei, 50ml) was added at room temperature. After the resultant solution was stirred and heated at a gentle reflux for 1.5 hours, an additional amount of Hydrogen Chloride, Methanol Reagent 10 (Tokyo Kasei, 100ml) was added. The stirring was continued with heating at reflux for an hour. The bright yellow reaction mixture was, then, concentrated *in vacuo*. The residue was ice-cooled, and basified with sat. Na₂CO₃ aq. solution to pH9-10. The inorganic precipitates were filtered off by the aid of Celite, and the filter cake was washed with CH₂Cl₂. From the combined filtrate and washings was separated the CH₂Cl₂ layer, and the aqueos layer was extracted with CH₂Cl₂. The combined CH₂Cl₂ layer and extracts were dried (Na₂SO₄), and concentrated *in vacuo* to give a mixture of title compounds (12.89g, 93.5%) as a dark red oil. IR ₘₐₓ (film): 3320(s), 1720(s), 1605(w), 1497(m), 1195(s), 1170(s), 740(m), 700(s) cm⁻¹; ¹H-NMR δ: 7.40-7.16(m, 5H), 4.40(d, J=5.1H, 0.33H), 3.90(d, J=3.7Hz, 0.67H), 3.60(d, J=6.6Hz, 0.66H), 3.52-3.40(m, 0.67H), 3.49(d, J=4.8Hz, 1.34H), 3.48(s, 1H), 3.41(s, 2H), 3.10-2.99(m, 1H), 2.93(dd, J=12.6, 4.0Hz, 0.33H), 2.72(dd, J=12.6, 7.7Hz, 0.33H), 2.53(dd, J=12.8, 11.4Hz, 0.67H), 2.25-1.93(m, 4H), 1.93-1.76(m, 0.33H), 1.65(ddd, J=14.3, 14.3, 5.4Hz, 0.67H) ppm. This was employed in the next step without further purification.

### (10) (2R*,3S*,5S*)- and (2R*,3S*,5R*)-5-(tert-Butyldimethylsilyloxy)-methyl-3-methoxycarbonyl-2-phenylpiperidine

A mixture of (2R*,3S*,5S*)- and (2R*,3S*,5R*)-5-hydroxymethyl-3-methoxycarbonyl-2-phenylpiperidine (12.89 g, 51.70 mmol) was dissolved in DMF (30 ml), and imidazole (8.80 g, 129.3 mmol) and TBDMS-Cl (9.35 g, 62.0 mmol) were added portionwise successively at room temperature; on the addition of TBDMS-Cl, threre took place heat evolution. After stirring at room temperature for 3 hours, the dark yellow reaction mixture was diluted with AcOEt-PhMe (2:1, 300 ml), washed with H₂O (30 ml x3), and sat. NaCl aq. solution (x1), dried (Na₂SO₄), and concentrated *in vacuo* to give an orange oil (18.36 g). This was flash chromatographed over SiO₂ (Merck Kieselgel 60, 320 g). Elution with CH₂Cl₂-MeOH (600:1/500:1/400:1/300:1/200: 1)gave (2R*,3S*,5S*)-title compound (11.46g, 61.0%) as a pale yellow oil. IR ₘₐₓ (film): 3320(w), 1725(s), 1605(w), 1495(w), 1250(s), 1165(s), 1105(s), 838(s), 775(m), 698(m) cm⁻¹; ¹H-NMR δ: 7.35-7.17(m, 5H), 3.88(d, J=3.3Hz, 1H), 3.53-3.34(m, 2H), 3.45(dd, J=11.5, 5.5Hz, 1H), 3.42(s, 3H), 3.13-3.01(m, 1H), 2.54(dd, J=13.2, 11.5Hz, 1H), 2.18-2.00(m, 2H), 1.80-1.60(m, 2H), 0.90(s, 9H), 0.05(s, 6H) ppm. Further elution with CH₂Cl₂-MeOH (200:1/100:1/80:1/40:1) gave (2R*,3S*,5R*)-title compound (4.86 g, 25.9 %) as a pale yellow oil. IR ₘₐₓ (film): 3340(w), 1730(s), 1602(w), 1495(m), 1250(s), 1195(s), 1110(s), 835(s), 777(s), 700(s) cm⁻¹; ¹H-NMR δ: 7.38-7.17(m, 5H), 4.46(d, J=5.1Hz, 1H), 3.60-3.40(m, 2H), 3.50(s, 3H), 3.06(ddd, J=10.3, 5.1, 5.1Hz, 1H), 2.87(dd, J=12.6, 4.0Hz, 1H), 2.65(dd, J=12.6, 8.8Hz, 1H), 2.13-1.95(m, 1H), 1.95-1.73(m, 1H), 1.65(br.s, 2H), 0.87(s, 9H), 0.031(s, 3H), 0.027(s, 3H) ppm.

### (11) (2R*,3S*,5S*)-1-Benzyloxycarbonyl-5-(tert- butyldimethylsilyloxy)methyl-3-methoxycarbonyl-2-phenylpiperidine

To a stirred and ice-cooled mixture of (2R*,3S*,5S*)-5-(*tert*-butyldimethylsilyloxy)methyl-3-methoxycarbonyl-2-phenylpiperidine (11.46 g, 31.52 mmol), NaHCO₃ (3.97g, 47.3mmol), H₂O (80.0 ml), and THF (10.0 ml) was added a solution of benzyl chloroformate (Cbz-Cl, 5.92 g, 34.67 mmol) in THF (15.0ml). A precipitated gummy material was dispersed by adding AcOEt (40.0ml). After the resultant milky suspension was stirred with ice-cooling for 2.5 hours, the organic layer was separated from the mixture, and the aqueous layer was extracted with AcOEt (40 ml x 4). The combined organic layer and AcOEt extracts were washed with sat. NaCl aq. solution (x1), dried (MgSO₄), and concentrated *in vacuo* to give a yellow oil (16.56 g). This was flash chromatographed over SiO₂ (Merck Kieselgel 60, 200 g). Elution with *n*-hexane-AcOEt (60:1/30:1/20:1) gave title compound (13.88 g, 88.5 %) as a pale yellow oil. IR ₘₐₓ (film): 1735(s), 1700(s), 1498(m), 1290(s), 1253(s), 1100(s), 775(s), 740(m), 700(s) cm⁻¹; ¹H-NMR δ: 7.42-7.28(m, 6H), 7.28-7.16(m, 4H), 5.98-5.87(m, 1H), 5.31-5.10(m, 2H), 3.92(dm, J=13.6Hz, 1H), 3.63(dd, J=10.1, 9.7Hz, 1H), 3.60(s, 3H), 3.53(dd, J=10.1, 7.9Hz, 1H), 3.09(ddd, J=11.2, 5.6, 5.6Hz, 1H), 3.12-2.95(m, 1H), 2.24-2.06(m, 1H), 2.06-1.93(m, 1H), 0.87(s, 9H), 0.02(s, 6H) ppm.

### (12)(2R*,3S*,5S*)-1-Benzyloxycarbonyl-5-(tert- butyldimethylsilyloxy)methyl-3-carbamoyl-2-phenylpiperidine

Under an atmosphere of N₂, a 2.0M solution of Me₃Al in PhMe (Aldrich, 140ml, 280mmol) was added dropwise to a stirred and ice-cooled suspension of NH₄Cl (14.92g, 279mmol) in dry PhMe (280ml). After the addition was complete, the reaction mixture was allowed to stirr at room temperature for 30 minutes; at this point, solid NH₄Cl disappeared completely, and the clear, homogeneous mixture resulted. To this was added a solution of (2R*,3S*,5S*)-1-benzyloxycarbonyl-5-(*tert*-butyldimethylsilyloxy) methyl-3-methoxycarbonyl-2-phenylpiperidine (13.88g, 27.89mmol) in dry PhMe (30.0ml) dropwise at room temperature: on the addition, the reaction mixture became turbid. This was stirred and heated at a bath temperature of 50 C overnight (ca. 20 hours). The turbid white reaction mixture was ice-cooled, and H₂O (80.0ml, 4.40mol) was added carefully, wherein H₂ gas evolved violently. The resultant white precipitates were filtered off by the aid of Celite, and washed with AcOEt (80ml x 5). The combined filtrate and AcOEt washings were washed with sat. NaCl aq. solution (x2), dried (MgSO₄), and concentrated *in vacuo* to give a viscous colorless oil (13.76g). This was flash chromatographed over SiO₂ (Merck Kieselgel 60, 100g). Elution with *n*-hexane-AcOEt (5:1/2:1/1:1) gave title compound (11.10g, 82.4%) as a colorless syrup. IR ₘₐₓ (film): 3405(s), 3340(s), 3200(s), 3100(w), 3070(m), 3045(m), 1735(m), 1675(br., v.s), 1610(s), 1498(s), 1160(s), 1100(s), 836(s), 775(s), 737(s) cm⁻¹; ¹H-NMR δ: 7.42-7.18(m, 10H), 5.83(d, J=5.1Hz, 1H), 5.67(br.s, 1H), 5.47(br.s, 1H), 5.20(d, J=12.5Hz, 1H), 5.13(d, J=12.5Hz, 1H), 3.91(br.d, J=13.9Hz, 1H), 3.63(dd, J=10.0, 6.8Hz, 1H), 3.53(dd, J=10.0, 7.7Hz, 1H), 3.08(dd, J=13.9, 7.7Hz, 1H), 2.98(ddd, J=17.6, 5.1, 4.8Hz, 1H), 2.25-1.91(m, 3H), 0.87(s, 9H), 0.02(s, 6H) ppm.

### (13)(2S*,3S*,5S*)-1-Benzyloxycarbonyl-3-(N-tert- butoxycarbonyl)amino-5-(tert-butyldimethylsilyloxy)methyl-2-phenylpiperidine

A mixture of (2R*,3S*,5S*)-1-benzyloxycarbonyl-5-(*tert*-butyldimethylsilyloxy)methyl-3-carbamoyl-2-phenylpiperidine (11.1g, 23.0mmol) and *tert*-BuOH (200ml) was stirred and heated to a bath temperature of 80 C. To the resultant homogeneous mixture was added Pb(OAc)₄ (17.3g, 39.1mmol) in one portion at that temperature. On the addition, the reaction mixture became yellow brown, and this was stirred and heated at reflux for 45 minutes. After cooling to room temperature, the mixture was neutralized with sat. NaHCO₃ aq. solution (ca. 200ml) with occational ice-cooling. The dark brown precipitates were filtered off by the aid of Celite, and washed with AcOEt. The combined filtrate and AcOEt washings were concentrated *in vacuo* to give an aqueous residue, from which white solids precipitated in the course of the concentration. This residue was extracted with AcOEt (60ml x 4). The combined AcOEt extracts were washed with sat. NaCl aq. solution (x1), dried (MgSO₄), and concentrated *in vacuo* to give a viscous colorless oil (14.18g). This was crystallized from *n*-hexane (ca. 30ml)-AcOEt (ca. 2ml) to give title compound (11.42g, 89.5%) as white powders. The mother liquor concentrated *in vacuo* (1.02g) was purified by SiO₂ flash chromatography [Merck Kieselgel 60, 15.0g; *n*-hexane-AcOEt (30:1/20:1)] to give an additional amount of title compopund (0.68g, 5.3%); a total yield: 12.10g (94.8%). mp 119.0-119.5 C (white powders); IR ₘₐₓ (nujol): 3330(s), 1700(s), 1685(s), 1520(s), 1248(s), 1160(s), 1118(s), 858(s), 840(s), 780(s), 770(s), 763(s), 738(m), 710(s), 698(m) cm⁻¹; ¹H-NMR δ: 7.41-7.20(m, 10H), 7.10(br.s, 1H), 5.35(br.d, J=5.9Hz, 1H), 5.13(br.s, 0.6H), 5.08(br.s, 0.4H), 5.01(br.s, 0.7H), 4.96(br.s, 0.3H), 4.28-4.05(m, 2H), 3.93-3.80(m, 1H), 3.73-3.55(m, 3H), 2.30-2.16(m, 1H), 1.77-1.60(m, 1H), 1.40(br.s, 7.5H), 0.88(br.s, 10.5H), 0.05(s, 3H), 0.04(s, 3H) ppm.

### (14) (2S*,3S*,5S*)-1-Benzyloxycarbonyl-3-(N-tert- butoxycarbonyl)amino-5-hydroxymethyl-2-phenylpiperidine

Toa stirred and ice-cooled suspension of (2S*,3S*,5S*)-1-benzyloxycarbonyl-3-(*N*-*tert*-butoxycarbonyl)amino-5-(*tert*-butyldimethylsilyloxy)methyl-2-phenylpiperidine (12.10g, 21.81mmol) and molecular sieves 4A (MS 4A, activated powder, Aldrich; 15.0g) in dry THF (100ml) was added a 1.0M solution of (*n*-Bu)₄NF in THF (Aldrich, 26.2ml, 26.2mmol) dropwise. After the addition was complete, the reaction mixture was allowed to stirr at room temperature for 1.5 hours. The zeolite was filtered off by the aid of Celite, and washed with THF. To the combined filtrate and washings was added H₂O (50.0ml), and the mixture was neutralized with 1.0M AcOH aq.solution (1.0ml). The solvent THF was evaporated *in vacuo*, and the aqueous residue was extracted with CH₂Cl₂ (40ml x 4). The combined CH₂Cl₂ extracts were washed with sat. NaHCO₃ aq. solution (x1), H₂O (x2), and sat. NaCl aq. solution (x1), dried (MgSO₄), and concentrated *in vacuo* to give a pale orange solid (12.82g). This was flash chromatographed over SiO₂ (Merck Kieselgel 60, 120g). Elution with CH₂Cl₂-MeOH (300:1/150:1) gave a white solid (7.81g), which was recrystallized from AcOEt-*n*-hexane to give title compound (7.05g, 73.4%) as a white powder. From the pale yellow mother liquor (0.16g) was recovered an additional amount of title compound (0.08g, 0.83%); a total yield: 7.13g (74.3%). mp 121.0-124.0 C (white powders); IR ₘₐₓ (nujol): 3300(s), 1712(s), 1699(s), 1671(s), 1538(s), 1498(w), 1263(s), 1170(s), 1050(s), 738(m), 699(s) cm⁻¹; ¹H-NMR δ: 7.43-7.24(m, 10H), 7.28-7.13(m, 1H), 5.48(br. d, J=5.5Hz, 1H), 5.20(d, J=12.5Hz, 1H), 5.03(br.d, J=12.5Hz, 1H), 4.31-4.13(m, 1H), 3.99(br.dd, J=14.5, 4.1Hz, 1H), 3.75-3.58(m, 2H), 3.36(br.dd, J=14.5, 4.6Hz, 1H), 2.88-2.11(m, 2H), 1.97-1.75(m, 2H), 1.39(s, 9H) ppm.

### (15)(2S*,3S*,5S*)-1-Benzyloxycarbonyl-3-(N-tert-butoxycarbonyl)amino-5-formyl-2-phenylpiperidine

To a stirred and ice-cooled solution of (2S*,3S*,5S*)-1-benzyloxycarbonyl-3-(*N*-*tert*-butoxycarbonyl)amino-5-hydroxymethyl-2-phenylpiperidine(2.50g, 5.68mmol) and Et₃N (3.55ml, *d*0.726, 25.5 mmol) in DMSO (25.0 ml) was added SO₃ pyridine (4.06g, 25.5 mmol) portionwise. The resultant orange mixture was stirred at room temperature for 45 minutes, and then pouered into ice-water (50ml). The mixture was extracted with CH₂Cl₂ (30ml x 4). The combined CH₂Cl₂ extracts were washed with 10% citric acid aq. solution (x2), H₂O (x1), sat. NaHCO₃ aq. solution (x1), and sat. NaCl aq. solution (x1), dried (MgSO₄), and concentrated *in vacuo* to give crude title compound (2.80g, quantitative) as a pale yellow solid. This was employed in the next step without further purification. A portion of it (0.112g) was recrystallied from AcOEt-Et₂O to give an analytical sample of title compound (0.051g). mp 125.0-127.0 C (white powders); IR ₘₐₓ (nujol): 3300(s), 1720(shoulder, s), 1710(v.s), 1698(shoulder, s), 1538(s), 1500(m), 1170(s), 773(m), 738(s), 700(s) cm⁻¹; ¹H-NMR δ: 9.79(br.s, 0.6H), 9.73(br.s, 0.4H), 7.50-7.20(m, 10H), 7.22(br.s, 1H), 5.04-4.92(m, 1H), 5.17(br.d, J=12.1Hz, 1H), 5.11-4.98(m, 1H), 4.68-4.54(m, 0.6H), 4.54-4.39(m, 0.4H), 4.30-3.95(m, 2H), 3.60-3.45(m, 0.6H), 3.38-3.15(m, 0.4H), 2.90-2.67(m, 1H), 2.52-2.39(m, 0.6H), 2.34-2.21(m, 0.4H), 1.41(br.s, 9H) ppm.

### (16)(2S*,3S*,5S*)-1-Benzyloxycarbonyl-3-(N-tert- butoxycarbonyl)amino-5-methoxycarbonyl-2-phenylpiperidine

To a stirred and ice-cooled suspension of crude (2S*,3S*,5S*)-1-benzyloxycarbonyl-3-(*N-tert*-butoxycarbonyl)amino-5-formyl-2-phenylpiperidine (2.80g, 5.86mmol) in THF (3.0ml) and MeOH-H₂O (9:1, 10.0ml) was added solid NaHCO₃ (17.2g, 205.1mmol). To this suspension was added a 2.0M stock solution of Br₂ in MeOH-H₂O (9:1, 20.5ml, 41.0mmol) dropwise with ice-cooling. After the addition was complete, the deep red reaction mixture was allowed to stirr at room temperature for 5 hours. The orange red mixture was ice-cooled, and solid Na₂S₂O₃ was added portionwise with stirring until the red color of the exess Br₂ was discharged. The inorganic precipitates were filtered off by the aid of Celite, and washed with CH₂Cl₂. The combined filtrate and CH₂Cl₂ washings were concentrated *in vacuo*. To the residue was added H₂O, and the mixture was extracted with AcOEt-CH₂Cl₂ (4: 1). The combined AcOEt-CH₂Cl₂ (4:1) extracts were washed with sat. NaHCO₃ aq. solution (x1), and sat. NaCl aq. (x1), dried (MgSO₄), and concentrated *in vacuo* to give a yellow solid (2.03g). This was flash chromatographed over SiO₂ (Merck Kieselgel 60, 35g). Elution with CH₂Cl₂-MeOH (30:1) gave a white solid (1.19g), which was recrystallized from AcOEt-*n*-hexane to afford title compound (1.01g, overall 36.7% from (2S*,3S*,5S*)-1-benzyloxycarbonyl-3-(*N*-*tert*-butoxycarbonyl)amino-5-hydroxymethyl-2-phenylpiperidine in two steps). mp 128.0-129.0 C (white fine needles); IR ₘₐₓ (nujol): 3320(s), 1735(s), 1710(v.s), 1700(v.s), 1673(v.s), 1583(s), 1498(m), 1250(s), 1175(s), 1110(s), 770(m), 738(s), 700(s) cm⁻¹; ¹H-NMR δ: 7.50-7.10(m, 10H), 7.18(br.s, 1H), 5.47(br.d, J=5.9Hz, 1H), 5.18(d, J=12.5Hz, 1H), 4.99(d, J=12.5Hz, 1H), 4.56-4.33(m, 1H), 4.36-4.05(m, 1H), 3.69(s, 3H), 3.65-3.40(m, 1H), 2.99-2.86(m, 1H), 2.42-2.25(m, 1H), 1.96-1.80(m, 1H), 1.41(br.s, 9H) ppm.

### (17) (2S*,3S*,5S*)-3-Amino-1-benzyloxycarbonyl-5-methoxycarbonyl-2-phenylpiperidine

A suspension of (2S*,3S*,5S*)-1-benzyloxycarbonyl-3-(*N*-*tert*-butoxycarbonyl)amino-5-methoxycarbonyl-2-phenylpiperidine (1.01g, 2.16mmol) in Hydrogen Chloride, Methanol Reagent 10 (20.0ml) was stirred and heated at reflux for 10 minutes; the reaction mixture became homogeneous with evoution of CO₂ gas. This mixture was concentrated *in vacuo*, and the syrupy residue was basified with sat. Na₂CO₃ aq. solution. The mixture was extracted with CH₂Cl₂ (x4). The combined CH₂Cl₂ extracts were dried (Na₂SO₄), and concentrated *in vacuo* to give crude title compound (0.789g, quantitative) as a pale yellow syrup. IR ₘₐₓ (film): 3375(m), 3310(w), 1730(br., s), 1700(br., s), 1603(m), 1585(m), 1497(s), 1122(s), 740(s), 700(s) cm⁻¹; ¹H-NMR δ: 7.45-7.15(m, 10H), 5.26(d, J=5.9Hz, 1H), 5.15(d, J=12.5Hz, 1H), 5.01(d, J=12.5Hz, 1H), 4.51(dd, J=13.9, 2.1Hz, 1H), 3.65(s, 3H), 3.56-3.43(m, 1H), 3.54(dd, J=13.9, 4.2Hz, 1H), 2.95-2.85(m, 1H), 2.22(ddd, J=14.9, 4.0, 4.0Hz, 1H), 1.95(ddd, J=14.9, 10.9, 6.6Hz, 1H) ppm.

### (18) (2S*,3S*,5S*)-1-Benzyloxycarbonyl-5-methoxycarbonyl-3-[N-(2-methoxy-5-trifluoromethoxybenzyl)amino]-2-phenylpiperidine

To a stirred solution of (2S*,3S*,5S*)-3-amino-1-benzyloxycarbonyl-5-methoxycarbonyl-2-phenylpiperidine (0.79g, 2.16mmol) and 2-methoxy-5-trifluoromethoxybenzaldehyde (0.59g, 2.70mmol) in CH₂Cl₂ (25.0ml) was added NaB(OAc)₃H (0.69g, 3.24mmol) potionwise at room temperature. The turbid reaction mixture was stirred at room temperature for 3 hours; the NaB(OAc)₃H added dissolved gradually with stirring, and the turbidity of the reaction mixture decreased. sat. Na₂CO₃ aq. solution was added, and from the mixture was swparated the CH₂Cl₂ layer. The aqueous layer was extracted with CH₂Cl₂. The combined CH₂Cl₂ layer and extracts were dried (Na₂SO₄), and concentrated *in vacuo* to give a pale yellow oil (1.55g). This was flash chromatographed over SiO₂ (Merck Kieselgel 60, 32.0g). Elution with CH₂Cl₂-MeOH (300:1/200:1) gave title compound (1.15g, 93.1%) as a colorless syrup, which crystallized spontaneously on standing in a refrigirator. This was recrystallized from (*i*-Pr)₂O to give analytically pure title compound (1.058g, 85.3%). mp 93.5-95.0 C (fine colorless needles); IR ₘₐₓ (nujol): 1730(s), 1700(s), 1610(w), 1498(s), 1220(s), 1147(s), 1110(s), 1080(s), 1055(s), 1028(s), 742(s), 730(s), 703(s), 695(s) cm⁻¹; ¹H-NMR δ: 7.43-7.23(m, 9H), 7.21(br.s, 1H), 7.09-7.00(m, 2H), 6.73(d, J=9.2Hz, 1H), 5.39(br.d, J=5.3Hz, 1H), 5.16(d, J=12.5Hz, 1H), 5.02(d, J=12.5Hz, 1H), 4.50(dd, J=13.7, 1.8Hz, 1H), 3.79(d, J=13.9Hz, 1H), 3.68(d, J=13.9Hz, 1H), 3.63(s, 3H), 3.54(dd, J=13.7, 5.5Hz, 1H), 3.28(ddd, J=10.2, 5.5, 4.8Hz, 1H), 2.89-2.28(m, 1H), 2.24-2.01(m, 1H), 1.54(br.s, 2H) ppm.

### (19) (2S*,3S*,5S*)-5-Methoxycarbonyl-3-[N-(2-methoxy-5-trifluoromethoxybenzyl)amino]-2-phenylpiperidine

A mixture of (2S*,3S*,5S*)-1-benzyloxycarbonyl-5-methoxycarbonyl-3-[*N*-(2-methoxy-5-trifluoromethoxybenzyl) amino]-2-phenylpiperidine (1.007g, 1.758mmol) and 20% Pd(OH)₂/C (0.258g), THF (5.0ml), and MeOH (30.0ml) was stirred under an atmosphere of H₂ (baloon) at room temperature for an hour. The catalyst was filtered off by the aid of Celite, and washed with THF. The combined filtrate and THF washings were concentrated i*in vacuo* to give title compound (0.789g, quantitatively) as a pale yellow oil. IR ₘₐₓ (film): 3340(s), 1735(s), 1718(s), 1608(m), 1250(br., s), 1150(br., s), 1033(s), 810(m), 750(m), 700(m) cm⁻¹; ¹H-NMR δ: 7.36-7.18(m, 5H), 7.00(dd, J=9.2, 2.2Hz, 1H), 6.84(d, J=2.2Hz, 1H), 6.62(d, J=9.2Hz, 1H), 3.89(d, J=2.2Hz, 1H), 3.71(s, 3H), 3.68(d, J=14.3Hz, 1H), 3.51(ddd, J=11.9, 3.7, 1.8Hz, 1H), 3.47(s, 3H), 3.40(d, J=14.3Hz, 1H), 3.05(dddd, J=12.3, 11.9, 3.7, 3.7Hz, 1H), 2.92-2.82(m, 1H), 2.88(dd, J=11.9, 11.9Hz, 1H), 2.37(dm, J=13.6Hz, 1H), 1.78(ddd, J= 13.6, 12.3, 2.9Hz, 1H), 1.68(br.s, 2H, NHx2) ppm.

### (20) (2S*,3S*,5S*)-5-Methoxycarbonyl-3-[N-(2-methoxy-5-trifluoromethoxybenzyl)amino]-2-phenylpiperidine dihydrochloride

To a solution of (2S*,3S*,5S*)-5-methoxycarbonyl-3-[*N*-(2-methoxy-5-trifluorometh-oxybenzyl)amino]-2-phenylpiperidine (0.856g, 1.952mmol) in MeOH (2.0ml) was added an exess amount of Hydrogen Chloride, Methanol Reagent 10 (Tokyo Kasei, > 1.60ml) until the mixture became sufficientky acidic (pH < 2); in the course of the addition, precipitation of a white solid tok place. After Et₂O (1.5ml) was added, the heterogeneous yellow mixture was left to stand in a refrigirator overnight. The white solids precipitated were collected by filtration, washed with *i*-PrOH-(*i*-Pr)₂O (2:1), and dried *in vacuo* with heating at 50 C to give title dihydrochloride (0.933g, 93.6%) as white powders. mp 217.8-218.3 C; IR ₘₐₓ (nujol): 3230-2150(br., s), 1748(s), 1620(w), 1580(m), 1557(m), 1502(s), 1272(s), 1238(s), 1210(s), 1173(s), 1152(s), 1130(s), 1030(s), 858(m), 810(m), 770(m), 752(m), 700(s), 687(m) cm⁻¹; ¹H-NMR δ (DMSO-d₆): 7.69-7.58(m, 2H), 7.57-7.42(m, 3H), 7.38-7.26(m, 2H), 7.02(br.d, J=8.8Hz, 1H), 4.93(br.s, 1H), 4.10-3.80(m, 1H), 3.87(br.d, J=12.84Hz, 1H), 3.71(s, 3H), 3.70(br.s, 2H), 3.68-3.60(m, 1H), 3.60-3.05(m, 8H), 2.63(br.d, J=12.8Hz, 1H), 2.19(br.dd, J = 12.8, 12.8Hz, 1H) ppm.

### (21) (2S*,3S*,5S*)-5-Carboxy-3-[N-(2-methoxy-5-trifluoromethoxybenzyl)amino]-2-phenylpiperidine dihydrochloride

To a suspension of (2S*, 3S*, 5S*)-5-methoxycarbonyl-3-[*N*-(2-methoxy-5-trifluoromethoxybenzyl)amino]-2-phenylpiperidine dihydrochloride(0.197g, 0.385mmol) in MeOH (6.0ml) was added 6M HCl aq. solution (8.0ml), and the mixture was stirred and heated at a gentle reflux for an hour; the mixture became homogeneous with heating. After conc. HCl (7.0ml) was added, the stirring was continued with heating at reflux for additional two hours. The mixture was cooled to room temperature, and acetone was added. After the mixture was ice-cooled, the white solids precipitated were collected by filtration, washed with acetone, dried *in vacuo* with heating at 60 C overnight to give title hydrochloride (0.138g, 71.8%) as white plates. 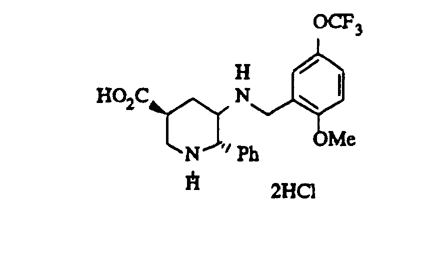 mp 207.8-211.8 C; IR ₘₐₓ (nujol): 3270-2200(br., s), 1760(s), 1745(s), 1580(w), 1550(m), 1500(s), 1253(s), 1238(s), 1210(s), 1180(s), 1157(s), 1125(m), 1026(m), 810(m), 759(m), 723(m), 700(s), 680(w) cm⁻¹;
¹H-NMR δ (DMSO-d₆): 7.69-7.55(m, 2H), 7.56-7.40(m, 3H), 7.37-7.24(m, 2H), 7.00(br.d, J=8.4Hz, 1H), 4.88(br.s, 1H), 4.00-3.75(m, 1H), 3.85(br.d, J=13.9Hz, 1H), 3.71(s, 3H), 3.69-3.55(m, 1H), 3.67(br.s, 2H), 3.57-3.10(m, 6H), 2.69-2.50(m, 1H), 2.26-2.04(m,1H) ppm.

### (22) (2S*,3S*,5S*)-3-Amino-1-benzyloxycarbonyl-5-hydroxymethyl-2-phenylpiperidine

A suspension of (2S*,3S*,5S*)-1-benzyloxycarbonyl-3-(*N*-*tert*-butoxycarbonyl)-amino-5-hydroxymethyl-2-phenylpiperidine (0.60g, 1.36mmol) in Hydrogen Chloride, Methanol Reagent 10 (Tokyo Kasei, 10.0ml) was stirred and heated at reflux for 15 minutes; the reaction mixture became homogeneous with heating. The mixture was, then, concentrated *in vacuo*. The pale yellow syrupy residue was basified with sat. Na₂CO₃ aq. solution. The mixture was extracted with CH₂Cl₂. The combined CH₂Cl₂ extracts were dried (Na₂SO₄), and concentrated *in vacuo* to give crude title compound (0.500g, quantitative) as a red oil. IR ₘₐₓ (film): 3380(s), 3300(s), 1700(s), 1683(s), 1603(w), 1585(w), 1495(m), 1130(m), 1077(m), 1048(m), 740(m), 698(s) cm⁻¹; ¹H-NMR δ: 7.43-7.13(m, 10H), 5.20(d, J=5.9Hz, 1H), 5.16(d,.J=12.6Hz, 1H), 5.04(d, J=12.6Hz, 1H), 4.04(dd, J=13.9, 2.0Hz, 1H), 3.60(d, J=7.0Hz, 2H), 3.49(dd, J=13.9, 4.6Hz, 1H), 3.54-3.33(m, 1H), 2.25-2.10(m, 1H), 1.92(ddd, J=13.9, 9.9, 7.3Hz, 1H), 1.65(ddd, J=13.9, 4.8, 4.5Hz, 1H), 1.65-1.26(m, 3H) ppm. This was employed in the next step without further purification.

### (23) (2S*,3S*,5S*)-1-Benzyloxycarbonyl-5-hydroxymethyl-3-[N-(2-methoxy-5-trifluoromethoxybenzyl)amino]-2-phenylpiperidine

To a stirred solution of (2S*,3S*,5S*)-3-amino-1-benzyloxycarbonyl-5-hydroxymethyl-2-phenylpiperidine (0.50g, 1.36mmol) ) and 2-methoxy-5-trifluoromethoxybenzaldehyde (0.37g, 1.70mmol) in CH₂Cl₂ (20.0ml) was added NaB(OAc)₃H (0.43g, 2.04mmol) portionwise. The reaction mixture was stirred at room temperature for 3 hours, and then basified with sat. Na₂CO₃ aq. solution. The mixture was extracted with CH₂Cl₂. The combined extracts were dried (Na₂SO₄), and concentrated *in vacuo* to give a red oil (0.90g), which solidified spontaneously on standing in a refrigerator (4 C). This was flash chromatographed over SiO₂ (Merck Kieselgel 60, 18.0g). Elution with CH₂Cl₂-MeOH (300:1/200:1/100:1) gave title compound (0.678g, 91.6%) as a colorless syrup. IR ₘₐₓ (film): 3450(s), 1698(s), 1682(s), 1670(s), 1498(s), 1230(br., s), 1160(br., s), 1030(s), 738(s), 700(s) cm⁻¹; ¹H-NMR δ: 7.44-7.21 (m, 9H), 7.18(br. s, 1H), 7.04(br. d, J=8.8Hz, 1H), 7. 00(br. s, 1H), 6.92(d, J=8.8Hz, 1H), 5.40-5.27(m, 1H), 5.17(d, J=121.5Hz, 1H), 5.05(d, J=12.5Hz, 1H), 4.03(br.d, J=13.9Hz, 1H), 3.74(d, J=13.9Hz, 1H), 3.70-3.41(m, 4H), 3.62(s, 3H), 3.23-3.11(m, 1H), 2.20-1.95(m, 2H), 1.70-1.45(m, 3H) ppm.

### (24) (2S*,3S*,5S*)-5-Hydroxymethyl-3-[N-(2-methoxy-5-trifluoromethoxybenzyl)amino]-2-phenylpiperidine

To a solution of (2S*,3S*,5S*)-1-benzyloxycarbonyl-5-hydroxymethyl-3-[*N*-(2-methoxy-5-trifluoromethoxybenzyl)amino]-2-phenylpiperidine(0.545g, 1.00mmol) and HCO₂NH₄ (0.252g, 4.00mmol) in MeOH (15.0ml) was added 20% Pd(OH)₂/C (0.159g) in one portion. The mixture was stirred and heated at reflux for 30 minutes. The catalyst was filtered off by the aid of Celite, and washed with MeOH. The combined filtrate and washings were concentrated *in vacuo* to give a yellow syrupy residue, which was basified with 1M NaOH aq. solution. The mixture was extracted CH₂CI₂, and the combined CH₂Cl₂ extracts were dried (Na2SO4), and concentrated *in vacuo* to give a pale yellow oil (0.419g). This was flash chromatographed over SiO₂ (Merck Kieselgel 60, 4.0g). Elution with CH₂Cl₂-MeOH (30:1/20:1) gave title compound(0.380g, 92.7%) as a yellow oil. IR ₘₐₓ (film): 3320(br., s), 1608(m), 1498(s), 1487(s), 1230(br., s), 1160(br., s), 1030(s), 810(m), 740(m), 700(s) cm⁻¹; ¹H-NMR δ: 7.40-7.20(m, 5H), 6.99(dd, J=8.8, 2.9Hz, 1H), 6.85(d, J=2.9Hz, 1H), 6.63(d, J=8.8Hz, 1H), 3.86(d, J=2.2Hz, 1H), 3.64(d, J=14.5Hz, 1H), 3.60-3.47(m, 2H), 3.50(s, 3H), 3.46-3.36(m, 1H), 3.38(d, J=14.5Hz, 1H), 2.92-2.84(m, 1H), 2.54(dd, J=1.5, 11.5Hz, 1H), 2.28-2.10(m, 1H), 2.12(dm, J=12.9Hz, 1H), 1.69(br.s, 3H; N*H*x2+O*H*), 1.35(ddd, J=12.9, 12.9, 2.8Hz, 1H) ppm.

### (25) (2S*,3S*,5S*)-5-Hydroxymethyl-3-[N-(2-methoxy-5-trifluoromethoxybenzyl)amino]-2-phenylpiperidine dihydrochloride

To a solution of (2S*, 3S*, 5S*)-5-hydroxymethyl-3-[*N*-(2-methoxy-5-trifluoromethoxybenzyl)amino]-2-phenylpiperidine(0.455g, 1. 11mmol) in *i*-PrOH was added Hydrogen Chloride, Methanol Reagent 10 (Tokyo Kasei), until the mixture became acidic (pH∼2). The volatiles were evaporated *in vacuo*. The yellow syrupy residue was crystallized from (*i*-Pr)₂O. After the mixture was left to stand in a refrigirator (at 4 C) overnight, the white crystals were collected by filtration, washed with (*i*-Pr)₂O, and dried *in vacuo* to give title hydrochloride (0.460g, 86.0%). An additional amount (0.021g, 3.9%) of title hydrochloride was obtained as a second crop; a total yield: 0.481g (89.9%). mp 188.0-192.0 C (white powders); IR ₘₐₓ (nujol): 3450(s), 3250-2320(br., s), 1580(m), 1555(s), 1502(s), 1260(s), 1213(s), 1030(s), 817(m), 755(s), 700(s) cm⁻¹; ¹H-NMR δ(DMSO-d₆): 7.75-7.62(m, 2H), 7.58-7.41(m, 3H), 7.40-7.30(m,2H), 7.03(d, J=9.5Hz, 1H), 4.89(br.s, 1H), 4.10-3.79(m, 2H), 3.70(br.s, 3H), 3.57-3.10(m, 9H), 2.97(br.dd, J=11.6, 11.6Hz, 1H), 2.60-2.40(m, 1H), 2.35-2.17(m, 1H), 2.00-1.78(m, 1H) ppm.

### (26) (2R*,3S*,5R*)-1-Benzyloxycarbonyl-5-(tert-butyldimethylsilyloxy)methyl-3-methoxycarbonyl-2-phenylpiperidine

To a stirred and ice-cooled mixture of (2R*,3S*,5R*)-5-(*tert*-butyldimethylsilyloxy)methyl-3-methoxycarbonyl-2-phenylpiperidine (4.86g, 13.4mmol), NaHCO₃ (1.68g, 20.1mmol), THF (5.0ml), and H₂O (30.0ml) was added Cbz-Cl (2.51g, 14.7mmol), and the mixture was stirred with ice-cooling for 30 minutes. The mixture was extracted with AcOEt (40.0ml x 3). The combined AcOEt extracts were washed with sat. NaCl aq. solution (x1), dried (MgSO₄), and concentrated *in vacuo* to give a viscous pale yellow oil (6.82g). This was flash chromatographed over SiO₂ (Merck Kieselgel 60, 80.0g). Elution with *n*-hexane-AcOEt (30:1/20:1) gave title compound (6.51g, 97.9%) as a viscous colorless oil. IR ₘₐₓ (film): 1735(s), 1700(s), 1605(w), 1585(w), 1495(m), 1250(s), 1115(s), 775(s), 740(m), 700(s) cm⁻¹; ¹H-NMR δ: 7.45-7.31(m, 4H), 7.31-7.15(m, 6H), 6.05(d, J=5.5Hz, 0.6H), 5.87(d, J=5.5Hz, 0.4H), 5.32-5.10(5.30, 5.25, 5.21, 5.18, and 5.13; m, 2H), 4.20-4.05(m, 1H), 3.65-3.45(m, 2.4H), 3.60(br.s, 3H), 3.40(dd, J=9.9, 6.6Hz, 0.6H), 3.08-2.91(m, 1H), 2.63(dd, J=11.7, 11.7Hz, 0.4H), 2.52(dd, J=13.6, 11.7Hz, 0.6H), 2.11-1.98(m, 1H), 1.98-1.70(m, 2H), 0.86(s, 3.6H), 0.83(s, 5.4H), 0.026, 0.018, and 0.008(each s, total 6H) ppm.

### (27) (2R*,3S*,5R*)-1-Benzyloxycarbonyl-5-(tert-butyldimethylsilyloxy)-methyl-3-carbamoyl-2-phenylpiperidine

Under an atmosphere of N₂, a 2.0M solution of Me₃Al in hexanes (Aldrich, 66.0ml, 132mmol) was added dropwise to a stirred and ice-cooled suspension of NH₄Cl (7.00g, 131mmol) in dry PhMe (130.0ml). After the addition was complete, the reaction mixture was allowed to stirr at room temperature. At the point when evolution of H₂ gas ceased, the mixture became gelatinous. Dry PhMe (20.0ml) was added, and the stirring was continued at room temperature for 30 minutes. To this white slurry was added dropwise a solution of (2R*,3S*,5R*)-1-benzyloxycarbonyl-5-(*tert*-butyldimethylsilyloxy)methyl-3-methoxycarbonyl-2-phenylpiperidine (6.51g, 13.1mmol) in dry PhMe (20.0ml). The reaction mixture was stirred and heated at a bath temperature of 50 C overnight (ca. 21hours); the mixture became homogeneous with stirring, and then turbid again. The mixture was ice-cooled, and H₂O (40.0ml) was added carefully. The resultant gelatinous white mixture was filtered through a pad Celite, and the filter cake was washed with AcOEt (80ml x 4). The combined filtrate and AcOEt washings were washed with sat. NaCl aq. solution (x2), dried (MgSO₄), and concentrated *in vacuo* to give a viscous colorless oil (6.56g), which crystallized spontaneously on standing at room temperature. These solids were recrystallized from AcOEt-n-hexane to give title compound (4.75g, 75.2%). The recovered pale yellow mother liquor (1.16g) was purified by flash chromatography [SiO₂, Merck Kieselgel 60, 15.0g; *n*-hexane-AcOEt (8:1/5:1/1:1)] to give an additional amount (0.44g, 7.0%) of title compound; a total yield:5.19g (82.1%). mp 120.0-122.5 C (fine colorless needles); IR ₘₐₓ (nujol): 3390(s), 3180(m), 1673(v.s), 1630(shoulder, m), 1500(w), 1162(s), 1100(s), 838(s), 773(m), 735(m), 695(s) cm⁻¹; ¹H-NMR δ: 7.55-7.21(m, 10H), 5.91(d, J=5.1Hz, 0.7H), 5.84(br.s, 0.7H), 5.79(d, J=4.8Hz, 0.3H), 5.42(br.s, 1.3H), 5.34(d, J=12.5Hz, 0.3H), 5.24(d, J=12.2Hz, 0.7H), 5.19(d, J=12.2Hz, 0.7H), 5.13(d, J=12.5Hz, 0.3H), 4.24-4.04(m, 1H), 3.57(dd, J=9.9, 4.4Hz, 1.3H), 3.39(dd, J=10.3, 6.6Hz, 0.7H), 3.00-2.85(m, 1H), 2.77(dd, J=13.2, 10.6Hz, 0.3H), 2.53(dd, J=13.6, 11.4Hz, 0.7H), 2.10-1.70(m, 3H), 0.87(s, 3H), 0.83(3, 6H), 0.03(s, 2H), 0.01 (s, 4H) ppm.

### (28) (2S*,3S*,5R*)-1-Benzyloxycarbonyl-3-(N-tert- butoxycarbonyl)amino-5-(tert-butyldimethylsilyloxy)methyl-2-phenylpiperidine

Solid (2R*,3S*,SR*)-1-benzyloxycarbonyl-5-(*tert*- butyldimethylsilyloxy)methyl-3-carbamoyl-2-phenylpiperidine (5.19g, 10.8mmol) was dissolved in *tert*-BuOH (100ml) by heating to a bath temperature of 70 C. To the resultant homogeneous mixture was added Pb(OAc)₄ (90%, 8.10g, 18.3mmol) in one portion. The red reaction mixture was stirred and heated at reflux for 45 minutes. The dark brown mixture was ice-cooled, and then neutralized with sat. NaHCO₃ aq. solution (ca. 90ml). The inorganic dark brown precipitates were filtered off by the aid of Celite, and washed with AcOEt. The combined filtrate and AcOEt washings were concentrated *in vacuo*, and the aqueous residue was extracted with AcOEt (50.0ml x 4). The combined AcOEt extracts were washed with sat. NaCl aq. solution (x1), dried (MgSO₄), and concentrated *in vacuo* to give crude title compound (6.35g, quantitative) as a viscous pale yellow oil. IR ₘₐₓ (film): 3450(shoulder, m), 3350(s), 1718(v.s), 1700(v.s), 1682(v.s), 1250(s), 1165(s), 1110(s), 838(s), 778(s), 700(s) cm⁻¹; ¹H-NMR δ: 7.51-7.19(m, 10H), 7.13(br.s, 1H), 5.59-5.37(m, 1H), 5.19, and 5.14 (each br.s, total 1H), 5.11-4.94(m, 1H), 4.26-4.00(m, 2H), 3.71-3.46(m, 2H), 3.26-3.00(m, 1H), 2.06-1.70(m, 2H), 1.40(br.s, 9H), 0.91(br.s, 9H), 0.05(br.s, 6H) ppm. This was employed in the next step without further purification.

### (29) (2S*,3S*,5R*)-1-Benzyloxycarbonyl-3-(N-tert-butoxycarbonyl)amino-5-hydroxymethyl-2-phenylpiperidine

To a stirred and ice-cooled solution of (2S*,3S*,5R*)-1-benzyloxycarbonyl-3-(*N-tert*-butoxycarbonyl)amino-5-(*tert*-butyldimethylsilyloxy)methyl-2-phenylpiperid-ine (6.35g, 10.8mmol) in dry THF (45.0ml) was added a 1.0M solution of (*n*-Bu)₄NF in THF (Aldrich, 13.0ml, 13.0mmol) dropwise. After the resultant orange reaction mixture was stirred at room temperature for an hour, MS 4A (activated powder, Aldrich; 8.0g) was added. The stirring was continued at room temperature for an additional hour. The zeolite was filtered off by the aid of Celite, and washed with THF. The combined filtrate and THF washings were adjusted to neutral with 1.0M AcOH aq. solution and sat. NaHCO₃ aq. solution. The mixture was concentrated *in vacuo*, and the aqueous residue was extracted with CH₂Cl₂ (20.0ml x 4). The combined CH₂Cl₂ extracts were washed with sat. NaCl aq. solution (x1), dried (MgSO₄), and concentrated *in vacuo* to give an orange oil (7.47g). This was flash chromatographed over SiO₂ (Merck Kieselgel 60, 75.0g). Elution with CH₂Cl₂-MeOH (200:1/150:1/100:1) gave a white solid (4.71g), which was recrystallized from AcOEt-*n*-hexane to give title compound [4.11g, overall 86.5% from(2R*,3S*,5R*)-1-benzyloxycarbonyl-5-(*tert*- butyldimethylsilyloxy)methyl-3-carbamoyl-2-phenylpiperidine in two steps] as white powders. mp 132.0-134.0 C; IR ₘₐₓ (nujol): 3485(s), 3303(s), 1690 (shoulder, v.s), 1675(v.s), 1605(w), 1538(v.s), 1500(m), 1288(s), 1170(s), 1075(s), 733(s), 700(s) cm⁻¹; ¹H-NMR δ: 7.48-7.20(m, 10H), 7.16(br.s, 1H), 5.48(br.s, 1H), 5.17(br.s, 0.25H), 5.13(br.s, 0.75H), 5.05(br.s, 0.75H), 5..00(br.s, 0.25H), 4.37-4.00(m, 2H), 3.73-3.56(m, 2H), 3.03(dd, J=12.8, 12.8Hz, 1H), 2.10-1.90(m, 1H), 2.05-1.82(m, 1H), 1.68-1.47(m, 2H), 1.40(br.s, 9H) ppm.

### (30) (2S*,3S*,5R*)-1-Benzyloxycarbonyl-3-(N-tert-butoxycarbonyl)amino-5-formyl-2-phenylpiperidine

To a stirred and ice-cooled solution of (2S*,3S*,5R*)-1-benzyloxycarbonyl-3-(*N*-*tert*-butoxycarbonyl)amino-5-hydroxymethyl-2-phenylpiperidine(0.50g,1.14mmol), and Et₃N (0.71ml, *d*0.726, 5.11ml) in DMSO (8.0ml) was added SO₃ pyridine (0.81g, 5.11 mol) portinwise. After the addition was complete, the reaction mixture was stirred at room temperature for an hour. The mixture was poured into ice-water (10.0ml), and the mixture was extracted with CH₂Cl₂ (x5). The combined CH₂Cl₂ extracts were washed with 10% citric acid aq. solution (x3), H₂O (x1), sat. NaHCO₃ aq. solution (x1), and sat. NaCl aqq. solution (x1), dried (MgSO₄), and concentrated *in vacuo* to give title compound (0.535g, quantitative) as a white solid. This was employed in the next step without further purification. A portuin of it (0.063g) was recrystallized from AcOEt-*n*-hexane to give an analytical sample of title compound (0.044g). mp 124.0-125.0 C (white powders); IR ₘₐₓ (nujol): 3300(s), 1720(shoulder, s), 1710(v.s), 1695(v.s), 1675(v.s), 1605(w), 1537(s), 1500(m), 1288(s), 1170(s), 1072(s), 773(m), 736(s), 698(s) cm⁻¹; ¹H-NMR δ: 9.79(s, 0.12H), 9.73(s, 0.88H), 7.40-7.15(m, 11H), 5.50(br.d, J=5.5Hz, 0.88H), 5.46(br.d, J=5.5Hz, 0.12H), 5.17(d, J=12.5Hz, 1H), 5.04(d, J=12.5Hz, 1H), 4.60(dm, J=14.1Hz, 0.12H), 4.45(dm, J=14.1Hz, 0.88H), 4.32-3.90(m, 1H), 3.21(dd, J=13.9, 11.7Hz, 1H), 2.90-2.68(m, 1H), 2.45(dm, J=12.7Hz, 0.12H), 2.27(dm, J=12.7Hz, 0.88H), 1.80(ddd, J=12.7, 12.7, 12.7Hz, 1H), 1.41(br.s, 9H) ppm.

### (31) (2S*,3S*,5R*)-1-Benzyloxycarbonyl-3-(N-tert-butoxycarbonyl)amino-5-methoxycarbonyl-2-phenylpiperidine

To a stirred and ice-cooled mixture of (2S*,3S*,5R*)-1-benzyloxycarbonyl-3-(*N*-*tert*-butoxycarbonyl)amino-5-formyl-2-phenylpiperidine (0.45g, 1.03mmol), NaHCO₃ (3.35g, 39.9mmol), THF (3.0ml), and MeOH-H₂O (9:1, 12.0ml) was added dropwise a 2.0M stock solution of Br₂ in MeOH-H₂O (9:1, 3.99ml. 7.98mmol). After the resultant red orange reaction mixture was stirred at room temperature for 2 hours and 35 minutes, the mixtre was ice-cooled, and then solid Na₂S₂O₃ was added to discharge the orange color of the excess Br₂. The inorganic precipitates were filtered off by the aid of Celite, and washed with CH₂Cl₂. The combined filtrate and CH₂Cl₂ washings were concentrated *in vacuo*. The residue was diluted with H₂O, and extracted with CH₂Cl₂. The combined CH₂Cl₂ extracts were dried (MgSO₄), and concentrated *in vacuo* to give a white solid (0.52g). This was flash chromatographed over SiO₂ (Merck Kieselgel 60, 13.0g). Elution with CH₂Cl₂-AcOEt (150:1/100:1) gave title compound (0.34g, 70.8%) as a white solid. A portion of it was recrystallized from AcOEt-*n*-hexane to give an analytically pure sample of the title compound as fine white needles. mp 128.0-129.0 C; IR ₘₐₓ (nujol): 3420(s), 3310(m), 1738(v.s), 1700(v.s), 1672(v.s), 1610(w), 1590(w), 1515(m), 1510(v.s), 1220(v.s), 1170(s), 1153(s), 775(s), 755(s), 710(s), 700(s) cm⁻¹; ¹H-NMR δ: 7.48-7.15(m, 11H), 5.56-5.41(m, 1H), 5.16(d, J=12.6Hz, 1H), 5.04(d, J=12.6Hz, 1H), 4.50-4.00(m, 2H), 3.72(s, 3H), 3.26(dd, J=13.6, 12.5Hz, 1H), 2.80-2.70(m, 1H), 2.30-2.15(m,1H), 1.89(ddd, J=12.7, 12.7, 12,7Hz, 1H), 1.40(br.s, 9H) ppm.

### (32) (2S*,3S*,5R*)-3-Amino-1-benzyloxycarbonyl-5-methoxycarbonyl-2-phenylpiperidine

A suspension of (2S*,3S*,5R*)-1-benzyloxycarbonyl-3-(*N*- *tert*-butoxycarbonyl)amino-5-methoxycarbonyl-2-phenylpiperidine (1.11g, 2.37mmol) in Hydrogen Chloride, Methanol Reagent 10 (Tokyo Kasei; 20.0ml) was stirred and heated at reflux for 30 minutes; violent evolution of CO₂ gas took place with heating, and the reaction mixure became homogeneous. The reaction mixture was, then, concentrated *in vacuo* to give a solid residue, which was basified with sat. Na₂CO₃ aq. solution. The mixture was extracted with CH₂Cl₂, and the combined CH₂Cl₂ extracts were dried (Na₂SO₄), and concentrated *in vacuo* to give crude title compound (0.777g, 89.0%) as a viscous pale yellow oil. IR ₘₐₓ (film): 3380(m), 3315(w), 1735(s), 1720(s), 1700(s), 1685(s), 1603(m), 1584(m), 1497(s), 1200(s), 765(s), 750(s), 740(s), 700(s) cm⁻¹; ¹H-NMR δ: 7.58-7.16(m, 10H), 5.45-5.23(m, 1H), 5.20-5.04(m, 2H), 4.55-4.26(m, 1H), 3.73(s, 3H), 3.36-3.22(m, 2H), 2.83-2.65(m, 1H), 2.20-2.02(m, 1H), 1.97(ddd, J= 12.5, 12.5, 12.5Hz, 1H), 1.43(br.s, 2H) ppm. This was employed in the next step without further purification.

### (33) (2S*,3S*,5R*)-1-Benzyloxycarbonyl-5-methoxycarbonyl-3-[N-(2-methoxy-5-trifluoromethoxybenzyl)amino]-2-phenylpiperidine

To a stirred solution of (2S*,3S*,5R*)-3-amino-1-benzyloxycarbonyl-5-methoxycarbonyl-2-phenylpiperidine (0.84g, 2.28mmol) and 2-methoxy-5-trifluoromethoxybenzaldehyde (0.63g, 2.85mmol) in CH₂Cl₂ (18.0ml) was added NaB(OAc)₃H (0.73g, 3.42mmol) potionwise at room temperature. After the turbid reactin mixture was stirred vigorously at room temperature for 4 hours, sat. Na₂CO₃ aq. solution (15.0ml) was added to make the mixture to be basic (pH10-11). The CH₂Cl₂ layer was separated from the mixture, and the aqueos layer was extracted with CH₂Cl₂ (x4). The combined CH₂Cl₂ layer and extracts were dried (Na₂SO₄), and concentrated *in vacuo* to give a viscous pale yellow oil (1.49g). This was chromatographed over SiO₂ (Merck Kieselgel 60, 30.0g). Elution with CH₂Cl₂-MeOH (300:1/250:1) gave title compound (1.26g, 96.2%) as a viscous colorless oil. IR νₘₐₓ (film): 3450(w), 3330(w), 1740(shoulder, s), 1735(s), 1703(s), 1700(s), 1687(s), 1610(w), 1497(s), 1160(br., s), 1030(s), 815(m), 765(s), 740(s), 700(s) cm⁻¹; ¹H-NMR δ: 7.65-7.50(m, 1H), 7.52-7.38(m, 1H), 7.40-7.11(m, 8H), 7.12-6.96(m, 2H), 6.74(br.d, J=8.8Hz, 1H), 5.45-5.34(m, 0.5H), 5.20-5.00(m, 2H), 5.17-5.05(m, 0.5H), 4.49-4.33(m, 0.5H), 4.35-4.17(m, 0.5H), 3.90-3.53(m, 2H), 3.69(br.s, 6H), 3.20(br.dd, J=13.2, 13.2Hz, 1H), 3.20-3.00(m, 1H), 2.83-2.60(m, 1H), 2.26-1.99(m,2H), 1.52(br.s, 1H) ppm.

### (34) (2S*,3S*,5R*)-5-Methoxycarbonyl-3-[N-(2-methoxy-5-trifluoromethoxybenzyl)amino]-2-phenylpiperidine

A mixture of (2S*,3S*,5R*)-1-benzyloxycarbonyl-5-methoxycarbonyl-3-[*N*-(2-methoxy-5-trifluoromethoxybenzyl)amino]-2-phenylpiperidine (1.26g, 2.20mmol) and 20% Pd(OH)₂/C (0.30g) in MeOH (20.0ml) was stirred at room temperature under an atmosphere of H₂ (baloon) for 5.5 hours. An additional amount (0.20g) of 20% Pd(OH)₂/C was added, and the stirring was continued under an atmosphere of H₂ (baloon) for an hour. The catalyst was filtered off by the aid of Celite, and washed with CH₂Cl₂. The combined filtrate and washings were concentrated *in vacuo*. The yelow oily residue was diluted with CH₂Cl₂, washed with sat. Na₂CO₃ aq. solution (x1), dried (Na₂SO₄), and concentrated *in vacuo* to give a viscous yellow oil (1.00g). This was chromatographed over SiO₂ (Merck Kieselgel 60, 10.0g). Elution with CH₂Cl₂-MeOH (250:1/50:1) gave title compound (0.873g, 90.6%) as a viscous yellow oil. IR ₘₐₓ (film): 3320(m), 1735(s), 1720(s), 1605(m), 1508(s), 1498(s), 1490(s), 1133(s), 810(s), 762(s), 741(s), 700(s) cm⁻¹; ¹H-NMR δ: 7.33-7.18(m, 4H), 7.17-7.10(m,1H), 7.01(dd, J=9.2, 2.6Hz, 1H), 6.87(d, J=2.6Hz, 1H), 6.60(d, J=9.2Hz, 1H), 3.94(d, J=1.7Hz, 1H), 3.77(ddd, J=13.6, 1.9, 1.9Hz, 1H), 3.94(d, J=1.7Hz, 1H), 3.77(ddd, J=13.6, 1.9, 1.9Hz, 1H), 3.66(s, 3H), 3.65(d, J=13.9Hz, 1H), 3.39(d, 13.9Hz, 1H), 3.36(s, 3H), 2.88(dd, J = 13.6, 3.7Hz, 1H), 2.84-2.73(m, 2H), 2.37-2.29(m, 1H), 1.95(ddd, J=15.0, 5.5, 3.3Hz, 1H), 1.68(br.s, 2H) ppm.

### (35) (2S*,3S*,5R*)-5-Methoxycarbonyl-3-[N-(2-methoxy-5-trifluoromethoxybenzyl)amino]-2-phenylpiperidine fumarate

To a solution of (2S*,3S*,5R*)-5-methoxycarbonyl-3-[*N*-(2-methoxy-5-trifluoromethoxybenzyl)amino]-2-phenylpiperidine (0.194g, 0.443mmol) in EtOH (3.0ml) was added fumaric acid (0.103g, 0.886mmol) in one portion. An additional volume of EtOH was added with heating at reflux until the mixture became homogeneous. The resultant clear solution was left to stand at room temperature, wherein recrystallization took place. After Et₂O (0.50ml) was added, the mixture was kept in a refrigerator. The white solids precipitated were collected by filtration, washed with Et₂O (x1), and dried *in vacuo* with heating at 50 C to give title fumarate salt(0.197g, 80.1%) as colorless fine needles. mp 176.7-176.9 C; IR ₘₐₓ (nujol): 3200-2100(br., s), 1749(s), 1704(s), 1693(s), 1655(w), 1643(w), 1548(s), 1500(m), 1268(s), 1253(s), 1208(s), 1182(s), 1158(s), 990(s), 750(s), 700(s) cm⁻¹; ¹H-NMR δ (DMSO-d₆): 7.40-7.16(m, 5H), 7.15(dd, J=8.8, 2.6Hz, 1H), 6.94(d, J=2.6Hz, 1H), 6.87(d, J=8.8Hz, 1H), 6.58(s, 2H), 4.07-3.97(m, 1H), 4.00-3.25(m, 8H), 3.56(s, 3H), 3.44(s, 3H), 2.91-2,80(m, 1H), 2.79-2.71(m, 1H), 2.66-2.54(m, 1H), 2.01-1.87(m, 1H) ppm.

### (36) (2S*,3S*,5R*)-5-Carboxy-3-[N-(2-methoxy-5-trifluoromethoxybenzyl)amino]-2-phenylpiperidine dihydrochloride

A mixture of (2S*,3S*,5R*)-5-methoxycarbonyl-3-[*N*-(2-methoxy-5-trifluoromethoxybenzyl)amino]-2-phenylpiperidine (0.241g, 0.551mmol) in conc. HCl (3.0ml) was stirred and heated ar reflux for 30 minutes. After cooling to room temperature, the dark orange mixture was filtered through a short pad of Celite to remove a black precipitate. The filtrate was concentrated *in vacuo*. The syrupy dark red residue was dissolved in acetone. After (*i*-Pr)₂O was added, the mixture was subjected to sonication to induce crystalization. The mixture was left to stand in a refrigirator (at 4 C) overnight. The solids precipitated were collected by filtration, washed with Et₂O, and dried *in vacuo* with heating at 50 C to give the title dihydrochloride salt (0.103g, 37.6%). The combined filtrate and Et₂O washings were concentrated *in vacuo*, and the dark red syrupy residue was crystallized from *i*-PrOH-(*i*-Pr)₂O to give an additional amount (0.041g) of the title dihydrochloride salt; a total yield: 0.144g (52.5%). 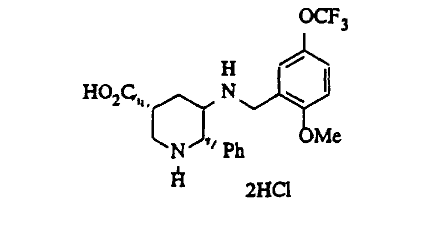 mp 179.9-182.9 C (colorless short prisms); IR ₘₐₓ (nujol): 3250-2100(br., s), 1758(w), 1720(s), 1502(s), 1255(s) cm⁻¹; ¹H-NMR δ (DMSO-d₆): 7.83-7.65(m, 2H), 7.64-7.77(m, 3H), 7.43-7.29(m, 2H), 7.15-6.98(m, 1H), 5.17-4.88(m, 1H), 4.15-3.80(m, 2H), 3.75-3.60(m, 5H), 3.62-3.05(m, 7H), 2.77-2.20(m, 2H) ppm.

### (37) (2S*,3S*,5R*)-3-Amino-1-benzyloxycarbonyl-5-hydroxymethyl-2-phenylpiperidine

To a stirred solution of (2S*,3S*,5R*)-1-benzyloxycarbonyl-3-(*N*-*tert*-butoxycarbonyl)amino-5-hydroxymethyl-2-phenylpiperidine (0.50g, 1.14mmol) in AcOEt (10.0ml) was added conc. HCl (5.0ml) dropwise with occasional ice-cooling. After stirring at room temperature for 40 minutes, the mixture was concentrated *in vacuo* to give a yellow syrupy residue, which was, then, basified to pH 10 with 1M NaOH aq. solution. The mixture was extracted with CH₂Cl₂, and the combined CH₂Cl₂ extracts were dried (K₂CO₃), and concentrated *in vacuo* to give crude title compound (0.405g, quantitative) as a pale yellow syrup. IR ₘₐₓ (film): 3360(s), 3300(s), 1693(shoulder, v.s), 1685(v.s), 1603(m), 1585(m), 1497(s), 1263(s), 1150(s), 1070(s), 760(s), 739(s), 700(s) cm⁻¹; ¹H-NMR δ: 5.42-5.19(m, 1H), 5.16-5.00(m, 2H), 4.35-4.14(m, 1H), 3.70-3.53(m, 2H), 3.38-3.25(m, 1H), 3.04(dd, J=13.2, 12.5Hz, 1H), 2.15-1.94(m, 1H), 1.86(ddd, J=13.2, 4.8, 4.2Hz, 1H), 1.75-1.35(m, 4H) ppm. This was employed in the next step without further purification.

### (38) (2S*,3S*,5R*)-1-Benzyloxycarbonyl-5-hydroxymethyl-3-[N-(2-methoxy-5-trifluoromethoxybenzyl)amino]-2-phenylpiperidine

To a stirred solution of (2S*,3S*,SR*)-3-amino-1-benzyloxycarbonyl-5-hydroxymethyl-2-phenylpiperidine (0.405g, 1.14mmol) and 3-methoxy-5-trifluoromethoxybenzaldehyde (0.301g, 1.71mmol) in dry CH₂Cl₂ (25.0ml) was added NaB(OAc)₃H (0.362g, 1.71mmol) portionwise. The mixture was sonicated to dissolve the NaB(OAc)₃H added, and then stirred at room temperature for 2 hours and 40 minutes. To this was added sat. Na₂CO₃ aq. solution (12.0ml), and the CH₂Cl₂ layer was separated. The aqueous layer was extracted with CH₂Cl₂. The combined CH₂Cl₂ layer and extracts were dried (K₂CO₃), and concentrated *in vacuo* to give a pale yellow oil (0.72g). This was flash chromatographed over SiO₂ (Merch Kieselgel 60, 14.0g). Elution with CH₂Cl₂-MeOH (300:1/200:1/100:1) gave title compound [0.416g, overall 67.0% from (2S*,3S*,5R*)-1-benzyloxycarbonyl-3-(*N*-*tert*-butoxy-carbonyl)amino-5-hydroxymethyl-2-phenylpiperidine in two steps] as a colorless syrup. IR ₘₐₓ (film): 3450(s), 1700(s), 1682(s), 1670(s), 1652(s), 1610(w), 1497(s), 1490(s), 1260(br., s), 1220(br., s), 1152(br., s), 1030(s), 738(s), 700(s) cm⁻¹; ¹H-NMR δ: 7.50-7.11(m, 10H), 7.13-6.93(m, 1H), 6.69(br.d, J=8.4Hz, 1H), 5.60-5.22(m, 1H), 5.20-4.95(m, 2H), 4.36-4.10(m, 1H), 3.79(d, J=13.9Hz, 1H), 3.65-3.45(m, 3H), 3.59(br.s, 3H), 3.23-2.95(m, 2H), 2.15(br.s, 1H), 2.02-1.86(m, 1H), 1.86-1.68(m, 1H), 1.68-1.45(m, 2H) ppm.

### (39) (2S*,3S*,5R*)-5-Hydroxymethyl-3-[N-(2-methoxy-5-trifluoromethoxybenzyl)amino]-2-phenylpiperidine

A mixture of (2S*,3S*,5R*)-1-benzyloxycarbonyl-5-hydroxymethyl-3-[*N*-(2-methoxy-5-trifluoromethoxybenzyl)- amino]-2-phenylpiperidine (0.416g, 0.76mmol), 20% Pd(OH)₂/C (0.11g), and MeOH (10.0ml) was stirred at room temperature under an atmosphere of H₂ (baloon) for 90 minutes. An additional amount of 20% Pd(OH)2/C (0.08g) was added, and the stirring was continued under an atmosphere of H₂ (baloon) for 45 minutes. The catalyst was filtered off by the aid of Celite, and washed with MeOH. The combined filtrate and washings were concentrated *in vacuo* to give a yellow semi-solid (0.309g). This was dissolved in MeOH (3.0ml) again, and the mixture was basified with 1M NaOH aq. solution. The solvent MeOH was evaporated *in vacuo*, and the aqueous residue was extracted with CH₂Cl₂. The combined CH₂Cl₂ extracts were dried (K₂CO₃), and concentrated *in vacuo* to give title compound (0.298g, 95.5%) as a yellow syrup. IR ₘₐₓ (film): 3310(s), 3070(br.s,), 1605(w), 1498(s), 1250(s), 1150(s), 1035(s), 756(s), 700(s) cm⁻¹; ¹H-NMR δ: 7.34-7.16(m, 4H), 7.15-7.03(m, 2H), 6.85(d, J=2.6Hz, 1H), 6.62(d, J=8.8Hz, 1H), 3.92(d, J=2.6Hz, 1H), 3.91-3.71(m, 3H), 3.45(d, J=11.4Hz, 1H), 3.40(d, J=11.4Hz, 1H), 3.35(s, 3H), 3.11(ddd, J=13.6, 4.8, 1.5Hz, 1H), 2.72(ddd, J=2.9, 2.9, 2.6Hz, 1H), 2.25-2.07(m, 2H), 1.85(br.s, 3H; OH+NHx2), 1.75-1.65(m, 1H) ppm.

### (40) (2S*,3S*,5R*)-5-Hydroxymethyl-3-[N-(2-methoxy-5-trifluoromethoxybenzyl)amino]-2-phenylpiperidine difumarate

To a solution of (2S*,3S*,5R*)-5-hydroxymethyl-3-[*N*-(2-methoxy-5-trifluoromethoxybenzyl)amino]-2-phenylpiperidine (0.298g, 0.726mmol) in EtOH (3.0ml) was added fumaric acid (0.169g, 1.45mmol) in one portion. An additional volume of EtOH was added with heating at reflux, until the mixture became homogeneous. After the resultant pale yellow solution was allowed to cool to room temperature, (*i*-Pr)₂O (2.0ml) was added. After the mixture was left to stand in a refrigirator (at 4 C) overnight, and then the precipitated white solids were collected by filtration, washed with (*i*-Pr)₂O-*i*-PrOH, and dried *in vacuo* with heating at 50 C to give title difumarete (0.382g, 82.1 %) as white powders. 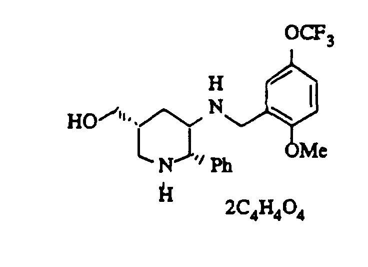 mp 162.0-165.0 C; IR ₘₐₓ (nujol): 2565(br., s), 17179s), 1705(s), 1641(s), 1630(s), 1561(s), 1502(s), 1280(s), 1250(s), 1162(s), 973(s), 790(m), 751(s), 648(s) cm⁻¹; ¹H-NMR δ(DMSO-d₆): 7.45-7.33(m, 4H), 7.22-7.12(m, 1H), 7.18-7.11(m, 2H), 6.91(d, J=8.8Hz, 1H), 6.54(s, 4H), 4.29(br.s, 1H), 3.88-3.13(m, 13H), 3.56(s, 3H), 3.06-2.96(m, 1H), 2.90-2.83(m, 1H), 2.07-1.95(m, 1H), 1.93-1.80(m, 1H) ppm.

## Claims

1. A compound of the chemical formula (I) and its pharmaceutically acceptable salts: 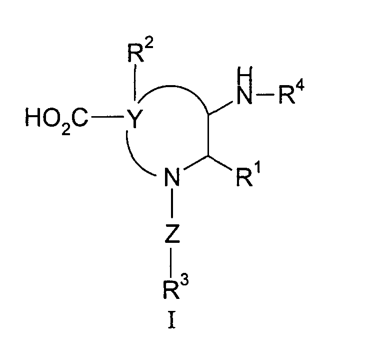 wherein Y is C₂-C₄ alkylene;
Z is a valence bond or C₁-C₆ alkylene;
R¹ is phenyl, biphenyl, indanyl, naphthyl, thienyl, furyl, pyridyl, thiazolyl, isothiazolyl, oxazolyl, isoxazolyl, tetrazolyl, quinolyl, phenyl C₁-C₆ alkyl- or benzhydryl, wherein each of the ring moieties may optionally be substituted by one or more substituents independently selected from halogen, C₁-C₆ alkyl, halosubstituted C₁-C₆ alkyl, C₁-C₆ alkoxy and halosubstituted C₁-C₆ alkoxy;
R² is hydrogen or C₁-C₆ alkyl;
R³ is hydrogen, hydroxy, cyano, amino or carboxy; and
R⁴ represents a group of the formula (II) 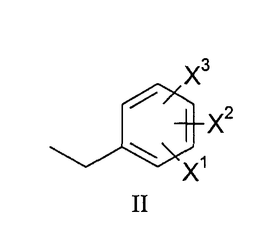 wherein X¹, X² and X³ are each halo, hydrogen, nitro, C₁-C₆ alkyl, halosubstituted C₁-C₆ alkyl, C₁-C₆ alkoxy, halosubstituted C₁-C₆ alkoxy, hydroxy, amino, C₁-C₆ alkylthio, C₁-C₆ alkylsulfinyl or C₁-C₆ alkylsulfonyl;
provided that (i) when Z is a valence bond, R₃ is hydrogen and (ii) when Z is a valence bond, R³ is hydrogen, Y is C₃ alkylene, R² is H and R¹ is phenyl, then R⁴ is other than a group of the formula:- 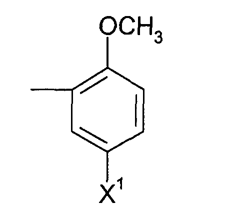 wherein X¹ is (C₁-C₅) alkyl, methylthio, methylsulfinyl or methylsulfonyl.

2. A compound according to claim 1, wherein Y is ethylene or propylene.

3. A compound according to any one of the preceding claims, wherein -ZR³ is hydrogen.

4. A compound according to any one of the preceding claims, wherein R¹ is phenyl.

5. A compound according to any one of the preceding claims, wherein R² is attached to the carbon atom adjacent to the nitrogen of NZR³.

6. A compound of claim 1 selected from

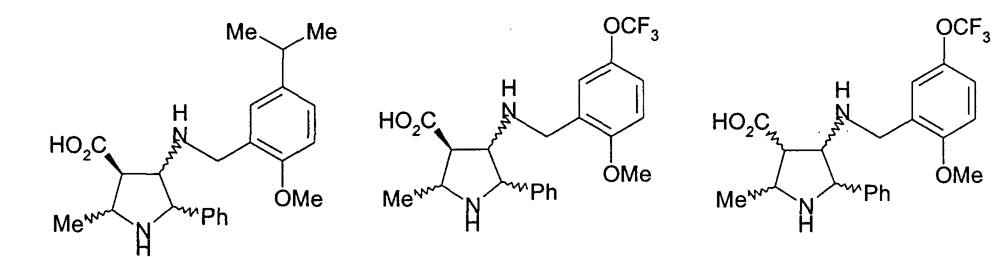

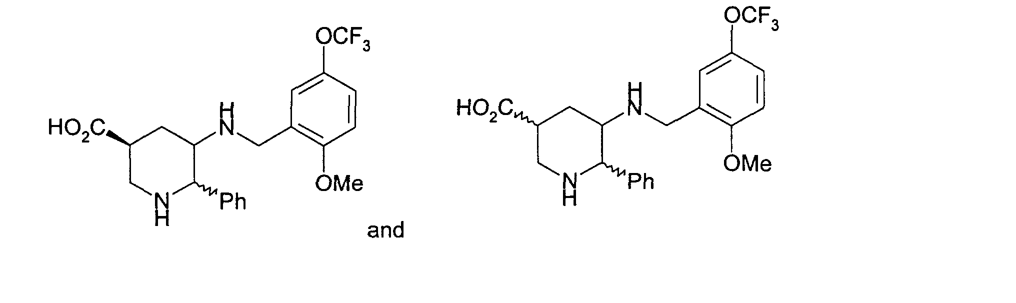

7. A pharmaceutical composition which comprises a compound of the formula (I) as claimed in any one of the preceding claims or a pharmaceutically acceptable salt thereof together with a pharmaceutically acceptable carrier.

8. A compound of the formula (I) or pharmaceutically acceptable salt thereof as claimed in any one of claims 1 to 6 for use as a medicament.

9. The use of a compound of the fomula (I) as claimed in any one of claims 1 to 6 or of a pharmaceutially acceptable salt thereof for the manufacture of a medicament for treating or preventing gastrointestinal disorders, central nervous system disorders, allergy, inflammatory diseases, asthma, pain, migraine or emesis.

## Patentansprüche

1. Verbindung der chemischen Formel (I) und ihre pharmazeutisch annehmbaren Salze: 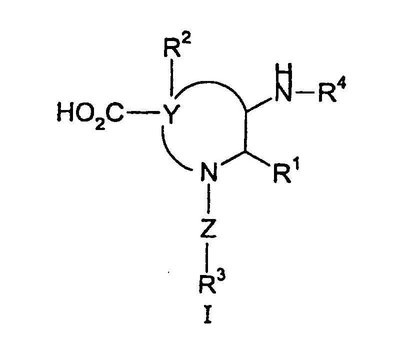 worin
Y (C₂ - C₄)-Alkylen ist;
Z eine Valenzbindung oder (C₁ - C₆)-Alkylen ist;
R¹ Phenyl, Biphenyl, Indanyl, Naphthyl, Thienyl, Furyl, Pyridyl, Thiazolyl, Isothiazolyl, Oxazolyl, Isoxazolyl, Tetrazolyl, Chinolyl, Phenyl-(C₁ - C₆)-alkyl- oder Benzhydryl ist, wobei jede der Ringeinheiten gegebenenfalls durch einen oder mehrere Substituenten substituiert sein kann, die unabhängig aus Halogen, (C₁ - C₆)-Alkyl, Halogen-substituiertem (C₁ - C₆)-Alkyl, (C₁ - C₆)-Alkoxy und Halogen-substituiertem (C₁ - C₆)-Alkoxy ausgewählt sind:
R² Wasserstoff oder (C₁ - C₆)-Alkyl ist;
R³ Wasserstoff, Hydroxy, Cyano, Amino oder Carboxy ist; und
R⁴ eine Gruppe der Formel (II) darstellt, 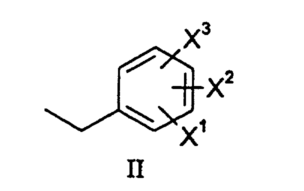 in der X¹, X² und X³ jeweils Halogen, Wasserstoff, Nitro, (C₁ - C₆)-Alkyl, Halogen-substituiertes (C₁ - C₆)-Alkyl, (C₁ - C₆)-Alkoxy, Halogen-substituiertes (C₁ - C₆)-Alkoxy, Hydroxy, Amino, (C₁ - C₆)-Alkylthio, (C₁ - C₆)-Alkylsulfinyl oder (C₁ - C₆)-Alkylsulfonyl sind;
vorausgesetzt, dass, (i) wenn Z eine Valenzbindung ist, R³ Wasserstoff ist, und (ii) wenn Z eine Valenzbindung ist, R³ Wasserstoff ist, Y C₃-Alkylen ist, R² für H steht und R¹ Phenyl ist, dann R⁴ keine Gruppe der Formel: 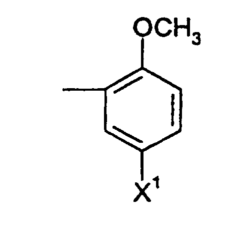 ist, in der X¹ für (C₁ - C₅)-Alkyl, Methylthio, Methylsulfinyl oder Methylsulfonyl steht.

2. Verbindung nach Anspruch 1, in der Y Ethylen oder Propylen ist.

3. Verbindung nach irgendeinem der vorangehenden Ansprüche, in der -ZR³ Wasserstoff ist.

4. Verbindung nach irgendeinem der vorangehenden Ansprüche, in der R¹ Phenyl ist.

5. Verbindung nach irgendeinem der vorangehenden Ansprüche, in der R² an das Kohlenstoffatom gebunden ist, das an den Stickstoff von NZR³ angrenzt.

6. Verbindung nach Anspruch 1, ausgewählt aus

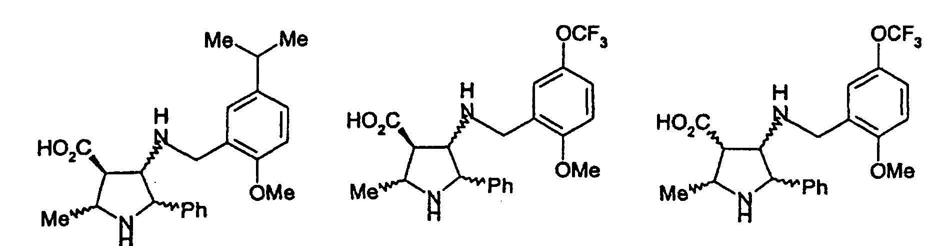

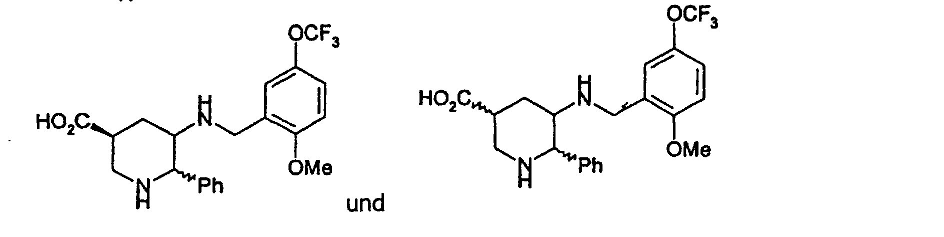

7. Pharmazeutische Zusammensetzung, die eine Verbindung der Formel (I), wie in irgendeinem der vorangehenden Ansprüche beansprucht, oder ein pharmazeutisch annehmbares Salz derselben zusammen mit einem pharmazeutisch annehmbaren Träger umfasst.

8. Verbindung der Formel (I) oder ein pharmazeutisch annehmbares Salz derselben, wie in irgendeinem der Ansprüche 1 bis 6 beansprucht, zur Verwendung als Medikament.

9. Verwendung einer Verbindung der Formel (I), wie in irgendeinem der Ansprüche 1 bis 6 beansprucht, oder eines pharmazeutisch annehmbaren Salzes derselben für die Herstellung eines Medikaments zur Behandlung oder Verhütung von gastrointestinalen Krankheiten, Krankheiten des zentralen Nervensystems, Allergie, entzündlichen Krankheiten, Asthma, Schmerzen, Migräne oder Emesis.

## Revendications

1. Composé de formule chimique (I) et ses sels pharmaceutiquement acceptables : 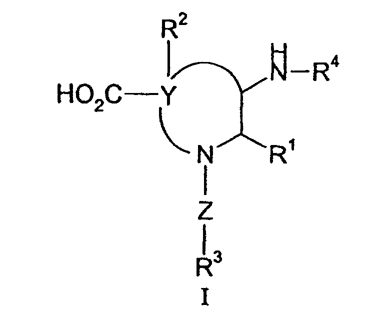 dans laquelle Y représente un groupe alkylène en C₂ à C₄ ;
Z représente une liaison de valence ou un groupe alkylène en C₁ à C₆ ;
R¹ représente un groupe phényle, biphényle, irdanyle, naphtyle, thiénylé, furyle, pyridyle, thiazolyle, iso-thiazolyle, oxazolyle, isoxazolyle, tétrazolyle, quinolyle, phényl(alkyle en C₁ à C₆)- ou benzhydryle, dans lequel chacun des groupements cycliques peut être facultativement substitué avec un ou plusieurs substituants choisis, indépendamment, entre des substituants halogéno, alkyle en C₁ à C₆, alkyle en C₁ à C₆ à substituant halogéno, alkoxy en C₁ à C₆ et alkoxy en C₁ à C₆ à substituant halogéno ;
R² représente un atome d'hydrogène ou un groupe alkyle en C₁ à C₆ ;
R³ représente un atome d'hydrogène, un groupe hydroxy, cyano, amino ou carboxy ; et
R⁴ représente un groupe de formule (II) 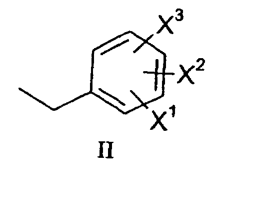 dans laquelle X¹, X² et X³ représentent chacun un groupe halogéno, un atome d'hydrogène, un groupe nitro, alkyle en C₁ à C₆, alkyle en C₁ à C₆ à substituant halogéno, alkoxy en C₁ à C₆, alkoxy en C₁ à C₆ à substituant halogéno, hydroxy, amino, alkylthio en C₁ à C₆, alkylsulfinyle en C₁ à C₆ ou alkylsulfonyle en C₁ à C₆ ;
sous réserve que (i) lorsque Z représente une liaison de valence, R₃ représente un atome d'hydrogène et (ii) lorsque Z représente une liaison de valence, R³ représente un atome d'hydrogène, Y représente un groupe alkylène en C₃, R² représente H et R¹ représente un groupe phényle, alors R⁴ soit autre qu'un groupe de formule : 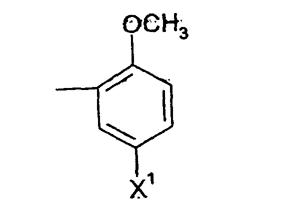 dans laquelle X¹ représente un groupe alkyle en C₁ à C₅, méthylthio, méthylsulfinyle ou méthylsulfonyle.

2. Composé suivant la revendication 1, dans lequel Y représente un groupe éthylène ou propylène.

3. Composé suivant l'une quelconque des revendications précédentes, dans lequel -ZR³ représente un atome d'hydrogène.

4. Composé suivant l'une quelconque des revendications précédentes, dans lequel R¹ représente un groupe phényle.

5. Composé suivant l'une quelconque des revendications précédentes, dans lequel R² est fixé à l'atome de carbone adjacent à l'atome d'azote de NZR³.

6. Composé suivant la revendication 1, choisi entre

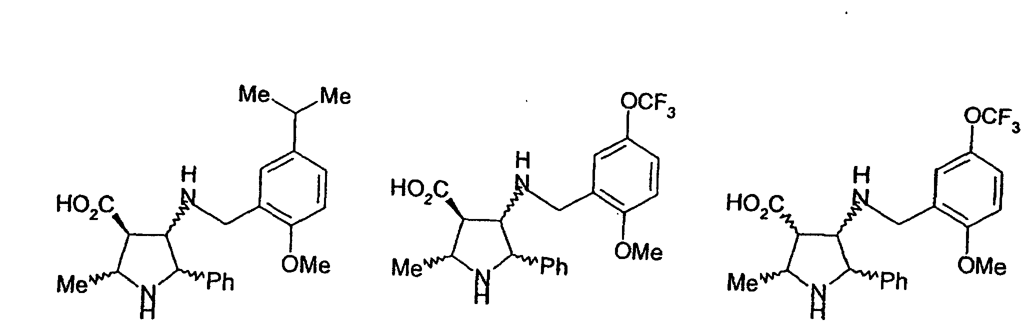

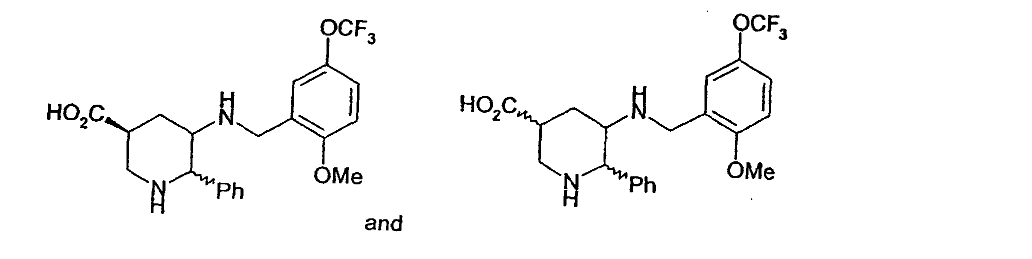

7. Composition pharmaceutique qui comprend un composé de formule (I) suivant l'une quelconque des revendications précédentes ou un de ses sels pharmaceutiquement acceptables avec un support pharmaceutiquement acceptable.

8. Composé de formule (I) ou un de ses sels pharmaceutiquement acceptables suivant l'une quelconque des revendications 1 à 6, destiné à être utilisé comme médicament.

9. Utilisation d'un composé de formule (I) suivant l'une quelconque des revendications 1 à 6 ou d'un de ses sels pharmaceutiquement acceptables pour la production d'un médicament destiné au traitement ou à la prévention de troubles gastro-intestinaux, de troubles du système nerveux central, de l'allergie, de maladies inflammatoires, de l'asthme, de la douleur, de la migraine ou des vomissements.
